(19) Europäisches Patentamt
European Patent Office
Office européen des brevets

(11) EP 1 491 544 A1

(12) DEMANDE DE BREVET EUROPEEN

(43) Date de publication:
**29.12.2004 Bulletin 2004/53**

(21) Numéro de dépôt: **04291568.6**

(22) Date de dépôt: **22.06.2004**

(51) Int Cl.[7]: **C07D 491/04**, C07D 491/14, A61K 31/436, A61P 35/00 // (C07D491/04, 311:00, 221:00), (C07D491/14, 317:00, 311:00, 221:00)

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **25.06.2003 FR 0307664**

(71) Demandeur: **Les Laboratoires Servier**
**92415 Courbevoie Cedex (FR)**

(72) Inventeurs:
- **Koch, Michel**
**78170 La Celle Saint Cloud (FR)**
- **Tillequin, François**
**75014 Paris (FR)**
- **Michel, Sylvie**
**75002 Paris (FR)**
- **Hickman, John**
**75017 Paris (FR)**
- **Pierre, Alain**
**78000 Les Alluets Le Roi (FR)**
- **Leonce, Stéphane**
**78000 Versailles (FR)**
- **Pfeiffer, Bruno**
**95320 Saint Leu La Foret (FR)**
- **Renard, Pierre**
**78150 Le Chesnay (FR)**

(54) **Dérivés de benzo(a)pyrano(3,2-h)acridin-7-one, leur procédé de préparation et les compositions pharmaceutiques qui les contiennent**

(57) Composés de formule (I) :

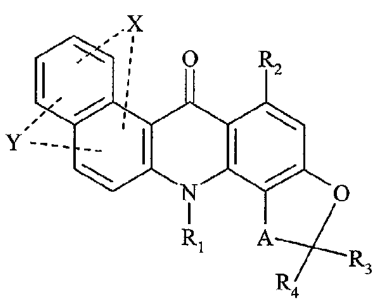

(I)

dans laquelle :

- X et Y représentent un groupement choisi parmi hydrogène, halogène, hydroxy, alkoxy, nitro, cyano, alkyle, trihalogénoalkyle et $NR_aR_b$ dans lequel $R_a$ et $R_b$ sont tels que définis dans la description
- $R_1$ représente un atome d'hydrogène ou un groupement alkyle
- $R_2$ représente un groupement choisi parmi atome hydrogène, alkyle, $-OR''_a$ ; $-NR'_aR'_b$ ; $-O-T_a-OR''_a$ ; $-NR''_a-T_a-NR'_aR'_b$ ; $-NR''_a-C(O)-T_aH$ ; $-O-C(O)-T_aH$ ; $-O-T_a-NR'_aR'_b$ ; $-NR''_a-T_a-OR''_a$ ; $-NR''_a-T_a-CO_2R''_a$ ; $-NR''_a-C(O)-T_a-NR'_aR'_b$ dans lesquels $R'_a$, $R''_a$, $R'_b$ et $T_a$ sont tels que définis dans la description
- $R_3$, $R_4$ représentent un atome d'hydrogène ou un groupement alkyle
- A représente un groupement de formule $-CH(R_5)-CH(R_6)-$, $-CH=C(R_7)-$, $-C(R_7)=CH-$, $-C(O)-CH(R_8)$, ou $-CH(R_8)-C(O)$ dans lesquelles $R_5$, $R_6$, $R_7$ et $R_8$ sont tels que définis dans la description leurs isomères définis dans la description, N-oxydes, ainsi que leurs sels d'addition à un acide ou à une base pharmaceutiquement acceptable

Médicaments.

EP 1 491 544 A1

## Description

La présente invention concerne de nouveaux dérivés de benzo[*a*]pyrano[3,2-*h*]acridin-7-one, leur procédé de préparation et les compositions pharmaceutiques qui les contiennent.

Les composés de l'invention constituent des dérivés de l'acronycine qui est un alcaloïde présentant des propriétés antitumorales mises en évidence dans des modèles expérimentaux *(J. Pharm.. Sci.,* 1966, 55 (8), 758-768). Cependant, malgré un spectre d'activité assez large, l'acronycine est peu puissante et modérément active. De plus, ce produit présente une faible solubilité limitant sa biodisponibilité ainsi que son utilisation dans des compositions pharmaceutiques administrables par voie intraveineuse.

Diverses modifications ont été réalisées sur cette molécule, comme celles décrites dans *J. Med. Chem.,* 1996, 39, 4762-4766 ou EP 1 042 326 et qui ont permis d'améliorer significativement la puissance, l'efficacité antitumorale et la solubilité de ces produits. Néanmoins, les besoins de la thérapeutique anticancéreuse exigent le développement constant de nouveaux agents antitumoraux, dans le but d'obtenir des médicaments à la fois plus actifs et mieux tolérés. Plus particulièrement, les tumeurs solides posent un problème majeur à la chimiothérapie anticancéreuse, de par leur résistance intrinsèque et/ou acquise, aux produits existants. Il est donc primordial d'avoir accès à une gamme la plus large possible de produits exprimant une activité cytotoxique forte afin de pouvoir disposer de traitements des plus efficaces sur l'ensemble des affections tumorales.

Les composés de l'invention, outre le fait qu'ils soient nouveaux, présentent une activité cytotoxique *in vitro* et *in vivo* surprenante et supérieure à celle observée jusqu'ici. Ainsi, les composés découverts par la Demanderesse possèdent des propriétés antitumorales qui les rendent particulièrement utiles pour le traitement des cancers. Parmi les types de cancers qui peuvent être traités par les composés de la présente invention, on peut citer à titre non limitatif les adénocarcinomes et carcinomes, sarcomes, gliomes et leucémies.

Plus particulièrement, la présente invention concerne les composés de formule (I) :

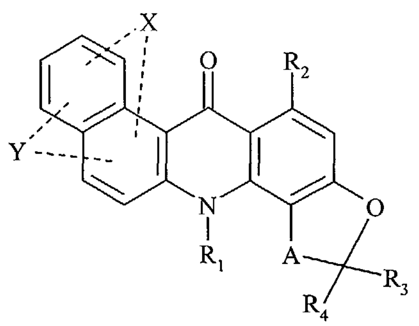

(I)

dans laquelle :

- X et Y, identiques ou différents, indépendamment l'un de l'autre, représentent chacun un groupement choisi parmi :

  - atome d'hydrogène, halogène,
  - groupement hydroxy, alkoxy ($C_1$-$C_6$) linéaire ou ramifié, nitro, cyano, alkyle ($C_1$-$C_6$) linéaire ou ramifié, alkényle ($C_2$-$C_6$) linéaire ou ramifié, polyhalogénoalkyle ($C_1$-$C_6$) linéaire ou ramifié, ou
  - groupement de formule $-NR_aR_b$ dans lequel:

    $R_a$ et $R_b$, identiques ou différents, indépendamment l'un de l'autre, représentent chacun un groupement choisi parmi atome d'hydrogène, $-C(O)-CF_3$, $-C(O)-NH_2$ ou groupement alkyle ($C_1$-$C_6$) linéaire ou ramifié, éventuellement substitué par un groupement $NR'_aR'_b$ dans lequel :
    $R'_a$ et $R'_b$, identiques ou différents, indépendamment l'un de l'autre, représentent chacun un groupement choisi parmi atome d'hydrogène, groupement alkyle ($C_1$-
    $C_6$) linéaire ou ramifié, aryle, arylalkyle ($C_1$-$C_6$) linéaire ou ramifié, ou $R'_a$ et $R'_b$ forment ensemble avec l'atome d'azote qui les portent, un hétérocycle de 5 à 7 chaînons, monocyclique, contenant éventuellement au sein du système cyclique un second hétéroatome choisi parmi oxygène et azote,
    ou $R_a$ et $R_b$ forment ensemble avec l'atome d'azote qui les portent, un hétérocycle de 5 à 7 chaînons, monocyclique, contenant éventuellement au sein du système cyclique un second hétéroatome choisi parmi oxygène et azote, étant entendu que les substituants X et Y peuvent être présents sur l'un ou l'autre des deux cycles benzéniques adjacents,

- $\underline{R_1}$ représente un atome d'hydrogène ou un groupement alkyle ($C_1$-$C_6$) linéaire ou ramifié,

- $\underline{R_2}$ représente un groupement choisi parmi atome d'hydrogène, groupement alkyle ($C_1$-$C_6$) linéaire ou ramifié, $-OR''_a$ ; $-NR'_aR'_b$ ; $—O—T_a—OR'_a$ ; $—NR''_a—T_a—NR'_aR'_b$; $—NR''_a—C(O)—T_aH$ ; $-O-C(O)-T_aH$ ; $—O—T_a—NR'_aR'_b$ ; $—NR''_a—T_a—OR''_a$ ; $-NR''_a-T_a-CO_2R''_a$ ; $-NR''_aC(O)-T_a-NR'_aR'_b$
  dans lesquels :

  - * $T_a$ représente une chaîne alkylène ($C_1$-$C_6$) linéaire ou ramifiée,
  - * $R'_a$ et $R'_b$ sont tels que définis précédemment,
  - * $R''_a$ représente un groupement choisi parmi atome d'hydrogène, groupement alkyle ($C_1$-$C_6$) linéaire ou ramifié,

- $\underline{R_3}$, $\underline{R_4}$, identiques ou différents, indépendamment l'un de l'autre, représentent un atome d'hydrogène ou un groupement alkyle ($C_1$-$C_6$) linéaire ou ramifié, ou $R_3$ et $R_4$ forment ensemble avec l'atome de carbone qui les portent, un cycle de 3 à 6 chaînons, monocyclique,

- <u>A</u> représente un groupement de formule:

  **a) $-CH(R_5)-CH(R_6)-$** dans lequel :

  * *$R_5$ et $R_6$, identiques ou différents, indépendamment l'un de l'autre représentent chacun un groupement choisi parmi:*

    **1)** atome d'hydrogène,

    **2)** groupement $OR_c$, $NR_cR_d$, $SR_c$, dans lesquels :

    - $R_c$, $R_d$, identiques ou différents, indépendamment l'un de l'autre, représentent chacun un groupement choisi parmi atome d'hydrogène, groupement alkyle ($C_1$-$C_6$) linéaire ou ramifié, ou un groupement aryle, arylalkyle ($C_1$-$C_6$) linéaire ou ramifié, ou un groupement $C(O)-R_e$ dans lequel $R_e$ représente un groupement choisi parmi atome d'hydrogène, groupement aryle, ou $NR'''_aR'''_b$ dans lequel $R'''_a$ et $R'''_b$, identiques ou différents, représentent un atome d'hydrogène, groupement alkyle ($C_1$-$C_6$) linéaire ou ramifié ou $R'''_a$ et $R'''_b$ forment ensemble avec l'atome d'azote qui les portent, un hétérocycle de 5 à 7 chaînons, monocyclique, contenant éventuellement au sein du système cyclique un second hétéroatome choisi parmi oxygène et azote,

    3) $W_1-C(W_2)-U-V$ dans lequel :

      α) $W_1$ représente un atome d'oxygène, de soufre ou $NR_c$ (dans lequel $R_c$ est tel que défini précédemment),
      β) $W_2$ représente un atome d'oxygène ou un atome de soufre,
      γ) U représente une chaîne alkylène ($C_1$-$C_8$) linéaire ou ramifiée ou une chaîne alkénylène ($C_2$-$C_8$) linéaire ou ramifiée,
      δ) V représente un groupement choisi parmi :

      - atome d'hydrogène,
      - groupement aryle,
      - groupements $OR_c$, $CO_2R_c$, $COR_c$, $CONR'_aR'_b$, $NR'_aR'_b$, $N(R_c)-CO_2R'_c$, $N(R_c)-COR'_c$, dans lesquels $R'_a$, $R'_b$, et $R_c$ sont tels que définis précédemment et $R'_c$ représente un groupement choisi parmi atome d'hydrogène, groupement alkyle ($C_1$-$C_6$) linéaire ou ramifié, ou un groupement aryle, arylalkyle ($C_1$-$C_6$) linéaire ou ramifié,

      ε) U représente une liaison lorsque $W_2$ ne représente pas un atome d'oxygène et V ne représente pas un groupement choisi parmi:

      - atome d'hydrogène,
      - groupement aryle,
      - $NH_2$,

4) $W_1$-C($W_2$)-$W_3$-$T_1$ dans lequel :

α) $W_1$ et $W_2$ sont tels que définis précédemment,
β) $W_3$ représente un atome d'oxygène, de soufre ou $NR_c$ dans lequel $R_c$ est tel que défini précédemment,
γ) $T_1$ représente un groupement choisi parmi:

- atome d'hydrogène
- alkyle ($C_1$-$C_6$) linéaire ou ramifié,
- alkényle ($C_2$-$C_6$) linéaire ou ramifié,
- aryle, arylalkyle ($C_1$-$C_6$) linéaire ou ramifié,
- chaîne alkylène ($C_1$-$C_6$) linéaire ou ramifiée ou une chaîne alkénylène ($C_2$-$C_6$) linéaire ou ramifiée, chacune étant substituée par un groupement $OR_c$ dans lequel $R_c$ est tel que défini précédemment ou par $NR'_aR'_b$ dans lequel $R'_a$ et $R'_b$ sont tels que définis précédemment,

**5)** $W_1$-S(O)$_n$-$W_3$-$T_1$ dans lequel :

α**)** $W_1$, $W_3$ et $T_1$ sont tels que définis précédemment,
β**)** n représente un nombre entier choisi parmi 1 et 2,

**6)** $W_1$-S(O)$_n$-U'-V' dans lequel :

α**)** U' représente une chaîne alkylène ($C_1$-$C_8$) linéaire ou ramifiée ou une chaîne alkénylène ($C_2$-$C_8$) linéaire ou ramifiée,
β**)** V' représente un groupement choisi parmi:

- atome d'hydrogène,
- groupement aryle,
- groupements $OR_c$, $CO_2R_c$, $COR_c$, $CONR'_aR'_b$, $NR'_aR'_b$, N($R_c$)-$CO_2R'_c$, N($R_c$)-$COR'_c$, dans lesquels $R'_a$, $R'_b$, $R_c$ et $R'_c$ sont tels que définis précédemment, et,

γ) $W_1$ et n sont tels que définis précédemment,

**7)** C($W_2$)-$T_1$ dans lequel $W_2$ et $T_1$ sont tels que définis précédemment,

* *ou $R_5$ et $R_6$, forment ensemble :*

1) un groupement

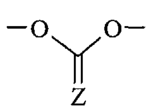 , 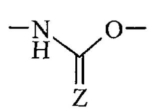 , 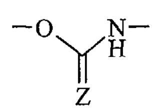 , 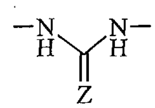

dans lesquels Z représente un atome d'oxygène ou un atome de soufre,

2) un groupement -O-($CH_2$)$_m$-O- dans lequel m représente un nombre entier compris entre 1 et 4 inclus,

**3)** un groupement

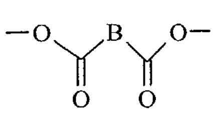 , 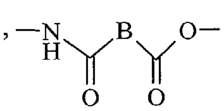 , 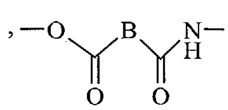

dans lesquels B représente une liaison simple, une chaîne alkylène ($C_1$-$C_6$) linéaire ou ramifiée ou une chaîne alkénylène ($C_2$-$C_6$) linéaire ou ramifiée,

* *ou $R_5$ et $R_6$, forment ensemble avec les atomes de carbone qui les portent, un groupement oxiranne ou un groupement aziridine,* éventuellement substitué sur l'atome d'azote par un groupement alkyle ($C_1$-$C_6$) linéaire ou ramifié,

**b) -CH=C($R_7$)- ou -C($R_7$)=CH-** dans lesquels $R_7$ représente un groupement choisi parmi :

- atome d'hydrogène,
- groupement OR"$_a$, $W_1$-C($W_2$)-U-V, $W_1$—C($W_2$)—$W_3$—$T_1$, $W_1$—S(O)$_n$—$W_3$—$T_1$, $W_1$-S(O)$_n$-U'-V' ou C ($W_2$)-$T_1$ dans lesquels R"$_a$, $W_1$, $W_2$, $W_3$, U, V, U', V', $T_1$ et n sont tels que définis précédemment,

**c) -C(O)-CH($R_8$)- ou - CH($R_8$)-C(O)-** dans lesquels $R_8$ représente un groupement choisi parmi :

- atome d'hydrogène,
- alkylcarbonyloxy ($C_1$-$C_6$) linéaire ou ramifié, groupement OR"$_a$ dans lequel R"$_a$ est tel que défini précédemment,

leurs énantiomères, diastéréoisomères, N-oxydes, ainsi que leurs sels d'addition à un acide ou à une base pharmaceutiquement acceptable, étant entendu que par aryle, on comprend un groupement phényle ou naphtyle, comportant éventuellement une ou plusieurs substitutions, identiques ou différentes, choisies parmi hydroxy, halogène, carboxy, nitro, amino, alkylamino ou dialkylamino ($C_1$-$C_6$) linéaire ou ramifié, alkoxy ($C_1$-$C_6$) linéaire ou ramifié, acyle ($C_1$-$C_6$) linéaire ou ramifié, et alkylcarbonyloxy ($C_1$-$C_6$) linéaire ou ramifié.

Parmi les hétérocycles de 5 à 7 chaînons, monocyclique, contenant éventuellement au sein du système cyclique un second hétéroatome choisi parmi oxygène et azote, on peut citer à titre non limitatif les groupements pyrrolidinyle, isoxazolidinyle, oxazolidinyle, pyrazolidinyle, imidazolidinyle, pipéridinyle, oxazinanyle, morpholinyle, hexahydropyridazinyle, hexahydropyrimidinyle, pipérazinyle, azépanyle, oxazépanyle, diazépanyle.

Parmi les cycles de 3 à 6 chaînons, monocyclique, on peut citer à titre non limitatif les groupements cyclopropyle, cyclobutyle, cyclopentyle et cyclohexyle.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthane sulfonique, benzène sulphonique, camphorique, la lysine, etc...

Parmi les bases pharmaceutiquement acceptables, on peut citer à titre non limitatif l'hydroxyde sodium, l'hydroxyde de potassium, la triéthylamine, la tertbutylamine, etc...

Selon une variante avantageuse de l'invention, les composés préférés sont les composés de formule (IA) :

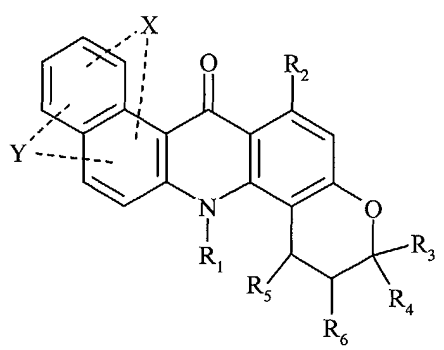

(IA)

dans laquelle X, Y, $R_1$, $R_2$, $R_3$, $R_4$, $R_5$ et $R_6$ sont tels que définis dans la formule (I).

Les composés préférés de formule (IA) sont les composés dans lesquels $R_5$ et $R_6$, identiques ou différents, représentent un groupement de formule -$OR_c$, $W_1$-C($W_2$)-U-V, $W_1$-C($W_2$)-$W_3$-$T_1$, C($W_2$)-T ou $R_5$ et $R_6$ forment ensemble un groupement

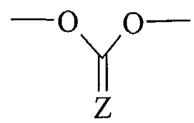

dans lesquels $R_c$, $W_1$, $W_2$, $W_3$, U, V, $T_1$ et Z sont tels que définis dans la formule (I).

D'une façon particulièrement intéressante, les composés préférés de formule (IA) sont les composés dans lesquels $R_5$ et $R_6$, identiques, représentent un groupement de formule $-OR_c$ dans laquelle $R_c$ représente un atome d'hydrogène.

D'une autre façon particulièrement intéressante, les composés préférés de formule (IA) sont les composés dans lesquels $R_5$ et $R_6$ identiques ou différents, représentent un groupement de formule $W_1$-C($W_2$)-U-V dans laquelle $W_1$ et $W_2$ représentent un atome d'oxygène, U est tel que défini dans la formule (I) et V représente un atome d'hydrogène ou U représente une chaîne alkylène ($C_1$-$C_8$) linéaire ou ramifiée et V représente un groupement $NR'_aR'_b$ dans lequel $R'_a$ et $R'_b$ identiques ou différents représentent un atome d'hydrogène ou un groupement alkyle ($C_1$-$C_6$) linéaire ou ramifié.

D'une façon encore plus intéressante, les composés préférés de formule (IA) sont les composés dans lesquels $R_5$ représente un groupement de formule $-OR_c$ dans laquelle $R_c$ représente un atome d'hydrogène et $R_6$ représente un groupement de formule $W_1$-C($W_2$)-U-V dans laquelle $W_1$ et $W_2$ représentent un atome d'oxygène, U représente une chaîne alkylène ($C_1$-$C_8$) linéaire ou ramifiée et V représente un atome d'hydrogène.

D'une autre façon encore plus intéressante, les composés préférés de formule (IA) sont les composés pour lesquels $R_5$ représente un groupement de formule $-OR_c$ ou $W_1$-C($W_2$)-U-V dans lesquels $R_c$ représente un atome d'hydrogène, $W_1$ et $W_2$ représentent un atome d'oxygène, U représente une chaîne alkylène ($C_1$-$C_8$) linéaire ou ramifiée et V représente un atome d'hydrogène et $R_6$ représente un groupement de formule $W_1$-C($W_2$)-U-V dans laquelle $W_1$ et $W_2$ représentent un atome d'oxygène, U représente une chaîne alkénylène ($C_2$-$C_8$) linéaire ou ramifiée et V représente un groupement choisi parmi atome d'hydrogène ou groupement aryle.

D'une façon intéressante, les composés préférés de formule (IA) sont les composés dans lesquels $R_5$ et $R_6$ identiques ou différents, représentent un groupement de formule $W_1$-C($W_2$)-$W_3$-$T_1$ dans laquelle $W_1$ et $W_2$ représentent un atome d'oxygène, $W_3$ représente un groupement $-NR_c$ dans lequel $R_c$ représente un groupement alkyle ($C_1$-$C_6$) linéaire ou ramifié et $T_1$ représente un groupement alkyle ($C_1$-$C_6$) linéaire ou ramifié.

D'une autre façon très intéressante, les composés préférés de formule (IA) sont les composés dans lesquels $R_5$ et $R_6$ forment ensemble un groupement

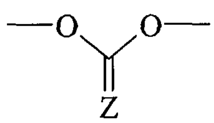

dans lequel Z représente un atome d'oxygène.

Selon une deuxième variante avantageuse de l'invention, les composés préférés sont les composés de formule (IB) :

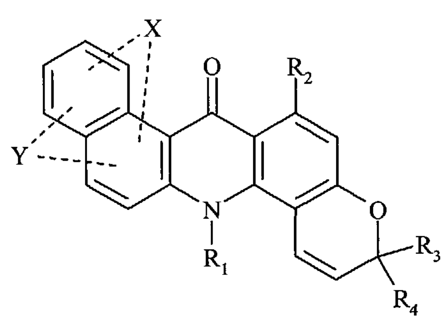

(IB)

dans laquelle X, Y, $R_1$, $R_2$, $R_3$ et $R_4$ sont tels que définis dans la formule (I).

Les substituants $R_3$ et $R_4$ préférés selon l'invention sont le groupement alkyle ($C_1$-$C_6$) linéaire ou ramifié.

Encore plus préférentiellement, les substituants $R_3$ et $R_4$ préférés selon l'invention sont le groupement méthyle.

Le substituant $R_2$ préféré selon l'invention sont les groupements -OR"$_a$ et -NR"$_a$-$T_a$-NR'$_a$R'$_b$ dans lesquels R'$_a$, R'$_b$, R"$_a$ et $T_a$ sont tels que définis dans la formule (I).

Encore plus préférentiellement, le substituant $R_2$ préféré selon l'invention sont le groupement -OR"$_a$ dans lequel R"$_a$ est tel que défini dans la formule (I) et le groupement - NR"$_a$-$T_a$-NR'$_a$R'$_b$ dans lequel R"$_a$ représente un atome d'hydrogène, $T_a$ est tel que défini dans la formule (I) et R'$_a$ et R'$_b$ identiques ou différents, représentent un groupement alkyle ($C_1$-$C_6$) linéaire ou ramifié.

Les substituants X et Y préférés selon l'invention sont l'atome d'hydrogène.

D'une façon particulièrement avantageuse, les composés préférés de l'invention sont le :

- (±)-*cis*-1,2-dihydroxy-6-méthoxy-3,3,14-triméthyl-1,2,3,14-tétrahydro-7*H*-benzo[α] pyrano[3,2-*h*]acridin-7-one,
- (±)-*cis*-1,2-diacétoxy-6-méthoxy-3,3,14-triméthyl-1,2,3,14-tétrahydro-7*H*-benzo[α] pyrano[3,2-*h*]acridin-7-one,
- (±)-*cis*-7-méthoxy-4,4,15-triméthyl-15,15c-dihydro-4*H*-benzo[α][1,3]dioxolo [4',5':4,5]pyrano[3,2-*h*]acridine-2,8 (3a*H*)-dione,
- 6-méthoxy-3,3,14-triméthyl-3,14-dihydro-7*H*-benzo[α]pyrano[3,2-*h*]acridin-7-one,
- 6-hydroxy-3,3,14-triméthyl-3,14-dihydro-7*H*-benzo[α]pyrano[3,2-*h*]acridin-7-one,
- 6-hydroxy-3,3-diméthyl-3,14-dihydro-7*H*-benzo[α]pyrano[3,2-*h*]acridin-7-one.

Les énantiomères, diastéréoisomères, N-oxydes, ainsi que les sels d'addition à un acide ou à une base, pharmaceutiquement acceptable, des composés préférés font partie intégrante de l'invention.

La présente invention s'étend également au procédé de préparation des composés de formule (I), caractérisé en ce qu'on utilise comme produit de départ un composé de formule (II) :

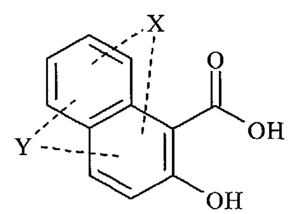

(II)

dans laquelle X et Y sont tels que définis dans la formule (I), composé de formule (II) qui est traité avec du diméthylsulfate en milieu basique, pour conduire aux composés de formule (III) :

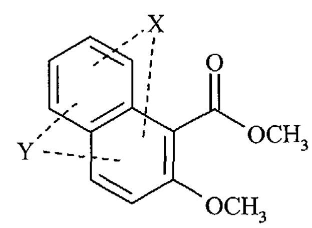

(III)

dans laquelle X et Y sont tels que définis précédemment, composés de formule (III) qui est traité par une solution alcaline de Claisen, une solution d'hydroxyde de potassium dans un mélange eau-méthanol, puis une solution d'acide chlorhydrique, pour conduire aux composés de formule (IV) :

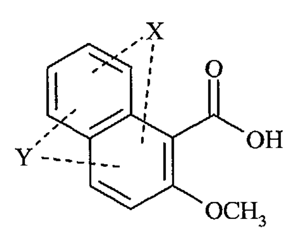

(IV)

dans laquelle X et Y sont tels que définis précédemment, composé de formule (IV) qui est traité par du chlorure de

thionyle puis en milieu anhydre par un composé de formule (V) :

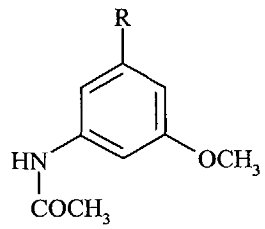

(V)

dans laquelle R représente un atome d'hydrogène, un groupement hydroxy, un groupement alkyle ($C_1$-$C_6$) linéaire ou ramifié ou un groupement alkoxy ($C_1$-$C_6$) linéaire ou ramifié pour conduire aux composés de formule (VI) :

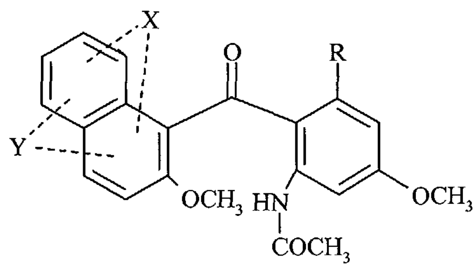

(VI)

dans laquelle X, Y et R sont tels que définis précédemment, composé de formule (VI) qui est traité par une suspension d'hydrure de sodium dans un solvant aprotique anhydre, pour conduire aux composés de formule (VII) :

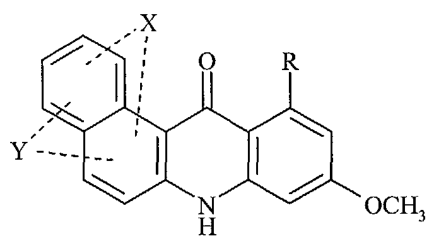

(VII)

dans laquelle X, Y et R sont tels que définis précédemment, composé de formule (VII) qui est traité par une solution aqueuse d'acide bromhydrique dans de l'acide acétique, pour conduire aux composés de formule (VIII) :

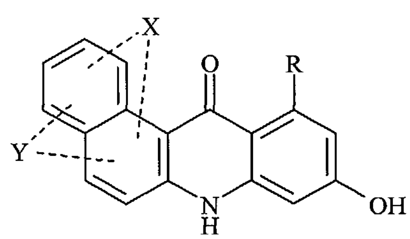

(VIII)

dans laquelle X, Y et R sont tels que définis précédemment, composé de formule (VIII) qui est ensuite traité en condition basique dans un solvant aprotique anhydre par un alcyne de formule (IX) :

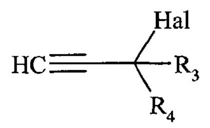

(IX)

dans laquelle Hal représente un atome d'halogène et $R_3$ et $R_4$ sont tels que définis dans la formule (I), pour conduire aux composés de formule (I/a), cas particulier des composés de formule (I) :

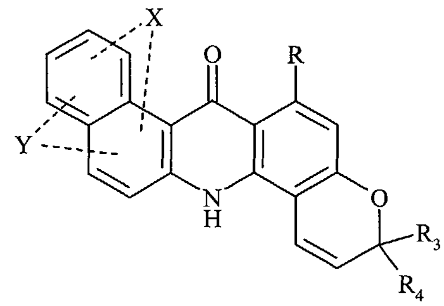

(I/a)

dans laquelle X, Y, R, $R_3$ et $R_4$ sont tels que définis précédemment, composés de formule (I/a) dont l'atome d'azote est éventuellement substitué, par action d'un halogénure d'alkyle ou d'un sulfate de dialkyle en présence d'un agent de déprotonation, en solvant polaire aprotique, pour conduire aux composés de formule (I/b), cas particulier des composés de formule (I) :

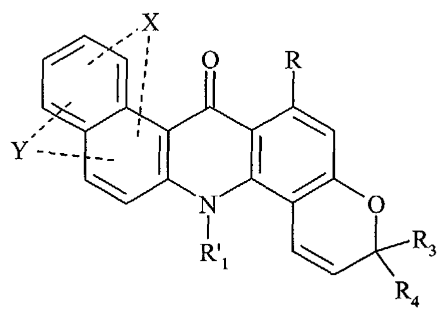

(I/b)

dans laquelle $R'_1$ représente un groupement alkyle ($C_1$-$C_6$) linéaire ou ramifié et X, Y, R, $R_3$ et $R_4$ sont tels que définis précédemment, composés de formule (I/b) qui sont éventuellement soumis, dans le cas où R représente un groupement hydroxy, à l'action d'un agent alkylant ou d'un agent acylant, pour conduire aux composés de formule (I/c), cas particulier des composés de formule (I) :

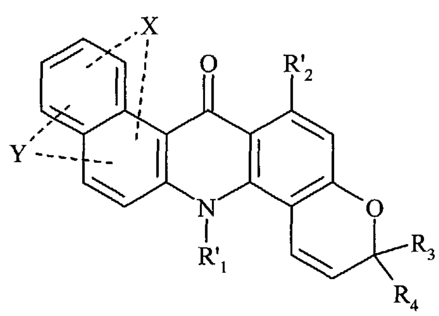

(I/c)

dans laquelle $R'_2$ représente un groupement choisi parmi $-OR''_a$, $-O-T_a-OR''_a$, $-O-C(O)-T_aH$, $-O-T_a-NR'_aR'_b$ dans lesquels $R''_a$, $R'_a$, $R'_b$ et $T_a$ sont tels que définis dans la formule (I) et X, Y, $R'_1$, $R_3$ et $R_4$ sont tels que définis précédemment,

composés de formule (I/c) qui sont éventuellement traités, dans le cas où $R'_2$ représente un groupement alkoxy ($C_1$-$C_6$) linéaire ou ramifié par un composé de formule (X) :

$$HNR_{10}R_{11} \text{ (X)}$$

dans laquelle $R_{10}$ représente un groupement choisi parmi $R'_a$ et $R''_a$ qui sont tels que définis dans la formule (I) et $R_{11}$ représente un groupement choisi parmi $R'_b$, $-T_a-NR'_aR'_b$, $-C(O)-T_aH$, $-T_a-OR''_a$ et $-T_a-CO_2R''_a$ dans lesquels $T_a$, $R'_a$, $R'_b$ et $R''_a$ sont tels que définis précédemment, pour conduire aux composés de formule (I/d), cas particulier des composés de formule (I) :

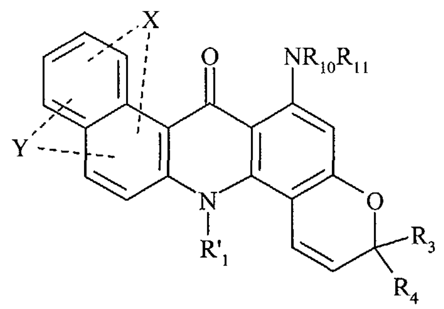

(I/d)

dans laquelle X, Y, $R'_1$, $R_3$, $R_4$, $R_{10}$ et $R_{11}$ sont tels que définis précédemment, l'ensemble des composés de formule (I/a) à (I/d) formant les composés de formule (I/e) :

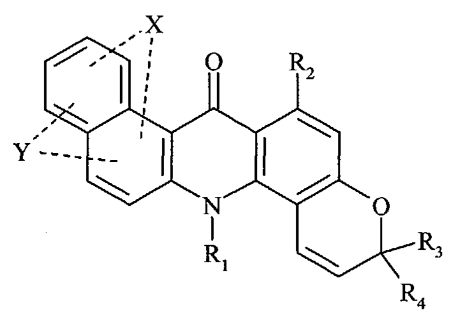

(I/e)

dans laquelle X, Y, $R_1$, $R_2$, $R_3$ et $R_4$ sont tels que définis dans la formule (I),

***composés de formule (I/e) pouvant être soumis,***

**a)** soit à l'action d'un agent réducteur, pour conduire aux composés de formule (I/f), cas particulier des composés de formule (I) :

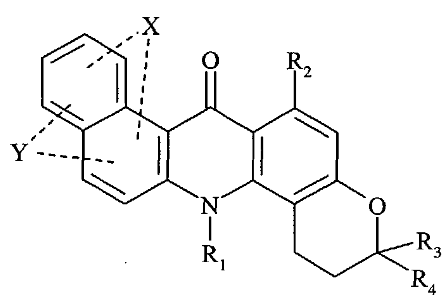

(I/f)

dans laquelle X, Y, $R_1$, $R_2$, $R_3$ et $R_4$ sont tels que définis précédemment,

**b)** soit à l'action du tétroxyde d'osmium en milieu polaire et en présence de 4-méthylmorpholine-N-oxyde, pour conduire aux composés de formules ($I/g_1$) et ($I/g_2$), cas particulier des composés de formule (I):

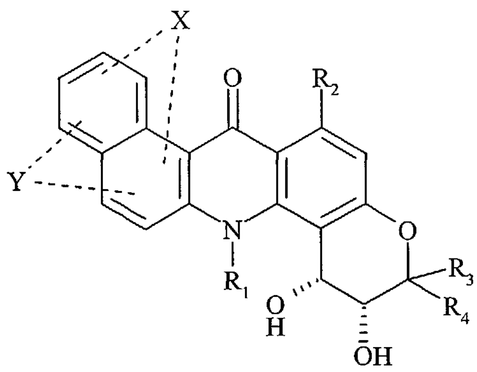

($I/g_1$)

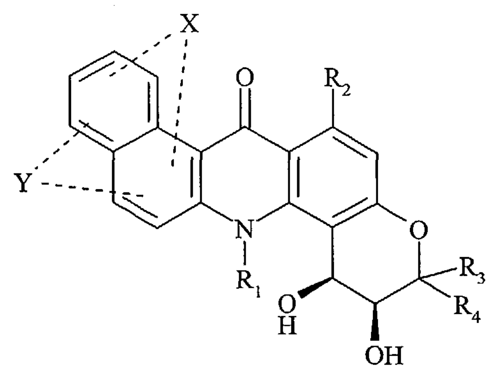

($I/g_2$)

dans laquelle X, Y, $R_1$, $R_2$, $R_3$ et $R_4$ sont tels que définis précédemment, l'ensemble des composés de formule ($I/g_1$) et ($I/g_2$) formant les composés *cis*-diol de formule *(cis*-I/g):

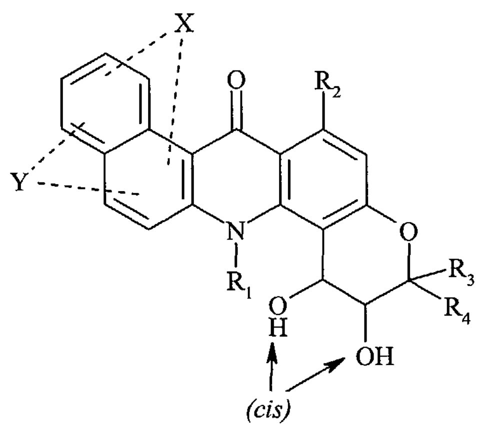


(*cis* - I/g)

dans laquelle X, Y, $R_1$, $R_2$, $R_3$ et $R_4$ sont tels que définis précédemment, composés *cis*-diol de formule (*cis*-I/g) qui sont soumis éventuellement à l'action d'un composé de formule (XI) :

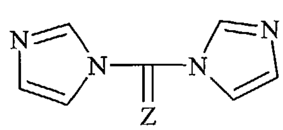

(XI)

dans laquelle Z est tel que défini dans la formule (I) pour conduire aux composés de formule (*cis*-I/h), cas particulier des composés de formule (I) :

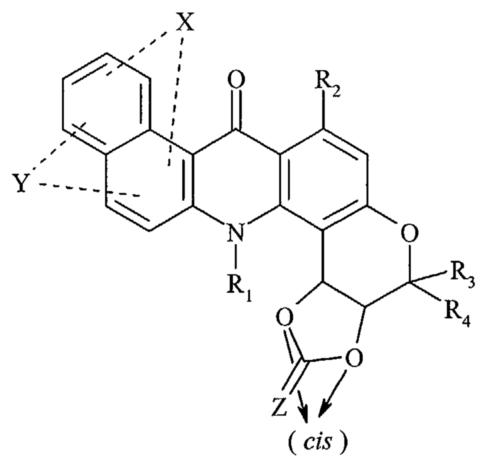

(*cis*-I/h)

dans laquelle X, Y, $R_1$, $R_2$, $R_3$ et $R_4$ sont tels que définis précédemment,

c) soit à l'action du permanganate de potassium en milieu polaire, pour conduire aux composés de formule (I/i), cas particulier des composés de formule (I) :

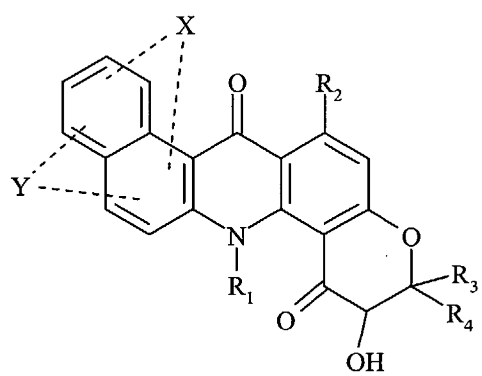

(I/i)

dans laquelle X, Y, $R_1$, $R_2$, $R_3$ et $R_4$ sont tels que définis précédemment,

**<u>composés de formule (I/i) pouvant être soumis</u> :**

α**)** soit à l'action d'un agent alkylant ou d'un agent acylant, pour conduire aux composés de formule (I/j), cas particulier des composés de formule (I) :

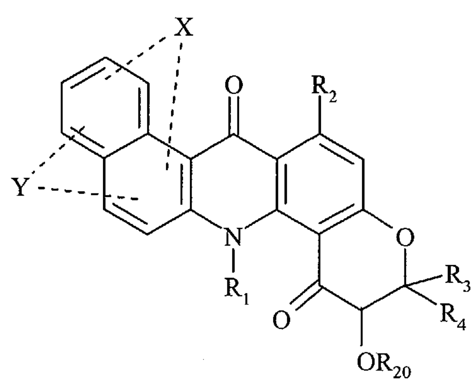

(I/j)

dans laquelle X, Y, $R_1$, $R_2$, $R_3$ et $R_4$ sont tels que définis précédemment, et $R_{20}$ représente un groupement choisi parmi alkyle ($C_1$-$C_6$) linéaire ou ramifié ou alkylcarbonyle ($C_1$-$C_6$) linéaire ou ramifié,

β**)** soit à des conditions réductrices en présence de $NaBH_4$, pour conduire aux composés de formule (I/k), cas particulier des composés de formule (I) :

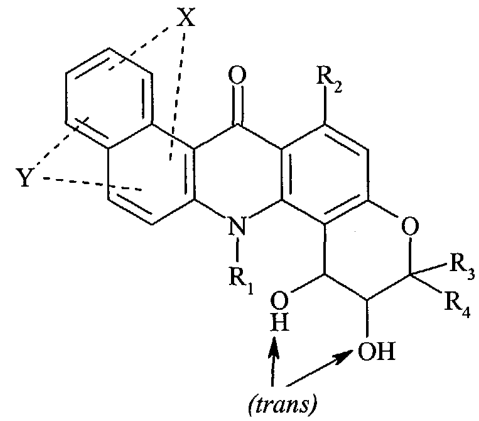


(I/k)

dans laquelle X, Y, $R_1$, $R_2$, $R_3$, et $R_4$ sont tels que définis précédemment, l'ensemble des composés de formules (*cis*-I/g) et (I/k) formant les composés de formule (I/l),

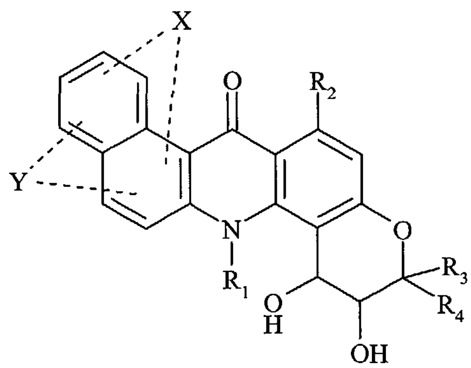

(I/l)

dans laquelle X, Y, $R_1$, $R_2$, $R_3$, et $R_4$ sont tels que définis précédemment,

γ**)** soit à l'action d'un chlorure de tosylate, suivi de l'action de $NaN_3$ en présence d'eau oxygénée, suivi d'une étape de réduction pour conduire aux composés de formule (I/m), cas particulier des composés de formule (I) :

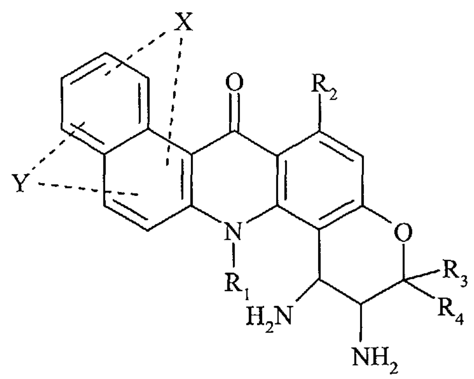

(I/m)

dans laquelle X, Y, $R_1$, $R_2$, $R_3$, et $R_4$ sont tels que définis précédemment,

*composés de formule I/m) qui sont soumis*

***1)*** soit à l'action du dioxyde de carbone, en présence de diphényl phosphite, pour conduire aux composés de formule (I/n), cas particulier des composés de formule (I):

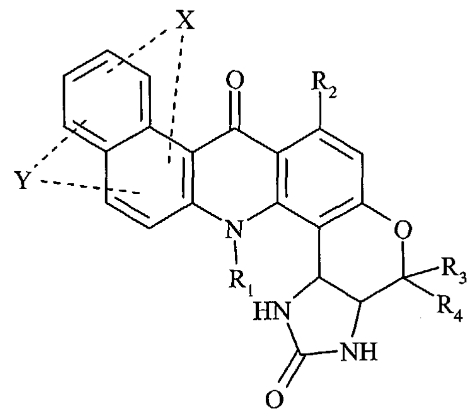

(I/n)

dans laquelle X, Y, $R_1$, $R_2$, $R_3$ et $R_4$ sont tels que définis précédemment,

***2)*** soit à l'action d'un composé de formule (XII) :

Hal-$G'_1$ (XII)

dans laquelle Hal est tel que défini précédemment et $G'_1$ représente un groupement choisi parmi -$R_c$, C($W_2$)-U-V, C($W_2$)-$W_3$-$T_1$, S(O)$_n$-$W_3$-$T_1$ et S(O)$_n$-U'-V' dans lesquels $R_c$, $W_2$, $W_3$, U, V, U', V', $T_1$ et n sont tels que définis dans la formule (I), pour conduire aux composés de formules (I/$o_1$), (I/$o_2$) et (I/$o_3$), cas particulier des composés de formule (I) :

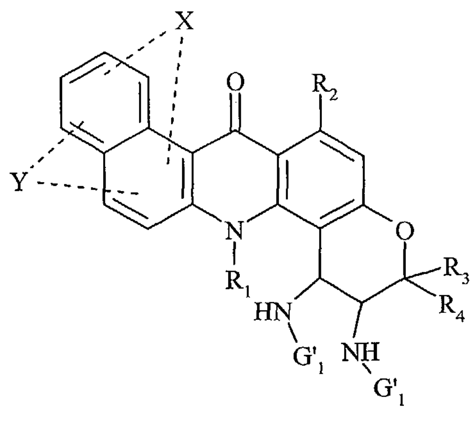

(I/$o_1$)

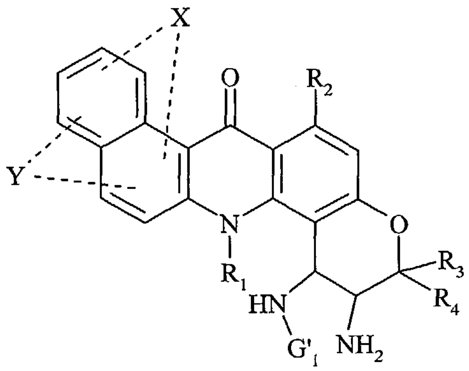

(I/o$_2$)

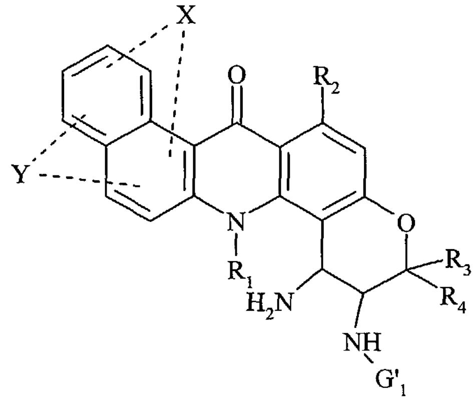

(I/o$_3$)

dans laquelle X, Y, $R_1$, $R_2$, $R_3$, $R_4$ et $G'_1$ sont tels que définis précédemment, composés de formules (I/o$_2$) et (I/o$_3$) dont la fonction amine primaire est protégée par un groupement protecteur des groupes amines primaires pour conduire aux composés de formules (XIII/a) et (XIII/b),

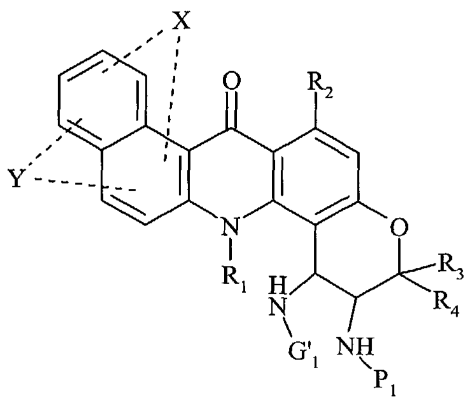

(XIII/a)

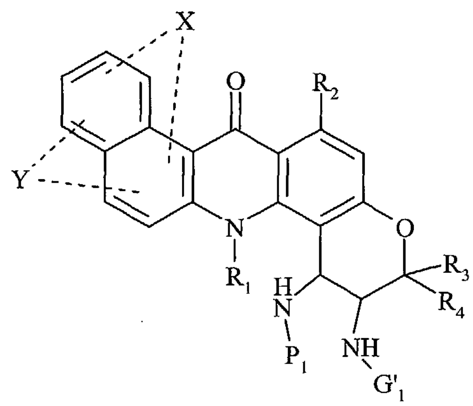

(XIII/b)

dans laquelle X, Y, $R_1$, $R_2$, $R_3$ $R_4$, $G'_1$ sont tels que définis précédemment et $P_1$ représente un groupement protecteur de fonctions amines primaires, composés de formules (I/o$_1$), (XIII/a) et (XIII/b) qui sont éventuellement soumis à l'action d'un composé de formule (XIV) :

$$R_{c1}\text{-Hal (XIV)}$$

dans laquelle Hal représente un halogène et $R_{c1}$ représente un groupement choisi parmi alkyle ($C_1$-$C_6$) linéaire ou ramifié, aryle ou arylalkyle ($C_1$-$C_6$) linéaire ou ramifié, puis qui sont soumis, dans le cas de composés de formule (XIII/a) et (XIII/b), à des conditions de déprotection de la fonction amine primaire, pour conduire aux composés de formules (I/p$_1$), (I/p$_2$) et (I/p$_3$), cas particuliers des composés de formule (I) :

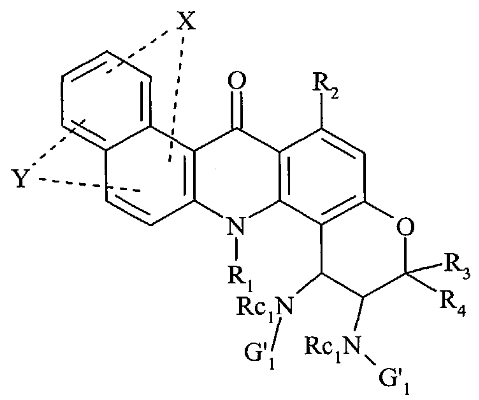

(I/$p_1$)

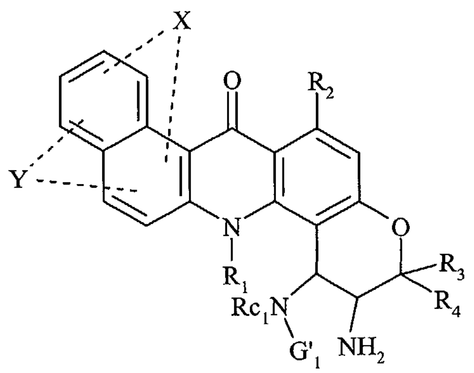

(I/$p_2$)

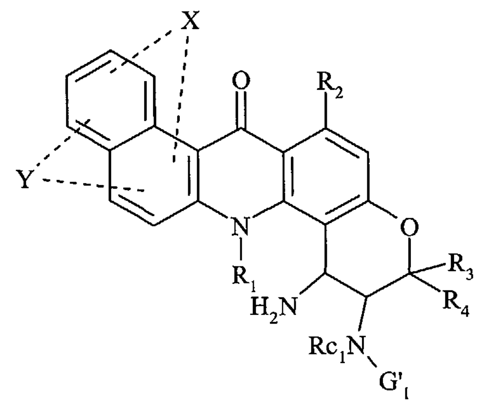

(I/$p_3$)

dans laquelle X, Y, $R_1$, $R_2$, $R_3$ $R_4$, $G'_1$ et $R_{c1}$ sont tels que définis précédemment, composés de formules (I/$p_2$) et (I/$p_3$) qui sont éventuellement soumis successivement à l'action d'un composé de formule (XIV) tel que défini précédemment puis d'un composé de formule (XV) :

$R_{d'1}$-Hal (XV)

dans laquelle Hal est tel que défini précédemment et $R_{d'1}$ peut prendre les même définitions que $R_{c1}$, pour conduire aux composés de formules (I/$q_2$) et (I/$q_3$), cas particuliers des composés de formule (I) :

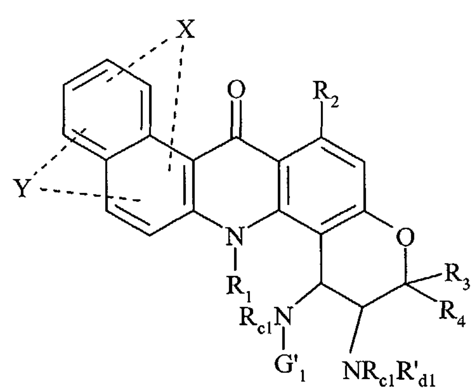

(I/$q_2$)

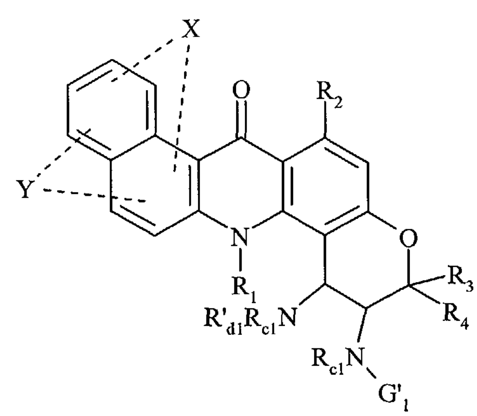

(I/$q_3$)

dans laquelle X, Y, $R_1$, $R_2$, $R_3$ $R_4$, G' $_1$, $R_{c1}$ et $R_{d1}$ sont tels que définis précédemment,

**composés de formule (I/I) pouvant être soumis :**

α) soit à l'action d'un composé de formule (XII) tel que défini précédemment, pour conduire aux composés de formule (I/$r_1$), (I/$r_2$) et (I/$r_3$), cas particuliers des composés de formule (I) :

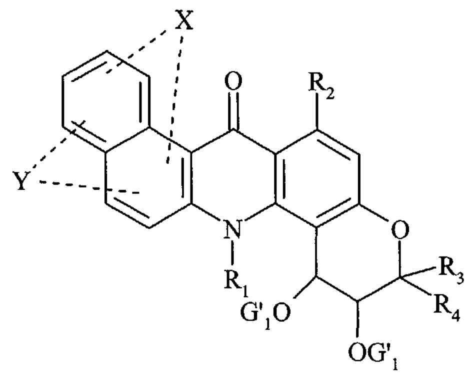

(I/$r_1$)

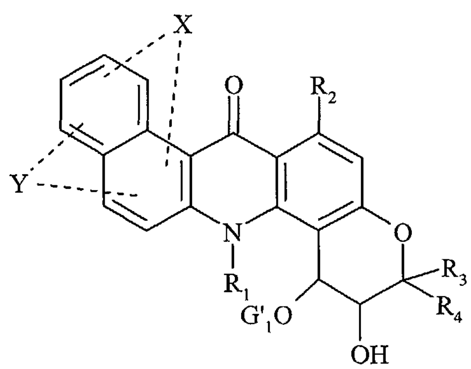

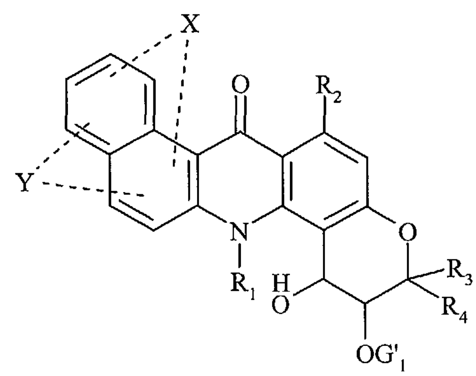

(I/$r_2$)

(I/$r_3$)

dans lesquelles X, Y, $R_1$, $R_2$, $R_3$, $R_4$ et G'$_1$ sont tels que définis précédemment,

*composés de formule (I/$r_1$) qui sont soumis:*

***1)*** soit à l'action, lorsque G'$_1$ représente un groupement C($W_2$)-U-V, d'un alcool de formule $R_{30}$-OH dans laquelle $R_{30}$ représente un groupement alkyle ($C_1$-$C_6$) linéaire ou ramifié, pour conduire aux composés de formule (I/$s_1$), cas particulier des composés de formule (I) :

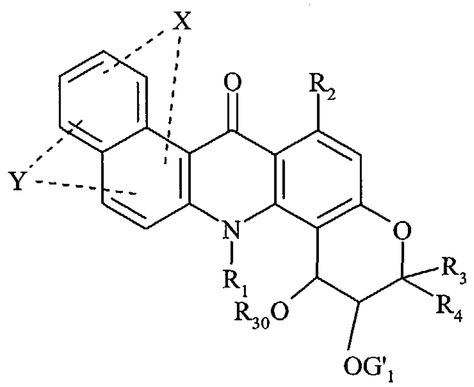

$(I/s_1)$

dans laquelle X, Y, $R_1$, $R_2$, $R_3$ $R_4$, $G'_1$ et $R_{30}$ sont tels que définis précédemment,

***2)*** soit à l'action, lorsque $G'_1$ représente un groupement $C(W_2)$-U-V, d'un thiol de formule (XVI) :

$$G_1S\text{-}H \quad (XVI)$$

dans laquelle $G_1$ représente un groupement choisi parmi $R_c$, $C(W_2)$-U-V, et $C(W_2)$-$W_3$-$T_1$ dans lesquels $R_c$, $W_2$, $W_3$, U, V et $T_1$ sont tels que définis dans la formule (I), pour conduire aux composés de formule $(I/t_1)$, cas particulier des composés de formule (I) :

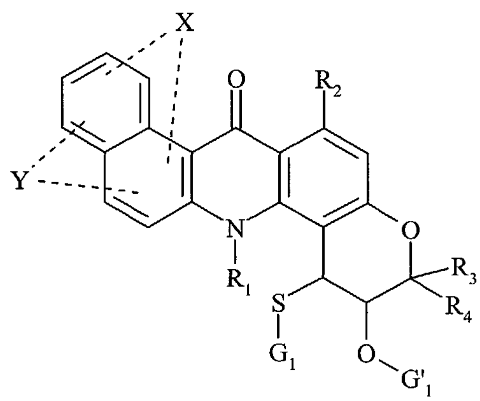

$(I/t_1)$

dans laquelle X, Y, $R_1$, $R_2$, $R_3$, $R_4$, $G_1$ et $G'_1$ sont tels que définis précédemment, composés de formule $(I/t_1)$ qui sont éventuellement traités par un agent de réduction, puis soumis à une réaction de déprotection de la fonction hydroxy, pour conduire aux composés de formule $(I/u_1)$, cas particulier des composés de formule (I) :

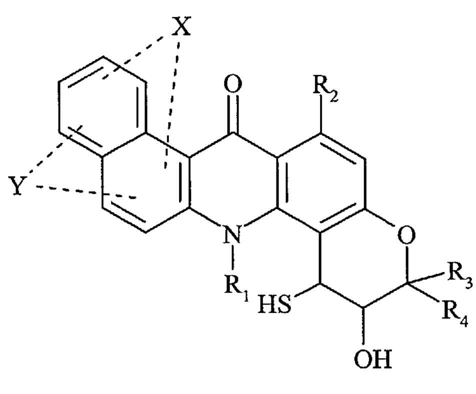

$(I/u_1)$

dans laquelle X, Y, $R_1$, $R_2$, $R_3$ et $R_4$ sont tels que définis précédemment,

*composés de formules (I/$r_2$) et (I/$r_3$)* qui sont soumis :

***1)*** soit à l'action d'un anhydride de formule (XVII) ou d'un chlorure d'acide de formule (XVIII) :

$$[V\text{-}U\text{-}C(W_2)]_2O \text{ (XVII) } Cl\text{-}C(W_2)\text{-}U\text{-}V \text{ (XVIII)}$$

dans lesquelles $W_2$, U et V sont tels que définis dans la formule (I), pour conduire aux composés de formules (I/$v_2$) et (I/$v_3$), cas particuliers des composés de formule (I) :

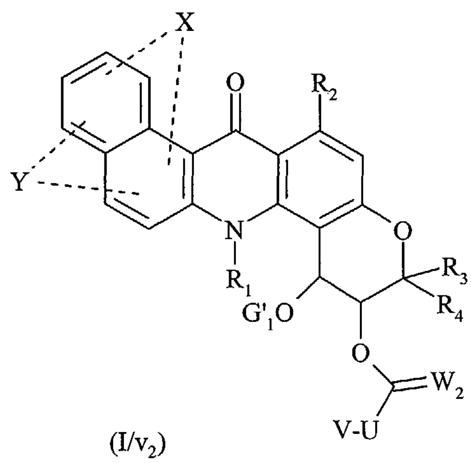

(I/$v_2$)

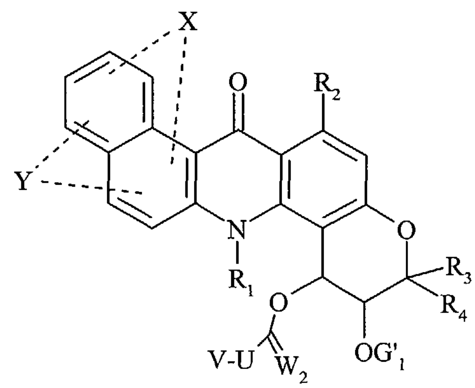

(I/$v_3$)

dans lesquelles X, Y, $R_1$, $R_2$, $R_3$, $R_4$, $G'_1$, $W_2$, U et V sont tels que définis précédemment,

***2)*** soit à des conditions de déshydratation en milieu acide, pour conduire aux composés de formule (I/$w_2$) et (I/$w_3$), cas particuliers des composés de formule (I):

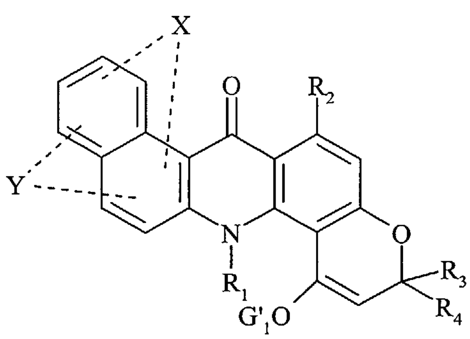

(I/$w_2$)

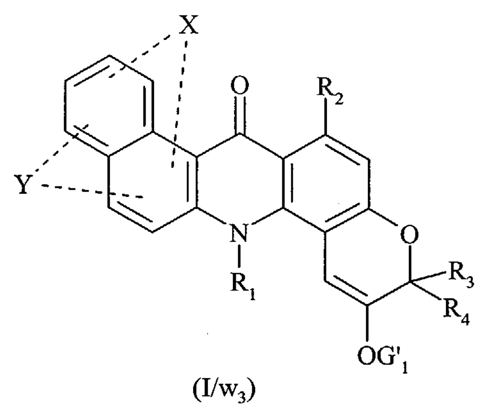

(I/$w_3$)

dans lesquelles X, Y, $R_1$, $R_2$, $R_3$, $R_4$ et $G'_1$ sont tels que définis précédemment,

β) soit à l'action d'un composé de formules (XIX) ou (XX)

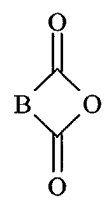 (XIX) 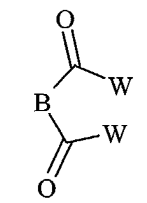 (XX)

dans laquelle B est tel que défini dans la formule (I) et W représente un atome d'halogène ou un groupement hydroxyle, pour conduire aux composés de formule (I/x), cas particulier des composés de formule (I):

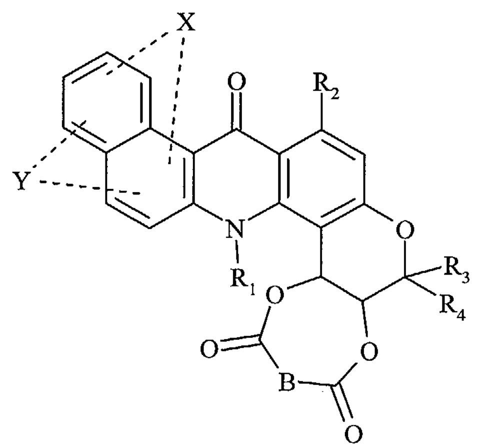

(I/x)

dans laquelle X, Y, $R_1$, $R_2$, $R_3$, $R_4$ et B sont tels que définis précédemment,

γ) soit à l'action d'un dihalogénure d'alkyle ($C_1$-$C_6$) linéaire, pour conduire aux composés de formule (I/y), cas particulier des composés de formule (I) :

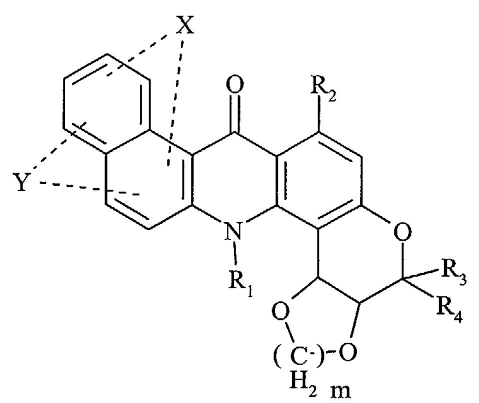

(I/y)

dans laquelle X, Y, $R_1$, $R_2$, $R_3$ et $R_4$ sont tels que définis précédemment, et m est tel que défini dans la formule (I),

δ) soit à l'action d'un équivalent d'un composé de formule (XVII) ou (XVIII), pour conduire aux composés de formule (I/z), cas particulier des composés de formule (I):

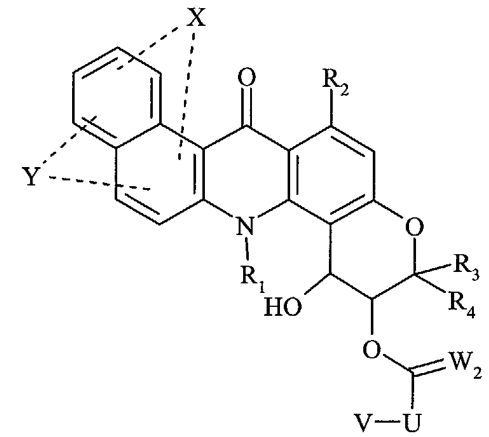

(I/z)

dans laquelle X, Y, $R_1$, $R_2$, $R_3$, $R_4$, $W_2$, U et V sont tels que définis précédemment, composés de formule (I/z) qui peuvent être soumis à un excès d'anhydride de formule (XVII') ou d'un chlorure d'acide de formule (XVIII')

$$[V_1\text{-}U_1\text{-}C(W_2)]_2O \text{ (XVII') } C1\text{-}C(W_2)\text{-}U_1\text{-}V_1 \text{ (XVIII')}$$

dans lesquelles $U_1$ et $V_1$ peuvent prendre les mêmes définitions que U et V et $W_2$ est tel que défini précédemment, pour conduire aux composés de formule (I/aa), cas particulier des composés de formule (I) :

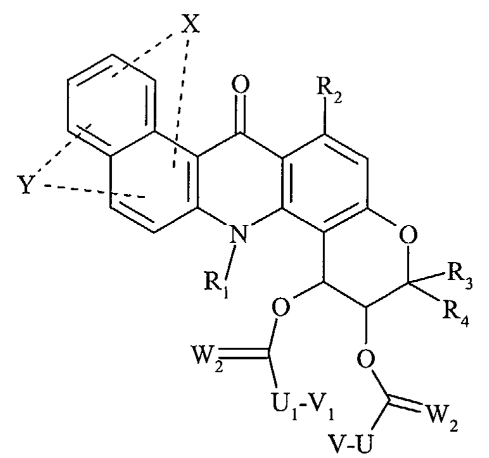

(I/aa)

dans laquelle X, Y, $R_1$, $R_2$, $R_3$, $R_4$, $W_2$, U, V, $U_1$ et $V_1$ sont tels que définis précédemment,

s) soit à des conditions de déshydratation en milieu acide, pour conduire aux composés de formule (I/ab), cas particulier des composés de formule (I) :

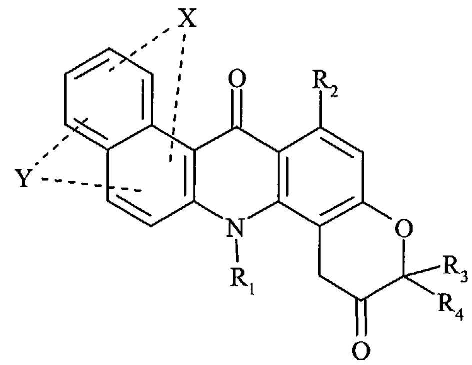

(I/ab)

dans laquelle X, Y, $R_1$, $R_2$, $R_3$ et $R_4$ sont tels que définis précédemment, composés de formule (I/ab) qui sont éventuellement réduits en présence de $NaBH_4$, pour conduire aux composés de formule (I/ac), cas particulier des composés de formule (I) :

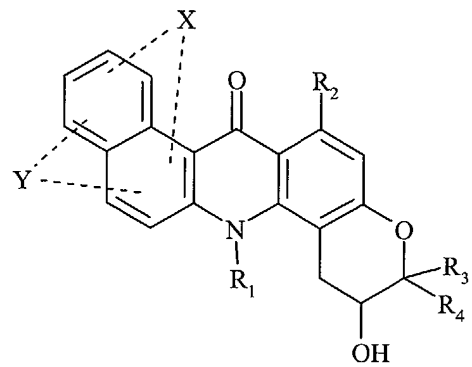

(I/ac)

dans laquelle X, Y, $R_1$, $R_2$, $R_3$ et $R_4$ sont tels que définis précédemment,

d) soit à l'action d'un péracide ou du diméthyle dioxirane, pour conduire aux composés de formule (I/ad), cas particulier des composés de formule (I) :

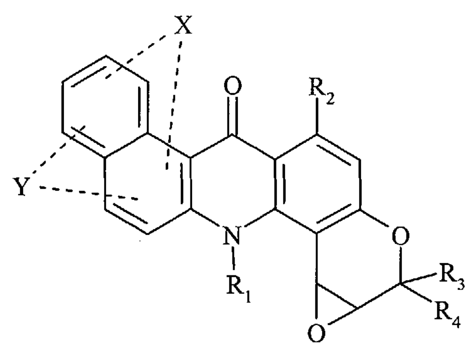

(I/ad)

dans laquelle X, Y, $R_1$, $R_2$, $R_3$ et $R_4$ sont tels que définis précédemment, composés de formule (I/ad) qui sont éventuellement traités par de l'ammoniac ou une amine primaire ou secondaire pour conduire aux composés de formules (I/$ae_1$) et (I/$ae_2$), cas particuliers des composés de formule (I) :

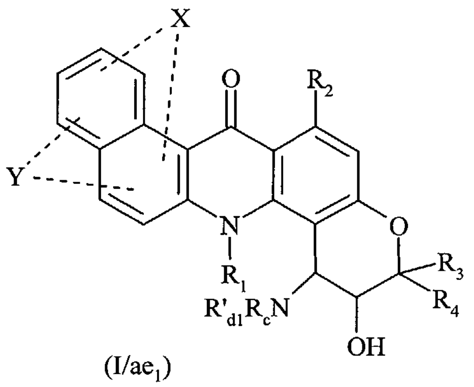

(I/ae$_1$)

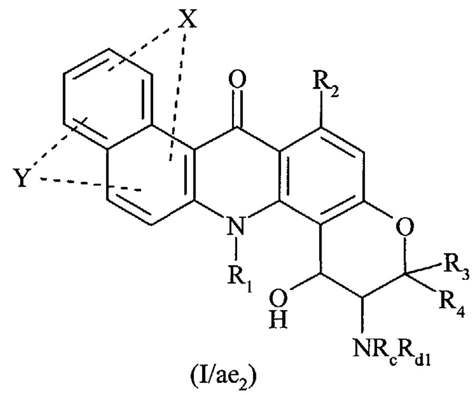

(I/ae$_2$)

dans laquelle X, Y, $R_1$, $R_2$, $R_3$ et $R_4$ sont tels que définis précédemment, $R_c$ est tel que défini dans la formule (I) et $R_{d1}$ représente un groupement choisi parmi $R_d$, - C($W_2$)-U-V, -C($W_2$)-$W_3$-$T_1$, -S(O)$_n$-$W_3$-$T_1$ et -S(O)$_n$-U'-V' dans lesquels $R_d$, $W_2$, $W_3$, U, V, $T_1$, U' et V' sont tels que définis précédemment,

**composés de formules (I/ae$_1$) et (I/ae$_2$) pouvant être soumis**

α) soit, dans le cas où $R_c$ et $Rd_1$ représentent un atome d'hydrogène, à l'action d'un composé de formule (XI) tel que défini précédemment, pour conduire aux composés de formules (I/af$_1$) et (I/af$_2$), cas particuliers des composés de formule (I) :

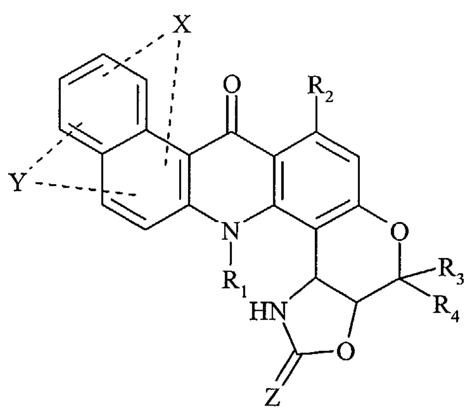

(I/af$_1$)

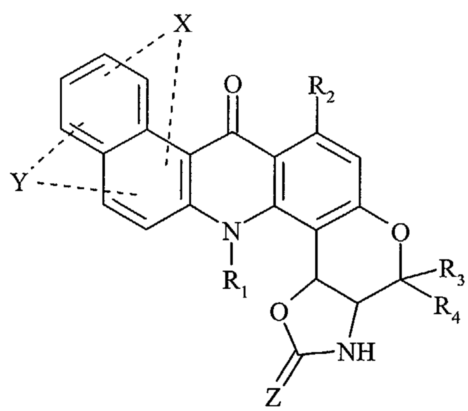

(I/af$_2$)

dans lesquelles X, Y, $R_1$, $R_2$, $R_3$, $R_4$ et Z sont tels que définis précédemment,

β) soit à l'action d'un composé de formules (XIX) ou (XX) tel que défini précédemment, pour conduire aux composés de formules (I/ag$_1$) et (I/ag$_2$), cas particuliers des composés de formule (I) :

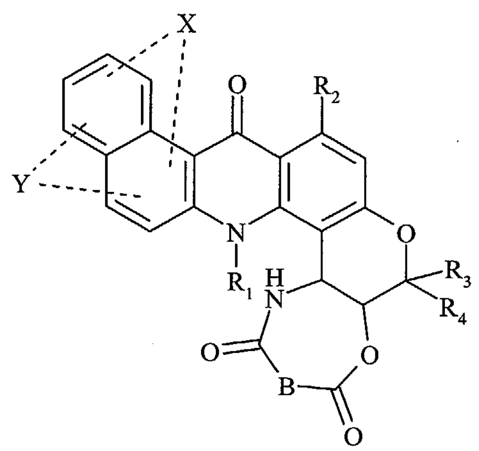

(I/ag₁)

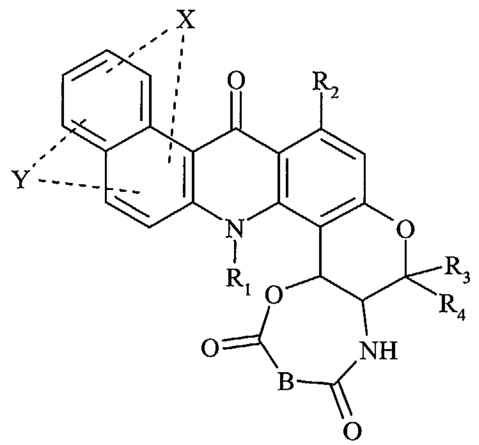

(I/ag₂)

dans lesquelles X, Y, $R_1$, $R_2$, $R_3$, $R_4$ et B sont tels que définis précédemment,

γ) soit à l'action d'un composé de formule (XII) tel que défini précédemment, pour conduire aux composés de formules (I/$ah_1$) et (I/$ah_2$), cas particuliers des composés de formule (I) :

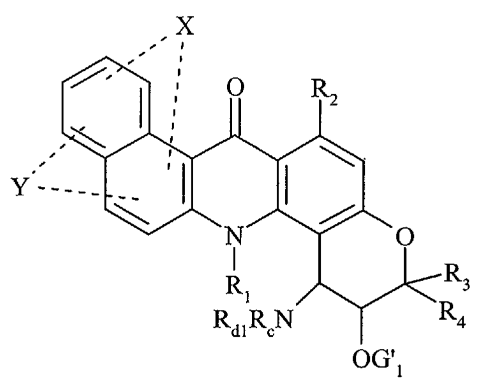

(I/$ah_1$)

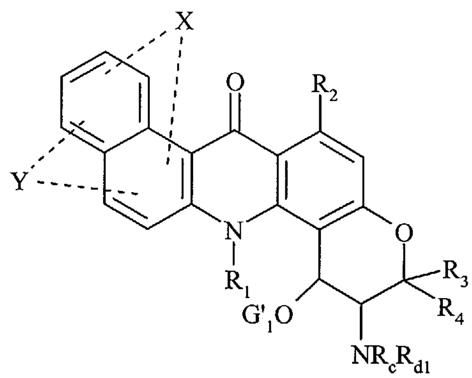

(I/$ah_2$)

dans lesquelles X, Y, $R_1$, $R_2$, $R_3$, $R_4$, $R_c$, $R_{d1}$ et $G'_1$ sont tels que définis précédemment,

δ) soit à l'action de dibromure de triphénylphosphine en présence de triéthylamine pour conduire aux composés de formules (I/ai), cas particulier des composés de formule (I) :

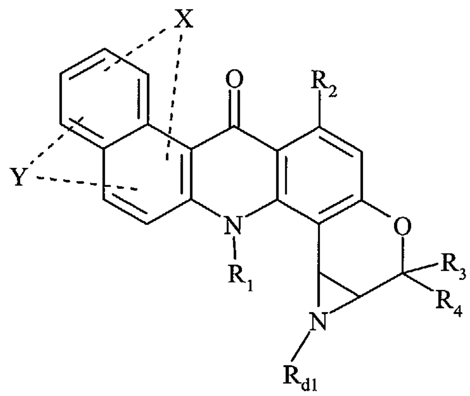

(I/ai)

dans laquelle X, Y, $R_1$, $R_2$, $R_3$, $R_4$ et $R_{d1}$ sont tels que définis précédemment,

ε) soit à des conditions de déshydratation en milieu acide, pour conduire aux composés de formules (I/$aj_2$) et (I/$aj_3$), cas particuliers des composés de formule (I) :

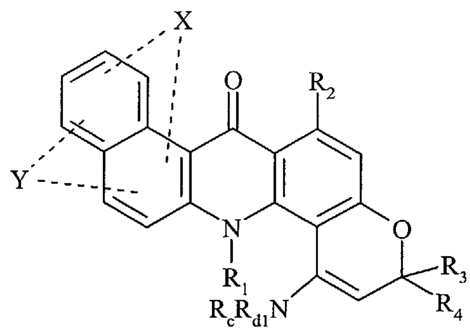

(I/$aj_2$)

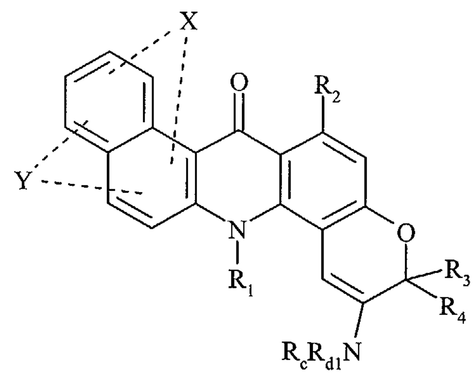

(I/$aj_3$)

dans laquelle X, Y, $R_1$, $R_2$, $R_3$ $R_4$, $R_c$ et $R_{d1}$ sont tels que définis précédemment,

e) soit à l'action d'un composé de formule (XXI) :

$$Hal\text{-}C(W_2)\text{-}T_1 \quad (XXI)$$

dans laquelle Hal représente un atome d'halogène et $W_2$ et $T_1$ sont tels que définis dans la formule (I), pour conduire aux composés de formule (I/aj), cas particulier des composés de formule (I) :

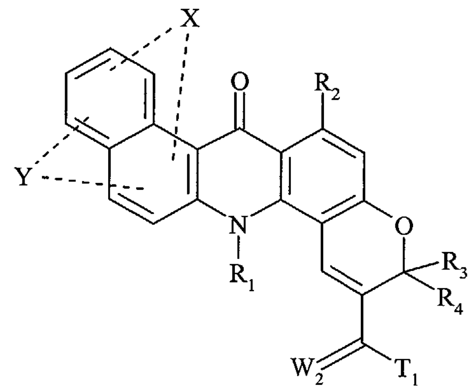

(I/aj)

dans laquelle X, Y, $R_1$, $R_2$, $R_3$ $R_4$, $W_2$ et $T_1$ sont tels que définis précédemment, les composés (I/a) à (I/aj) formant l'ensemble des composés de l'invention qui sont purifiés, le cas échéant, selon une technique classique de purification, qui peuvent, si on le désire, être séparés en leurs différents isomères selon une technique classique de séparation et qui sont transformés, si on le souhaite, en leurs N-oxydes et, le cas échéant, en leurs sels d'addition à un acide ou à une base pharmaceutiquement acceptable.

Les composés de formules (II), (IV), (IX) à (XII), (XIV) à (XXI), (XVIII') et (XVIII') sont soit des produits commerciaux, soit obtenus selon des méthodes classiques de la synthèse organique bien connues de l'homme du métier.

Les composés de formule (I) pour lesquels A représente un groupement de formule - $CH(R_5)$-$CH(R_6)$ dans laquelle $R_5$ et $R_6$, identiques ou différents, indépendamment l'un de l'autre, représentent chacun un groupement choisi parmi l'atome d'hydrogène, un groupement $OR_c$, $W_1$-$C(W_2)$-U-V dans lesquels $R_c$ est tel que défini dans la formule (I), $W_1$ et $W_2$ représentent un atome d'oxygène, U représente une chaîne alkylène ($C_1$-$C_8$) linéaire ou ramifiée et V représente un atome d'hydrogène ou $R_5$ et $R_6$ forment ensemble un groupement

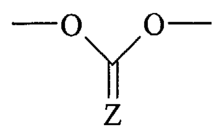

dans lequel Z est tel que défini dans la formule (I) peuvent avantageusement être obtenus à partir des composés de formule (I/e) :

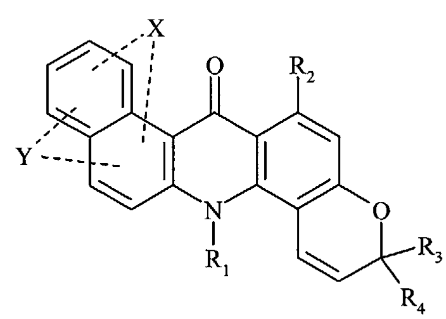

(I/e)

dans laquelle X, Y, $R_1$, $R_2$, $R_3$ et $R_4$ sont tels que définis précédemment, qui sont soumis à l'action de tétroxyde d'osmium en milieu polaire et en présence de N-oxyde de 4-méthylmorpholine, ou à l'action de permanganate de potassium suivi d'une réduction au borohydrure de sodium, pour conduire selon la méthode aux composés *cis*- ou *trans*-diol de formule (I/ak), cas particulier des composés de formule (I) :

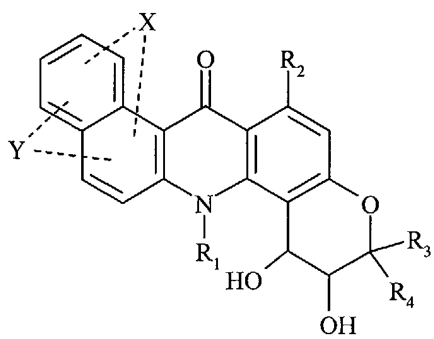

(I/ak)

dans laquelle X, Y, $R_1$, $R_2$, $R_3$ et $R_4$ sont tels que définis précédemment, composés *cis*-diol de formule (*cis*-I/ak) que l'on soumet éventuellement :

- ***soit à l'action*** de N,N'-carbonyldiimidazole ou de N,N'-thiocarbonyldiimidazole en présence de 2-butanone, pour conduire aux composés de formule (*cis*-I/al), cas particulier des composés de formule (I) :

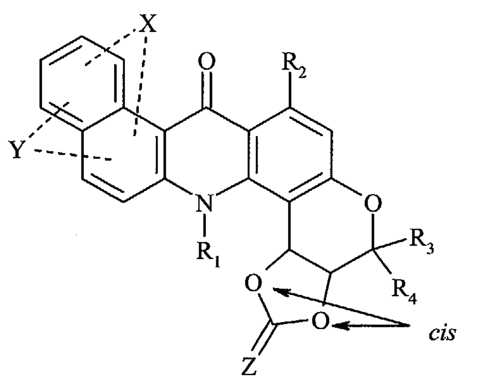

(*cis*-I/al)

dans laquelle X, Y, $R_1$, $R_2$, $R_3$ et $R_4$ sont tels que définis précédemment et Z est tel que défini dans la formule (I),

- ***soit à l'action*** d'un composé de formule (XXII) ou d'un composé de formule (XXIII) :

$$Hal\text{-}G_2 \text{ (XXII) ou } G_2\text{-O-}G_2 \text{ (XXIII)}$$

dans laquelle Hal représente un atome d'halogène et $G_2$ représente un groupement choisi parmi $-C(W_2)-U-V$, $-C(W_2)-W_3-T_1$, $-S(O)_n-W_3-T_1$ et $-S(O)_n-U'-V'$ dans lesquels $W_2$, $W_3$, U, V, U', V' et n sont tels que définis dans la formule (I), pour conduire aux composés de formule (I/am), cas particulier des composés de formule (I):

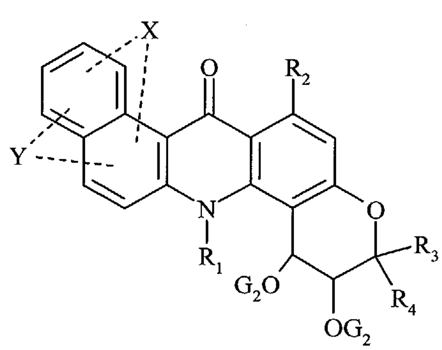

(I/am)

dans laquelle X, Y, $R_1$, $R_2$, $R_3$, $R_4$ et $G_2$ sont tels que définis précédemment, composé de formule (I/am), qui constitue

l'ensemble des composés de formule (I), que l'on purifie, le cas échéant, selon une technique classique de purification , qui peuvent, si on le désire, être séparés en leurs différents isomères selon une technique classique de séparation et que l'on transforme , éventuellement, en leurs N-oxydes et, le cas échéant, en leurs sels d'addition à un acide ou à une base pharmaceutiquement acceptable.

Les composés de formules (XXII) et (XXIII) sont soit des produits commerciaux, soit obtenus selon des méthodes classiques de la synthèse organique bien connues de l'homme du métier.

Les composés de formule (I) présentent des propriétés antitumorales particulièrement intéressantes. Ils ont une excellente cytotoxicité in vitro sur des lignées cellulaires, issues de tumeurs murines et humaines, due à un blocage spécifique du cycle cellulaire, et sont actifs in vivo, chez la souris, sur des tumeurs transplantables murines et humaines. Les propriétés caractéristiques de ces composés permettent leur utilisation en thérapeutique en tant qu'agents antitumoraux.

La présente invention a également pour objet les compositions pharmaceutiques contenant comme principe actif au moins un composé de formule (I), ses énantiomères, diastéréoisomères, N-oxydes ou un de ses sels d'addition à une base ou un acide pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 0,1 mg à 1000 mg en une ou plusieurs prises par jour.

Les exemples suivants illustrent l'invention mais ne la limitent en aucune façon.

Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples et dans les préparations ont été déterminées selon les techniques spectrophotométriques usuelles (infrarouge, résonance magnétique nucléaire, spectrométrie de masse, ...).

Les points de fusion ont été déterminés soit à la platine chauffante de Kofler, soit à la platine chauffante sous microscope. Lorsque le composé existe sous forme de sel, le point de fusion correspond à celui du produit salifié.

**PREPARATION 1 : N-(3,S-diméthoxphényl)acétamide**

Une solution de 15,3 g de 3,5-diméthoxyaniline et 57,5 ml d'anhydride acétique dans 40 ml de pyridine anhydre est chauffée à reflux pendant 1 heure. Le milieu réactionnel est ensuite refroidi et versé sur 350 ml d'eau. Le précipité formé est mis en suspension dans une solution aqueuse de $Na_2CO_3$ à 20%, puis filtré sur büchner, lavé à l'eau, séché sous vide phosphorique. Un précipité de 14,5 g du produit attendu est obtenu sous forme d'un solide amorphe blanc.

Spectre de masse ($DIC/NH_3$) : $m/z$ = 196 $(M+H)^+$

**PREPARATION 2: 9,11-Dihydroxybenzo[α]acridin-12(7*H*)-one**

**Stade A : Méthyl 2-méthoxy-1-naphthoate**

A une solution de 26,32 g d'acide 2-hydroxy-1-naphtoïque dans 70 ml d'une solution aqueuse de NaOH 4,5 *N* on additionne 54 ml de diméthylsulfate. Le milieu réactionnel est ensuite maintenu à température ambiante, sous agitation pendant 4h, dilué par 250 ml d'eau et extrait par le dichlorométhane (4×100 ml). Les phases organiques réunies sont lavées à l'eau, séchées sur sulfate de sodium et évaporées à sec sous pression réduite. Une chromatographie sur gel de silice (cyclohexane puis un gradient d'acétone dans le cyclohexane de 1 à 10 %) permet d'isoler 21,5 g du produit attendu.

Spectre de masse (DIC/$NH_3$) : $m/z$ = 217 $(M+H)^+$

**Stade B : Acide 2-méthoxy-1-naphtoïque**

Une solution de 21,6 g du composé du stade A précédent dans 48 ml d'une solution alcaline de Claisen (solution de 21 g de KOH dans 15 ml d'eau, complétée à 60 ml par du méthanol) est chauffée à reflux pendant 4 heures. Le milieu réactionnel est ensuite dilué par 50 ml d'eau et versé sur 75 ml d'une solution à 15,5% d'acide chlorhydrique refroidi à 0°C sous agitation. Le précipité blanc formé est alors filtré sur büchner puis, lavé à l'eau et séché sous vide phosphorique. Le résidu sec obtenu, est dissout dans une solution d'éthanol et eau (80:20, v/v) à ébullition. Après refroidissement à 0°C on obtient 13,7 g du produit attendu.

**Point de fusion : 56-57°C**

Spectre de masse (DIC/$NH_3$) : $m/z$ = 203 $(M+H)^+$

**Stade C : N-[3,5-diméthoxy-2-(2-méthoxy-1-naphthoyl)phényl]acétamide**

On additionne 30 ml de $SOCl_2$, goutte à goutte, pendant 30 minutes sur 15,15 g du composé du stade B précédent dans un ballon surmonté d'un réfrigérant et relié à un piège à gaz. Le milieu réactionnel est ensuite chauffé à 60°C pendant 3 heures et évaporé à sec sous pression réduite. Le chlorure de l'acide de 2-méthoxy-1-naphtoïque ainsi obtenu est mis en solution dans 50 ml de dichloroéthane anhydre et additionné, goutte à goutte, à un mélange de 12,5 g d'$AlCl_3$ et de 12,7 g du composé de la préparation 1 dans 100 ml de dichloroéthane anhydre préalablement refroidi à 0°C. Le milieu réactionnel est maintenu à 0°C pendant 3 heures puis à 20°C pendant 3 heures. Le milieu réactionnel est alors versé sur 250 ml d'une solution à 15% d'acide chlorhydrique préalablement refroidi dans un bain de glace sous agitation, et extrait par le dichlorométhane (3×40 ml). Les phases organiques réunies sont lavées avec une solution de $NaHCO_3$ puis à l'eau, séchées sur sulfate de sodium anhydre, filtrées et évaporées à sec sous pression réduite. Une chromatographie sur gel de silice (dichlorométhane puis un gradient de méthanol dans le dichlorométhane de 0,2 à 4%) permet d'isoler 7,74 g du produit attendu.

**Point de fusion : 149-150°C**

Spectre de masse (DIC/$NH_3$) : $m/z$ = 379 $(M+H)^+$

**Stade D : 9,1 1-Diméthoxybenzo[α]acridin-12(7*H*)-one**

Une solution de 3,79 g du composé du stade C précédent dans 60 ml de diméthylformamide anhydre est additionnée, goutte à goutte, à une suspension de 1,2 g de NaH dans 50 ml de diméthylformamide anhydre préalablement refroidi à 0°C. Le milieu réactionnel est maintenu à 0°C sous agitation et sous atmosphère inerte pendant 15 minutes et ensuite maintenu à 20°C pendant 4h30. Le milieu réactionnel est alors versé sur 200 ml d'eau et extrait par l'acétate d'éthyle (3×50 ml). Les phases organiques réunies sont lavées successivement par une solution de NaOH

puis à l'eau, séchées sur sulfate de sodium anhydre, filtrées puis évaporées à sec sous pression réduite. Une chromatographie sur gel de silice (dichlorométhane puis un gradient de méthanol dans le dichlorométhane de 0,5 à 5%) permet d'isoler 2,05 g du produit attendu.

**Point de fusion : 258-259°C**

Spectre de masse ($DIC/NH_3$) : $m/z$ = 305 $(M+H)^+$

**Stade E : 9,11-Dihydroxybenzo[α]acridin-12(7*H*)-one**

Une solution de 1,89 g du composé du stade D précédent dans 90 ml d'acide acétique est additionnée de 80 ml d'une solution aqueuse à 48% de HBr puis chauffée à reflux pendant 4 jours. Après refroidissement, le milieu réactionnel est versé sur 1000 ml d'eau glacée. Le précipité brun formé est filtré sur büchner, lavé à l'eau et séché sous vide phosphorique. Une chromatographie sur gel de silice (dichlorométhane puis un gradient de méthanol dans le dichlorométhane de 1 % à 10 %) permet d'isoler 1,55 mg du produit attendu.

**Point de fusion : 311-312°C**

Spectre de masse ($DIC/NH_3$) : $m/z$ = 278 $(M+H)^+$

**PREPARATION 3 : N-(3-méthoxyphényl)acétamide**

Le produit est obtenu selon le procédé de la préparation 1 en utilisant la 3-méthoxyaniline à la place de la 3,5-diméthoxyaniline.

**PREPARATION 4 : 9-Hydroxybenzo[α]acridin-12(7*H*)-one**

**Stade A :N-[5-méthoxy-2-(2-méthoxy-1-naphthoyl)phényl]acétamide**

Le produit est obtenu selon le procédé du stade C de la préparation 2 en utilisant le composé de la préparation 3 à la place du composé de la préparation 1.

**Stade B : 9-Méthoxybenzo[α]acridin-12(7*H*)-one**

Le produit est obtenu selon le procédé du stade D de la préparation 2 en utilisant le composé du stade A précédent.

**Stade C : 9-Hydroxybenzo[α]acridin-12(7*H*)-one**

Le produit est obtenu selon le procédé du stade E de la préparation 2 en utilisant le composé du stade B précédent.

**PREPARATION 5: 3-Bromo,-9,11-dihydroxybenzo[α]acridin-12(7*H*)-one**

Le produit est obtenu selon le procédé de la préparation 2, stade A au stade E en utilisant l'acide 6-bromo-2-hydroxy-1-naphtoïque à la place de l'acide 2-hydroxy-1-naphtoïque.

Spectre de masse ($ESI^+$): $m/z$ = 354 $(M+H)^+$

**EXEMPLE 1 : 6-Hydroxy-3,3-diméthyl-3,14-dihydro-7*H*-benzo[α]pyrano-[3,2-*h*] acridin-7-one**

On dissout 732 mg du composé de la préparation 2 dans 20 ml de diméthylformamide anhydre, puis on ajoute ensuite 732 mg de carbonate de potassium anhydre. Le mélange ainsi obtenu est laissé sous agitation sous argon, à 65°C pendant 15 minutes, puis on additionne 876,5 mg d'iodure de potassium anhydre et 2,47 g de 3-chloro-3-méthyl-1-butyne. Au bout de 5 heures, le milieu réactionnel est porté à 130°C pendant 2 heures pour obtenir le réarrangement de l'éther propargylique. Le milieu réactionnel est ensuite dilué par 50 ml d'eau et est extrait par le dichlorométhane (3x40 ml). Les phases organiques réunies sont lavées à l'eau, puis par une solution de potasse 1M, séchées sur sulfate de sodium anhydre, filtrées puis évaporées à sec sous pression réduite. Une chromatographie sur gel de silice (cyclohexane puis un gradient d'acétone dans le cyclohexane de 1 à 5 %) permet d'isoler 326 mg du produit attendu.

Spectre de masse (DIC/$NH_3$) :m/z = 345 (M+ H)$^+$

**EXEMPLE 2 : 6-Hydroxy-3,3,14-triméthyl-3,14-dibydro-7*H*-benzo[α]pyrano[3,2-*h*] acridin-7-one**

A 411,6 mg du composé de l'exemple 1 en solution dans 50 ml d'acétone anhydre sont ajoutés 1,324 g de carbonate de sodium. Le mélange est maintenu sous agitation sous argon et à 0°C pendant 15 min., puis après addition de 852 mg d'iodure de méthyle, le milieu réactionnel est chauffé à reflux pendant 2 heures. Après refroidissement, l'excès d'iodure de méthyle est détruit par addition de 40 ml de méthanol et 50 ml d'eau. Le méthanol est éliminé par distillation et la phase aqueuse est extraite par le dichlorométhane (3x30 ml). Les phases organiques réunies sont lavées avec une solution aqueuse de NaOH à 10%, puis à l'eau, séchées sur sulfate de sodium anhydre puis filtrées et évaporées à sec sous pression réduite. Une chromatographie obtenu sur gel de silice (cyclohexane puis un gradient d'acétone dans le cyclohexane de 0,5 à 5 %) permet d'isoler 374,2 mg du produit attendu.

Spectre de masse (DIC/$NH_3$) : *m*/*z* = 358 (M+ H)$^+$

**EXEMPLE 3 : 6-Méthoxy-3,3,14-triméthyl-3,14-dihydro,7*H*-benzo[α]pyrano[3,2-*h*] acridin-7-one**

Une solution de 604,3 mg du composé de l'exemple 2 dans 40 ml d'acétone anhydre est ajoutée petit par petit à une suspension, maintenue sous argon et à 0°C, de 61 mg d'hydrure de sodium dans 10 ml d'acétone. Le mélange ainsi obtenu est maintenu à 0°C pendant 30 min puis on additionne 1,2 g d'iodure de méthyle. Le milieu réactionnel est chauffé à reflux pendant 6 heures. Après refroidissement, l'excès d'iodure de méthyle est détruit par addition de 40 ml de méthanol et 50 ml d'eau. Le méthanol est éliminé par distillation et la phase aqueuse est extraite par le dichlorométhane (3x30 ml). Les phases organiques réunies sont lavées avec une solution aqueuse de NaOH à 10 %, puis à l'eau, séchées sur sulfate de sodium anhydre puis filtrées et évaporées à sec sous pression réduite. Une chromatographie sur gel de silice (cyclohexane puis un gradient d'acétone dans le cyclohexane de 1 à 10 %) permet d'isoler 469,5 mg du produit attendu.

Spectre de masse (DIC/$NH_3$): *m*/*z* = 372 (M+ H)$^+$

**EXEMPLE 4 : (±)-*cis*-1,2-Dihydroxy-6-méthoxy-3,3,14-triméthyl-1,2,3,14-tétrahydro-7*H*-benzo[α]pyrano[3,2-*h*] acridin-7-one**

Un mélange de 0,4859 g du composé de l'exemple 3, de tétroxyde d'osmium (2,5 %) en solution dans 1,05 ml de 2-méthyl-2-propanol et de 96,7 mg de monohydrate de *N*-oxyde de 4-méthylmorpholine est mise en solution dans 40 ml d'un mélange de *t*BuOH-THF-$H_2O$ (10 : 3 : 1). Le milieu réactionnel est maintenu sous agitation à température ambiante pendant 4 jours. Une solution saturée de $NaHSO_3$ (30 ml) est alors ajoutée. Après agitation pendant une heure, le milieu réactionnel est extrait par le dichlorométhane (4×25 ml). Les phases organiques réunies sont séchées sur sulfate de sodium anhydre puis filtrées et évaporées à sec sous pression réduite. Une chromatographie sur gel de silice (cyclohexane puis un gradient d'acétone dans le cyclohexane de 1 à 15%) permet d'isoler 452,7 mg du produit attendu.

Spectre de masse (DIC/$NH_3$) : *m*/*z* = 406 (M+ H)$^+$

**EXEMPLE 5 : (±)-*cis*-1,2-Diacétoxy-6-méthoxy-3,3,14-triméthyl-1,2,3,14-tétrahydro *7H*-benzo[α]pyrano[3,2-*h*] acridin-7-one**

61 mg du composé de l'exemple 4 et 1 mg de DMAP sont ajoutées à un mélange préalablement refroidi (par un bain de glace) de pyridine anhydre (4 ml) et d'anhydride acétique (0,4 ml). Le milieu réactionnel est maintenu sous agitation à température ambiante pendant 3 jours, à l'abri de la lumière. Le milieu réactionnel est ensuite versé sur 10 ml d'$H_2O$ glacée, le précipité formé est filtré, lavé par $H_2O$ (2×5 ml) puis séché sous vide phosphorique. 62,4 mg du produit attendu cristallise dans un mélange dichlorométhane-acétate d'éthyle (9 : 1, v/v) sous forme de fins prismes blancs.

Point de fusion : 161-162°C

Spectre de masse (DIC/$NH_3$) : $m/z$ = 477 $(M+H)^+$

**EXEMPLE 6 : (±)-*cis*-7-Méthoxy-4,4,15-triméthyl-15,15c-dihydro-4*H*-benzo[α][1,3] dioxolo[4',5':4,5] pyrano [3,2-*h*]acridin-2,8[3a*H*]-dione**

A une solution de 109,4 mg du composé de l'exemple 4 dans 5 ml de 2-butanone sont additionnés 230,5 mg de *N,N'*-carbonyldiimidazole. Le milieu réactionnel est maintenu à reflux pendant 3 heures sous argon puis, après refroidissement, dilué par une solution aqueuse à 5 % de $Na_2CO_3$ (7 ml) et extrait par l'acétate d'éthyle (3×10 ml). Les phases organiques réunies sont séchées sur sulfate de sodium anhydre puis filtrées et évaporées à sec sous pression réduite. Une chromatographie sur gel de silice (dichlorométhane puis un gradient d'acétone dans le dichlorométhane de 1 à 7 %) permet d'isoler 65,7 mg du produit attendu.

Spectre de masse (DIC/$NH_3$) : $m/z$ = 432 $(M+H)^+$

**EXEMPLE 7 : (±)-*cis*-1-{[(Diméthylamino)carbonyl]oxy-6-méthoxy-3,3,14-triméthyl-7-oxo-2,3,7,14-tétrahydro-1*H*-benzo[α]pyrano[3,2-*h*] acridin-2-yl diméthylcarbamate**

Ajouter à -10°C une solution de 0,123 mmol du composé de l'exemple 4 dans 4 ml de tétrahydrofurane anhydre a 0,698 mmol d'hydrure de potassium lavé à l'hexane. Après addition goutte à goutte à -10°C de 0,327 mmol de chlorure de *N,N*-diméthylcarbamoyle, l'agitation est maintenue 3h30 à température ambiante. Après addition de 50 ml d'acétate d'éthyle et de 10 ml d'une solution saturée en $NaHCO_3$, la phase organique est lavée à l'eau et séchée sur sulfate de magnésium puis évaporée sous pression réduite pour conduire au produit attendu.

**EXEMPLE 8 : (±)-*cis*-6-Méthoxy-3,3,14-triméthyl-2-{[(4-méthylphényl)sulfonyl] oxy}-7-oxo-2,3,7,14-tétrahydro-1*H*-benzo[α]pyrano[3,2-*h*]acridin - 1-yl 4-méthylbenzènesulfonate**

Le produit est obtenu selon le procédé de l'exemple 7 en utilisant le chlorure de tosyle à la place du chlorure de *N,N*-diméthylcarbamoyle.

**EXEMPLE 9 : Acide(±)-*cis*-{[1-(acétyloxy)-6-méthoxy-3,3,14-triméthyl-7-oxo-2.3,7,14-tétrahydro-1*H*-benzo[α] pyrano[3,2-*h*]acridin-2-yl]oxy}-4-oxobutanoïque**

A une solution de 0,5 mmol du composé de l'exemple 4 dans 3 ml de pyridine anhydre sont ajoutés 1,1 équivalents d'anhydride succinique et 1 mg de diméthylaminopyridine. Agiter 2 jours et dans l'obscurité à température ambiante puis ajouter 25 ml d'anhydride acétique à - 15°C et agiter pendant 1,5 heures avant de concentrer sous pression réduite. Une chromatographie sur gel de silice (dichlorométhane/acide acétique : 99/1) permet d'isoler le produit attendu.

**EXEMPLE 10 : Acide(±)-*cis*-5-{[(1-(acétyloxy)-6-méthoxy-3,3,14-triméthyl-7-oxo-2,3,7,14-tétrahydro-1*H*-benzo [α]pyrano[3,2-*h*]acridin-2-yl]oxy}-5-oxopentanoïque**

Le produit est obtenu selon le procédé de l'exemple 9 en utilisant l'anhydride glutarique à la place de l'anhydride succinique.

**EXEMPLE 11 : (±)-*cis*-1-(Acétyloxy)-6-méthoxy-3,3,14-triméthyl-7-oxo,-2,3,7,14-tétrahydro-1*H*-benzo[α]pyrano[3,2-*h*]acridin-2-yl-[(tert-butoxycarbonyl)amino]acétate**

Ajouter lentement 0,6 mmol de dicyclohexylcarbodiimide à une solution à 0°C de 0,5 mmol du composé de l'exemple 4 et 0,5 mmol d'acide 2-[(*tert*-butoxycarbonyl)amino]acétique dans 10 ml de diméthylformamide. Le milieu réactionnel est maintenu 5 heures à 0°C puis 16 heures à température ambiante. Après filtration et évaporation sous pression réduite, le résidu est mis en solution dans 2 ml de pyridine anhydre, additionné de 2 ml d'anhydride acétique, et agité à température ambiante et à l'obscurité pendant 48 heures. Après concentration sous pression réduite du milieu réactionnel, une chromatographie sur gel de silice du résidu (dichlorométhane) permet d'isoler le produit attendu.

**EXEMPLE 12 : (±)-*cis*-1-(Acétyloxy)-6-méthoxy-3,3,14-triméthyl-7-oxo-2,3,7,14-tétrahydro-1*H*-benzo[α]pyrano[3,2-*h*]acridin-2-yl aminoacétate**

Ajouter 0,14 µl d'iodotriméthylsilane à une solution à température ambiante de 0,1 mmol du composé de l'exemple 11 dans 1 ml de chloroforme. Le milieu réactionnel est agité 5 mn à température ambiante puis évaporé à sec sous pression réduite. Une chromatographie sur gel de silice (dichlorométhane/méthanol : 85/15) permet d'isoler le produit attendu.

**EXEMPLE 13: 2-Butyryl-6-méthoxy-3,3,14-triméthyl-3,14-dihydro-7*H*-benzo[α]pyrano[3,2-*h*]acridin-7-one**

Un mélange de 0,81 mmol de chlorure de butyryle et 0,673 mmol d'$AlCl_3$ dans 2 ml de dichlorométhane anhydre est ajouté par petites portions à 0,135 mmol du produit de l'exemple 3 dans 2 ml de dichlorométhane à 0°C. Le milieu réactionnel est agité 4 heures à température ambiante, puis versé sur une solution d'HCl à 10%. Après traitement classique des phases organiques et évaporation sous pression réduite de celles-ci, une chromatographie sur gel de silice du résidu (dichlorométhane/méthanol) permet d'isoler le produit attendu.

**EXEMPLE 14 : *(±)-cis* Butyrate de 1-hydroxy-6-méthoxy-3,3,14-triméthyl-7-oxo-2,3,7,14-tétrahydro-1*H*-benzo[α]pyrano[3,2-*h*]acridin-2,yl**

Additionner 2 équivalents de chlorure de butyryle à une solution de 0,74 mmol du composé de l'exemple 4 en présence de 4-diméthylaminopyridine dans 7 ml de pyridine anhydre. Agiter à température ambiante pendant 72 heures puis ajouter 5 équivalents de chlorure de butyryle et reprendre l'agitation 72 heures, puis évaporés à sec. Une chromatographie sur gel de silice permet d'isoler le produit attendu.

**EXEMPLE 15: Butyrate de 6-méthoxy-3,3,14-triméthyl-7-oxo-7,14-dihydro-3*H*-benzo[α]pyrano[3,2-*h*]acridin-2-yl**

Ajouter 4 gouttes d'une solution d'HCl à 10% à une solution de 0,29 mmol du composé de l'exemple 14 dans 6 ml de dichlorométhane. Le milieu réactionnel est agité 3 jours à température ambiante, puis séché et concentré sous pression réduite. Une chromatographie sur gel de silice du résidu (dichlorométhane/méthanol) permet d'isoler le produit attendu.

**EXEMPLE 16 : 2-Hydroxy-6-méthoxy-3,3,14-triméthyl-2,3-dihydro-1*H*-benzo[α]pyrano[3,2-*h*]acridin-1,7(14*H*)-dione**

Ajouter goutte à goutte pendant 30 minutes une suspension de 1,28 g de $KMnO_4$ dans 15 ml d'eau à une solution de 0,5 g du produit de l'exemple 3 dissous dans 25 ml d'acétone. Le milieu réactionnel est agité à température ambiante pendant 8 heures, puis après extraction et traitement classique le produit attendu est isolé par chromatographie sur gel de silice du résidu (dichlorométhane/méthanol : 98/2).

**EXEMPLE 17 : Acétate de 6-méthoxy-3,3,14-triméthyl-1,7-dioxo-2,3,7,14-tétrahydro-1*H*-benzo[α]pyrano[3,2-*h*]acridin-2-yl**

Le produit est obtenu selon le procédé de l'exemple 14 à partir du composé de l'exemple 16 en utilisant l'anhydride acétique à la place du chlorure de butyryle.

**EXEMPLE 18 : 3,3-Diméthyl-3,14-dihydro-7*H*-benzo[α]pyrano[3,2-*h*]acridin-7-one**

Le produit est obtenu selon le procédé de l'exemple 1 en utilisant le composé de la préparation 4 à la place du composé de la préparation 2.

**EXEMPLE 19 : 3,3,14-Triméthyl-3,14-dihydro-7*H*-benzo[α]pyrano[3,2-*h*]acridin-7-one**

Le produit est obtenu selon le procédé de l'exemple 2 en utilisant le composé de l'exemple 18 à la place du composé de l'exemple 2.

**EXEMPLE 20 : (±)-*cis*-1,2-Dihydroxy-3,3,14-triméthyl-1,2,3,14-tétrabydro-7*H*-benzo[α]pyrano[3,2-*h*]acridin-7-one**

Le produit est obtenu selon le procédé de l'exemple 4 en utilisant le composé de l'exemple 19 à la place du composé de l'exemple 3.

**EXEMPLE 21 : (±)-*cis*-1-(Acétyloxy)-3,3,14-triméthyl-7-oxo-2,3,7,14-tétrahydro-1*H*-benzo[α]pyrano[3,2-*h*]acridin-2-yl acetate**

Le produit est obtenu selon le procédé de l'exemple 5 en utilisant le composé de l'exemple 20 à la place du composé de l'exemple 4.

**EXEMPLE 22 : Acétate de 1-hydroxy-6-méthoxy-3,3,14-triméthyl-7-oxo-2,3,7,14-tétrahydro-*1H*-benzo[α]pyrano[3,2-*h*]acridin-2-yl**

A une solution refroidie à 0°C de 2 mmol du composé de l'exemple 4 dans 5 ml de pyridine anhydre sont ajoutés 2,2 mmol d'anhydride acétique. Après agitation à température ambiante pendant 3 heures, le milieu réactionnel est concentré sous pression réduite. Une chromatographie sur gel de silice (dichlorométhane, puis dichlorométhane/méthanol : 99/1) permet d'isoler le produit attendu.

**EXEMPLE 23 : Benzoate, de 1-(acétyloxy)-6-méthoxy-3,3,14-triméthyl-7-oxo-2,3,7,14-tétrahydro-1*H*-benzo[α]pyrano[3,2-*h*]acridin-2-yl**

Le produit est obtenu selon le procédé de l'exemple 9 en utilisant l'anhydride benzoïque à la place de l'anhydride succinique.

**EXEMPLE 24: Propionate de 1-hydroxy-6-méthoxy-3,3,14-triméthyl-7-oxo-2,3,7,14-tétrahydro-1*H*-benzo[α]pyrano[3,2-*h*] acridin-2-yl**

Le produit est obtenu selon le procédé de l'exemple 22 en utilisant l'anhydride propionique à la place de l'anhydride acétique.

**EXEMPLE 25 : Propionate de 6-méthoxy-3,3,14-triméthyl-7-oxo-1-(propionyloxy)-2,3,7,14-tétrahydro-1*H*-benzo[a]pyrano[3,2-*h*]acridin-2-yl**

Le produit est obtenu selon le procédé de l'exemple 5 en utilisant l'anhydride propionique à la place de l'anhydride acétique.

**EXEMPLE 26: : 4-Pentenoate de 1-hydroxy-6-méthoxy-3,3,14-triméthyl-7-oxo-2,3,7,14-tétrahydro-1*H*-benzo[α]pyrano[3,2-*h*]acridin-2-yl**

Le produit est obtenu selon le procédé de l'exemple 14 en utilisant l'anhydride penténoïque à la place du chlorure de butyryle.

**EXEMPLE 27 : 4-Pentenoate de 6-méthoxy-3,3,14-triméthyl-7-oxo-1-(4-pentenoyloxy)-2,3,7,14-tétrahydro-1*H*-benzo[α]pyrano[3,2-*h*] acridin-2-yl**

Le produit est obtenu selon le procédé de l'exemple 14 en utilisant l'anhydride penténoïque à la place du

chlorure de butyryle. Une chromatographie sur gel de silice (dichlorométhane, puis dichlorométhane/méthanol : 99/1 à 98/2) permet d'isoler le produit attendu.

**EXEMPLE 28: 4-Pentenoate de 1-(acétyloxy)-6-méthoxy-3,3,14-triméthyl-7-oxo-2,3,7,14-tétrahydro-1*H*-benzo[α]pyrano[3,2-*h*]acridin-2-yl**

Le produit est obtenu selon le procédé de l'exemple 9 en utilisant l'anhydride penténoïque à la place de l'anhydride succinique.

**EXEMPLE 29 : 3-Méthylbutanoate de 6-méthoxy-3,3,14-triméthyl-1-[(3-méthylbutanoyl)oxy]-7-oxo-2,3,7,14-tétrahydro-1*H*-benzo[α]pyrano[3,2,*h*]acridin-2-yl**

Le produit est obtenu selon le procédé de l'exemple 5 en utilisant le chlorure d'isovaléryle à la place de l'anhydride acétique.

**EXEMPLE 30: Acide 4-{[1-(acétyloxy)-6-méthoxy-3,3,14-triméthyl-7-oxo-2,3,7,14-tétrabydro-1*H*-benzo[α]pyrano[3,2-*h*]acridin-2-yl]oxy}-4-oxobutanoique**

Le produit est obtenu selon le procédé de l'exemple 9 avec un excès d'anhydride succinique. Une chromatographie sur gel de silice (dichlorométhane, puis dichlorométhane/méthanol : 99/1) permet d'isoler le produit attendu.

**EXEMPLE 31 : Acide 5-({1-[(4-carboxybutanoyl)oxy]-6-méthoxy-3,3,14-triméthyl-7-oxo-2,3,7,14-tétrahydro-1*H*-benzo[α]pyrano[3,2-*h*]acridin-2-yl}oxy)-5-oxopentanoique**

Le produit est obtenu selon le procédé de l'exemple 9 en utilisant un excès d'anhydride glutarique à la place de l'anhydride succinique.

**EXEMPLE 32 : Diéthylcarbamate de 1-{[(diéthylamino)carbonyl]oxy}-6-méthoxy-3,3,14-triméthyl-7-oxo-2,3,7,14-tétrahydro-1*H*-benzo[α]pyrano [3,2-*h*]acridin-2-yl**

Le produit est obtenu selon le procédé de l'exemple 7 en utilisant le chlorure de *NN*-diéthylcarbamoyle à la place du chlorure de *N,N*-diméthylcarbamoyle.

**EXEMPLE 33 : 6-{[2-(Diméthylamino)éthyl)amino}-3,3,14-triméthyl-3,14-dihydro[α]pyrano[3,2-*h*]acridin-7-one**

A 0,15 g du produit de l'exemple 3 est additionné 4 ml de *N,N*-diméthylethylènediamine. Après 5 jours de réaction à 70 °C sous atmosphère inerte, le milieu réactionnel est évaporé sous pression réduite. Le résidu obtenu est chromatographié sur gel de silice (cyclohexane/acétate d'éthyle : 80/20) permettant d'isoler le produit attendu.

Point de fusion : huile.

**EXEMPLE 34 : 6-{[3-(Diéthylamino)propyl)amino}-3,3,14-triméthyl-3,14-dihydro[α]pyrano[3,2-*h*]acridin-7-one**

On procède comme dans l'exemple 33 en utilisant comme réactif la *N,N*-diéthyl-propyldiamine.

Point de fusion : huile.

**EXEMPLE 35 : 1-Hydroxy-6-méthox-3,3,14-triméthyl-7-oxo-2,3,7,14-tétrahydro**

**1*H*-benzo[α]pyrano[3,2-*h*]acridin-2-yl (2*E*)-3-phényl-2-propenoate**

A une solution préalablement refroidie de 0,30 mmol du composé de l'exemple 4 dans 4 ml de pyridine sont additionnés 1,33 mmol de chlorure de cinnamoyle. Après agitation à 0°C pendant 90 minutes, le mélange réactionnel est évaporé sous pression réduite. Une chromatographie sur gel de silice (cyclohexane puis cyclohexane/acétone : 94/6 à 90/10) permet d'isoler le produit attendu.

**EXEMPLE 36 : 1-(Acétyloxy)-6-méthoxy-3,3,14-triméthyl-7-oxo-2,3,7,14-tétrahydro-1*H*-benzo[α]pyrano[3,2-*h*] acridin-2-yl (2*E*)-3-phényl-2-propenoate**

A une solution préalablement refroidie de 0,18 mmol du composé de l'exemple 35 dans 3 ml de pyridine sont additionnés 31 mmol d'anhydride acétique. Après agitation à température ambiante pendant 3 jours, le mélange réactionnel est évaporé sous pression réduite. Une chromatographie sur gel de silice (cyclohexane/acétane : 94/6) permet d'isoler le produit attendu.

**EXEMPLE 37 : 6-Méthoxy-3,3-diméthyl-3,14-dihydro-7*H*-benzo[α]pyrano[3,2-*h*]acridin-7-one**

Uns solution de 300 mg du composé de l'exemple 1 dans 15 ml de diméthylformamide anhydre est additionnée, goutte à goutte, à une suspension de 125 mg d'hydrure de sodium dans 10 ml de diméthylformamide anhydre préalablement refroidi à 0°C. Le milieu réactionnel est maintenu à 0°C sous agitation et sous atmosphère inerte pendant 15 minutes et ensuite maintenu à température ambiante pendant 30 minutes. Puis, on additionne 0,57 ml de diméthylsulfate et le mélange ainsi obtenu est laissé sous agitation. Après 17 heures, le milieu réactionnel est versé sur 150 ml d'eau glacée et extrait par le dichlorométhane (3x50 ml). Les phases organiques réunies sont ensuite lavées à l'eau, séchées sur sulfate de sodium anhydre, filtrées et évaporées à sec sous pression réduite. Une chromatographie sur gel de silice (cyclohexane puis un gradient d'acétone de 5 à 20%) permet d'isoler le produit attendu.

Spectre de masse (ESI): $m/z$ = 358 $(M+H)^+$

**EXEMPLE 38 : (±)-*cis*-1-Hydroxy-6-méthoxy-3,3,14-triméthyl-3,14-dihydro-7*H*-benzo[α]pyrano[3,2-*h*]acridin-2-yl diméthylcarbamate**

70,5 mg d'hydrure de potassium sont lavés trois fois à l'hexane puis refroidit à -10°C. On ajoute une solution de 50 mg du composé de l'exemple 4 dans 8 ml de THF anhydre. Après addition goutte à goutte à -10°C de 45 µl de *N,N*-diméthylcarbamoyle, le milieu réactionnel est maintenu pendant 20h à température ambiante. La réaction est arrêtée par addition de 25 ml d'acétate d'éthyle et de 10 ml d'une solution saturée de $NaHCO_3$ afin d'obtenir un pH = 8. La phase organique est lavée à l'eau et séchée sur $Na_2SO_4$. Après évaporation du solvant, une chromatographie sur gel de silice (dichlorométhane/acétone : 80/20) permet d'isoler le produit attendu.

Spectre de masse ($ESI^+$) : $m/z$ = 477 $(M+H)^+$

**EXEMPLE 39 : 10-Bromo-6-hydroxy-3,3-diméthyl-3,14-dihydro-7*H*-benzo[α]pyrano-[3,2-*h*]acridin-7-one**

Le produit est obtenu selon le procédé de l'exemple 1 en utilisant le composé de la préparation 5 à la place du composé de la préparation 2.

Spectre de masse ($ESI^+$) : $m/z$ = 422 $(M+ H)^+$

**EXEMPLE 40:10-Bromo-6-méthoxy-3,3,14-triméthyl-3,14-dihydro-7*H*-benzo[α]-pyrano[3,2-*h*]acridin-7-one**

Le produit est obtenu selon le procédé de l'exemple 3 en utilisant le composé de l'exemple 39 à la place du composé de l'exemple 2.

Spectre de masse ($ESI^+$) : $m/z$ = 450 $(M+ H)^+$

**EXEMPLE 41 : 10-Bromo-6-hydroxy-3,3,14-triméthyl-3,14-dihydro-7*H*-benzo[α]-pyrano[3,2-*h*]acridin-7-one**

Le produit est obtenu selon le procédé de l'exemple 2 en utilisant le composé de l'exemple 39 à la place du composé de l'exemple 1.

***ETUDE PHARMACOLOGIQUE DES COMPOSES DE L'INVENTION***

**EXEMPLE 42 : Cytotoxicité in vitro**

Quatre lignées cellulaires ont été utilisées:

- 1 leucémie murine, L1210,
- 1 carcinome épidermoïde humain : KB-3-1,
- 1 carcinome colique humain : HT-29,
- 1 carcinome de la prostate humain : LNCap

Les cellules sont cultivées dans du milieu RPMI 1640 complet contenant 10 % de sérum de veau foetal, 2 mM de glutamine, 50 unités/ml de pénicilline, 50 µg/ml de streptomycine et 10 mM d'Hepes, pH = 7,4. Les cellules sont réparties dans des microplaques et exposées aux composés cytotoxiques. Les cellules sont ensuite incubées pendant 2 jours (L1210) ou 4 jours (lignées humaines). Le nombre de cellules viables est ensuite quantifié par un essai colorimétrique, le Microculture Tetrazolium Assay (Cancer Res. 1987, 47, 939-942). Les résultats sont exprimés en $IC_{50}$, concentration en cytotoxique qui inhibe à 50 % la prolifération des cellules traitées. A titre d'exemple, les composés des exemples 5 et 6 présentent respectivement des $IC_{50}$ de 0,73 µM et 0,06 µM sur L1210 et de 0,14 µM et 0,015 µM sur KB-3-1. Le composé de l'exemple 5 présente une $IC_{50}$ de 1,18 µM sur HT-29 et 0,57 µM sur LNCap.

**EXEMPLE 43 : Activité in vivo**

*Activité antitumorale sur l'adénocarcinome du colon C38*

Les fragments tumoraux d'adénocarcinome du colon C38, pesant environ 30 mg, ont été implantés au jour 0 sous la peau de souris B6D2F1 (Iffa Credo, France). Après croissance de la tumeur, les souris ont été séparées en groupes contrôle (18 animaux) et traité (6 à 7 animaux), homogènes quant à la taille tumorale. Les produits ont été administrés deux fois par voie i.v. aux jours 12 et 22, à leur Dose Maximale Tolérée (MTD), MTD/2 et MTD/4. Les tumeurs ont été mesurées deux fois par semaine et les volumes tumoraux ont été calculés suivant la formule : Volume ($mm^3$) = longueur (mm) x largeur ($mm^2$) /2. L'activité antitumorale est exprimée en % de T/C :

$$\%T/C = \frac{\text{Vt/V0 médian des animaux traités}}{\text{Vt/V0 médian des animaux contrôle}} \times 100$$

V0 et Vt étant respectivement le volume initial de la tumeur et son volume au temps de mesure t.

La dose optimale est la dose qui donne le T/C le plus bas sans toxicité (mort prématurée ou perte de poids supérieure à 20%). A titre d'exemple, le composé de l'exemple 5 montre une inhibition de la croissance tumorale de 95 % (T/C = 5%) à la dose optimale de 4 mg/kg alors que l'acronycine montre un T/C de 27% à la dose optimale de 100 mg/kg permettant de démontrer leur fort potentiel thérapeutique.

**EXEMPLE 44 : Composition pharmaceutique: soluté injectable**

| | |
|---|---|
| Composé de l'exemple 6 | 10 mg |
| Eau distillée pour préparations injectables | 25 ml |

**Revendications**

**1.** Composés de formule (I) :

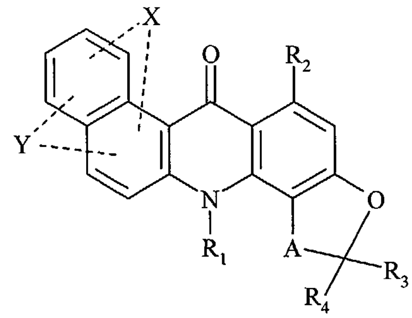

(I)

dans laquelle :

- X et Y, identiques ou différents, indépendamment l'un de l'autre, représentent chacun un groupement choisi parmi :

  - atome d'hydrogène, halogène,
  - groupement hydroxy, alkoxy ($C_1$-$C_6$) linéaire ou ramifié, nitro, cyano, alkyle ($C_1$-$C_6$) linéaire ou ramifié, alkényle ($C_2$-$C_6$) linéaire ou ramifié, polyhalogénoalkyle ($C_1$-$C_6$) linéaire ou ramifié, ou
  - groupement de formule $-NR_aR_b$ dans lequel :

    $R_a$ et $R_b$, identiques ou différents, indépendamment l'un de l'autre, représentent chacun un groupement choisi parmi atome d'hydrogène, $-C(O)-CF_3$, $-C(O)-NH_2$ ou groupement alkyle ($C_1$-$C_6$) linéaire ou ramifié, éventuellement substitué par un groupement $NR'_aR'_b$ dans lequel :

      $R'_a$ et $R'_b$, identiques ou différents, indépendamment l'un de l'autre, représentent chacun un groupement choisi parmi atome d'hydrogène, groupement alkyle ($C_1$-$C_6$) linéaire ou ramifié, aryle, arylalkyle ($C_1$-$C_6$) linéaire ou ramifié, ou $R'_a$ et $R'_b$ forment ensemble avec l'atome d'azote qui les portent, un hétérocycle de 5 à 7 chaînons, monocyclique, contenant éventuellement au sein du système cyclique un second hétéroatome choisi parmi oxygène et azote,
      ou $R_a$ et $R_b$ forment ensemble avec l'atome d'azote qui les portent, un hétérocycle de 5 à 7 chaînons, monocyclique, contenant éventuellement au sein du système cyclique un second hétéroatome choisi parmi oxygène et azote, étant entendu que les substituants X et Y peuvent être présents sur l'un ou l'autre des deux cycles benzéniques adjacents,

- $R_1$ représente un atome d'hydrogène ou un groupement alkyle ($C_1$-$C_6$) linéaire ou ramifié,

- $R_2$ représente un groupement choisi parmi atome d'hydrogène, groupement alkyle ($C_1$-$C_6$) linéaire ou ramifié, $-OR''_a$ ; $-NR'_aR'_b$ ; $—O—T_a—OR''_a$ ; $—NR''_a-T_a-NR'_aR'_b$ ; $—NR''_a—C(O)—T_aH$ ; $-O-C(O)-T_aH$ ; $-O-T_a-NR'_aR'_b$ ; $—NR''_a—T_a—OR''_a$; $—NR''_a—T_a—CO_2R''_a$ ; $-NR''_a-C(O)-T_a-NR'_aR'_b$
  dans lesquels :

* $T_a$ représente une chaîne alkylène ($C_1$-$C_6$) linéaire ou ramifiée,
* $R'_a$ et $R'_b$ sont tels que définis précédemment,
* $R''_a$ représente un groupement choisi parmi atome d'hydrogène, groupement alkyle ($C_1$-$C_6$) linéaire ou ramifié,
* $R_3$, $R_4$, identiques ou différents, indépendamment l'un de l'autre, représentent un atome d'hydrogène ou un groupement alkyle ($C_1$-$C_6$) linéaire ou ramifié, ou $R_3$ et $R_4$ forment ensemble avec l'atome de carbone qui les portent, un cycle de 3 à 6 chaînons, monocyclique,
* A représente un groupement de formule:

  **a) $-CH(R_5)-CH(R_6)-$** dans lequel :

  * *$R_5$ et $R_6$, identiques ou différents, indépendamment l'un de l'autre représentent chacun un groupement choisi parmi:*

**1)** atome d'hydrogène,

**2)** groupement $OR_c$, $NR_cR_d$, $SR_c$, dans lesquels :

- $R_c$, $R_d$, identiques ou différents, indépendamment l'un de l'autre, représentent chacun un groupement choisi parmi atome d'hydrogène, groupement alkyle ($C_1$-$C_6$) linéaire ou ramifié, ou un groupement aryle, arylalkyle ($C_1$-$C_6$) linéaire

ou ramifié, ou un groupement C(O)-$R_e$ dans lequel $R_e$ représente un groupement choisi parmi atome d'hydrogène, groupement aryle, ou $NR'''_aR'''_b$ dans lequel $R'''_a$ et $R'''_b$, identiques ou différents, représentent un atome d'hydrogène, groupement alkyle ($C_1$-$C_6$) linéaire ou ramifié ou $R'''_a$ et $R'''_b$ forment ensemble avec l'atome d'azote qui les portent, un hétérocycle de 5 à 7 chaînons, monocyclique, contenant éventuellement au sein du système cyclique un second hétéroatome choisi parmi oxygène et azote,

**3)** $W_1$-C($W_2$)-U-V dans lequel :

α) $W_1$ représente un atome d'oxygène, de soufre ou $NR_c$ (dans lequel $R_c$ est tel que défini précédemment),
β) $W_2$ représente un atome d'oxygène ou un atome de soufre,
γ) U représente une chaîne alkylène ($C_1$-$C_8$) linéaire ou ramifiée ou une chaîne alkénylène ($C_2$-$C_8$) linéaire ou ramifiée,
δ) V représente un groupement choisi parmi:

- atome d'hydrogène,
- groupement aryle,
- groupements $OR_c$, $CO_2R_c$, $COR_c$, $CONR'_aR'_b$, $NR'_aR'_b$, $N(R_c)$-$CO_2R'_c$, $N(R_c)$-$COR'_c$, dans lesquels $R'_a$, $R'_b$, et $R_c$ sont tels que définis précédemment et $R'_c$ représente un groupement choisi parmi atome d'hydrogène, groupement alkyle ($C_1$-$C_6$) linéaire ou ramifié, ou un groupement aryle, arylalkyle ($C_1$-$C_6$) linéaire ou ramifié,

ε) U représente une liaison lorsque $W_2$ ne représente pas un atome d'oxygène et V ne représente pas un groupement choisi parmi:

- atome d'hydrogène,
- groupement aryle,
- $NH_2$,

**4)** $W_1$-C($W_2$)-$W_3$-$T_1$ dans lequel :

α) $W_1$ et $W_2$ sont tels que définis précédemment,
β) $W_3$ représente un atome d'oxygène, de soufre ou $NR_c$ dans lequel $R_c$ est tel que défini précédemment,
γ) $T_1$ représente un groupement choisi parmi:

- atome d'hydrogène
- alkyle ($C_1$-$C_6$) linéaire ou ramifié,
- alkényle ($C_2$-$C_6$) linéaire ou ramifié,
- aryle, arylalkyle ($C_1$-$C_6$) linéaire ou ramifié,
- chaîne alkylène ($C_1$-$C_6$) linéaire ou ramifiée ou une chaîne alkénylène ($C_2$-$C_6$) linéaire ou ramifiée, chacune étant substituée par un groupement $OR_c$ dans lequel $R_c$ est tel que défini précédemment ou par $NR'_aR'_b$ dans lequel $R'_a$ et $R'_b$ sont tels que définis précédemment,

**5)** $W_1$-S(O)$_n$-$W_3$-$T_1$ dans lequel :

α) $W_1$, $W_3$ et $T_1$ sont tels que définis précédemment,
β) n représente un nombre entier choisi parmi 1 et 2,

**6)** $W_1$-$S(O)_n$-U'-V' dans lequel :

α) U' représente une chaîne alkylène ($C_1$-$C_8$) linéaire ou ramifiée ou une chaîne alkénylène ($C_2$-$C_8$) linéaire ou ramifiée,
β) V' représente un groupement choisi parmi :

- atome d'hydrogène,
- groupement aryle,
- groupements $OR_c$, $CO_2R_c$, $COR_c$, $CONR'_aR'_b$, $NR'_aR'_b$, $N(R_c)$—$CO_2R'_c$ $N(R_c)$-$COR'_c$, dans lesquels $R'_a$, $R'_b$, $R_c$ et $R'_c$ sont tels que définis précédemment, et,

γ) $W_1$ et n sont tels que définis précédemment,

**7)** $C(W_2)$-$T_1$ dans lequel $W_2$ et $T_1$ sont tels que définis précédemment,

* *ou $R_5$ et $R_6$, forment ensemble :*

1) un groupement

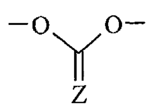 , 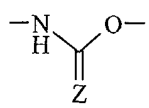 , 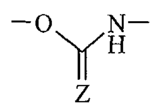 , 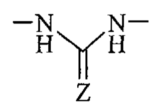

dans lesquels Z représente un atome d'oxygène ou un atome de soufre,

**2)** un groupement -O-$(CH_2)_m$-O- dans lequel m représente un nombre entier compris entre 1 et 4 inclus,

3) un groupement

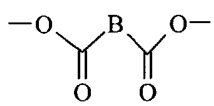 , 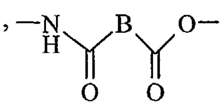 , 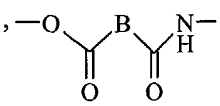

dans lesquels B représente une liaison simple, une chaîne alkylène ($C_1$-$C_6$) linéaire ou ramifiée ou une chaîne alkénylène ($C_2$-$C_6$) linéaire ou ramifiée,

* *ou $R_5$ et $R_6$, forment ensemble avec les atomes de carbone qui les portent, un groupement oxiranne ou un groupement aziridine,* éventuellement substitué sur l'atome d'azote par un groupement alkyle ($C_1$-$C_6$) linéaire ou ramifié,

**b) -CH=C($R_7$)- ou -C($R_7$)=CH-** dans lesquels $R_7$ représente un groupement choisi parmi :

- atome d'hydrogène,
- groupement $OR''_a$, $W_1$-$C(W_2)$-U-V, $W_1$-$C(W_2)$-$W_3$-$T_1$, $W_1$-$S(O)_n$-$W_3$-$T_1$, $W_1$-$S(O)_n$-U'-V' ou $C(W_2)$-$T_1$ dans lesquels $R''_a$, $W_1$, $W_2$, $W_3$, U, V, U', V', $T_1$ et n sont tels que définis précédemment,

**c) -C(O)-CH($R_8$)- ou - CH($R_8$)-C(O)-** dans lesquels $R_8$ représente un groupement choisi parmi :

- atome d'hydrogène,
- alkylcarbonyloxy ($C_1$-$C_6$) linéaire ou ramifié, groupement $OR''_a$ dans lequel $R''_a$ est tel que défini précédemment,

leurs énantiomères, diastéréoisomères, N-oxydes, ainsi que leurs sels d'addition à un acide ou à une base pharmaceutiquement acceptable, étant entendu que :

par aryle, on comprend un groupement phényle ou naphtyle, comportant éventuellement une ou plusieurs substitutions, identiques ou différentes, choisies parmi hydroxy, halogène, carboxy, nitro, amino, alkylamino ou dialkylamino ($C_1$-$C_6$) linéaire ou ramifié, alkoxy ($C_1$-$C_6$) linéaire ou ramifié, acyle ($C_1$-$C_6$) linéaire ou ramifié, et alkylcarbonyloxy ($C_1$-$C_6$) linéaire ou ramifié,

par hétérocycles de 5 à 7 chaînons, monocyclique, contenant éventuellement au sein du système cyclique un second hétéroatome choisi parmi oxygène et azote, on peut citer à titre non limitatif les groupements pyrrolidinyle, isoxazolidinyle, oxazolidinyle, pyrazolidinyle, imidazolidinyle, pipéridinyle, oxazinanyle, morpholinyle, hexahydropyridazinyle, hexahydropyrimidinyle, pipérazinyle, azépanyle, oxazépanyle, diazépanyle,

par cycles de 3 à 6 chaînons, monocyclique, on peut citer à titre non limitatif les groupements cyclopropyle, cyclobutyle, cyclopentyle et cyclohexyle.

**2.** Composés de formule (I) selon la revendication 1, **caractérisés en ce qu'**ils représentent des composés de formule (IA) :

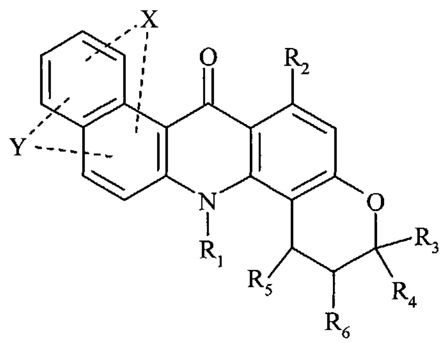

(IA)

dans laquelle X, Y, $R_1$, $R_2$, $R_3$, $R_4$ $R_5$ et $R_6$ sont tels que définis dans la formule (I), leurs énantiomères, diastéréoisomères, N-oxydes ainsi que leurs sels d'addition à un acide ou à une base pharmaceutiquement acceptable.

**3.** Composés de formule (I) selon l'une quelconque des revendications 1 à 2, **caractérisés en ce qu'**ils représentent des composés de formule (IA) dans laquelle $R_5$ et $R_6$, identiques, représentent un groupement de formule $-OR_c$, $W_1$-C($W_2$)-U-V, $W_1$-C($W_2$)-$W_3$-$T_1$, C($W_2$)-$T_1$, ou $R_5$ et $R_6$ forment ensemble un groupement

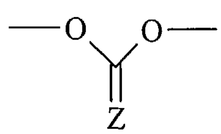

dans lesquels $R_c$, $W_1$' $W_2$, $W_3$, U, V, $T_1$ et Z sont tels que définis dans la formule (I), leurs énantiomères, diastéréoisomères, N-oxydes ainsi que leurs sels d'addition à un acide ou à une base pharmaceutiquement acceptable.

**4.** Composés de formule (I) selon l'une quelconque des revendications 1 à 3, **caractérisés en ce qu'**ils représentent des composés de formule (IA) dans laquelle $R_5$ et $R_6$, identiques, représentent un groupement de formule $-OR_c$ dans laquelle $R_c$ représente un atome d'hydrogène, leurs énantiomères, diastéréoisomères, N-oxydes ainsi que leurs sels d'addition à un acide ou à une base pharmaceutiquement acceptable.

**5.** Composés de formule (I) selon l'une quelconque des revendications 1 à 4, **caractérisés en ce qu'**ils représentent des composés de formule (IA) dans laquelle $R_5$ et $R_6$, identiques ou différents, représentent un groupement de formule $W_1$-C($W_2$)-U-V dans laquelle $W_1$ et $W_2$ représentent un atome d'oxygène, U est tel que défini dans la formule (I) et V représente un atome d'hydrogène ou U représente une chaîne alkylène ($C_1$-$C_8$) linéaire ou ramifié et V représente un groupement $NR'_aR'_b$ dans lequel $R'_a$ et $R'_b$ identiques ou différents représentent un atome

d'hydrogène ou un groupement alkyle ($C_1$-$C_6$) linéaire ou ramifié, leurs énantiomères, diastéréoisomères, N-oxydes ainsi que leurs sels d'addition à un acide ou à une base pharmaceutiquement acceptable.

**6.** Composés de formule (I) selon l'une quelconque des revendications 1 à 5 **caractérisés en ce qu'**ils représentent des composés de formule (IA) dans laquelle $R_5$ représente un groupement de formule -$OR_c$ dans laquelle $R_c$ représente un atome d'hydrogène et $R_6$ représente un groupement de formule $W_1$-C($W_2$)-U-V dans laquelle $W_1$ et $W_2$ repésentent un atome d'oxygène, U représente une chaîne alkylène ($C_1$-$C_8$) linéaire ou ramifiée et V représente un atome d'hydrogène, leurs énantiomères, diastéréoisomères, N-oxydes ainsi que leurs sels d'addition à un acide ou à une base pharmaceutiquement acceptable.

**7.** Composés de formule (I) selon l'une quelconque des revendications 1 à 6 **caractérisés en ce qu'**ils représentent des composés de formule (IA) dans laquelle $R_5$ représente un groupement de formule -$OR_c$ ou $W_1$-C($W_2$)-U-V dans lesquels $R_c$ représente un atome d'hydrogène, $W_1$ et $W_2$ représentent un atome d'oxygène, U représente une chaîne alkylène ($C_1$-$C_8$) linéaire ou ramifiée et V représente un atome d'hydrogène et $R_6$ représente un groupement de formule $W_1$-C($W_2$)-U-V dans laquelle $W_1$ et $W_2$ représentent un atome d'oxygène, U représente une chaîne alkénylène ($C_2$-$C_8$) linéaire ou ramifiée et V représente un groupement choisi parmi atome d'hydrogène ou groupement aryle, leurs énantiomères, diastéréoisomères, N-oxydes ainsi que leurs sels d'addition à un acide ou à une base pharmaceutiquement acceptable.

**8.** Composés de formule (I) selon l'une quelconque des revendications 1 à 7, **caractérisés en ce qu'**ils représentent des composés de formule (IA) dans laquelle $R_5$ et $R_6$ identiques ou différents, représentent un groupement de formule $W_1$-C($W_2$)-$W_3$-$T_1$ dans laquelle $W_1$ et $W_2$ représentent un atome d'oxygène, $W_3$ représente un groupement -$NR_c$ dans lequel $R_c$ représente un groupement alkyle ($C_1$-$C_6$) linéaire ou ramifié et $T_1$ représente un groupement alkyle ($C_1$-$C_6$) linéaire ou ramifié, leurs énantiomères, diastéréoisomères, N-oxydes ainsi que leurs sels d'addition à un acide ou à une base pharmaceutiquement acceptable.

**9.** Composés de formule (I) selon l'une quelconque des revendications 1 à 8, **caractérisés en ce qu'**ils représentent des composés de formule (IA) dans laquelle $R_5$ et $R_6$, forment ensemble un groupement

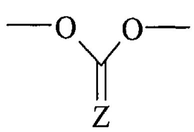

dans lequel Z représente un atome d'oxygène, leurs énantiomères, diastéréoisomères, N-oxydes ainsi que leurs sels d'addition à un acide ou à une base pharmaceutiquement acceptable.

**10.** Composés de formule (I) selon la revendication 1 **caractérisés en ce qu'**ils représentent des composés de formule (IB) :

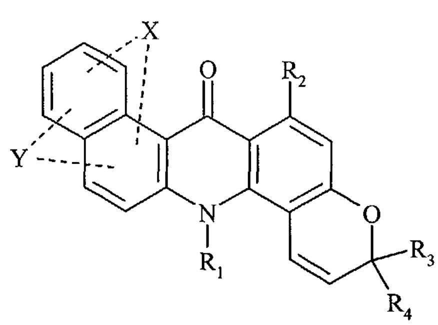

(IB)

dans laquelle X, Y, $R_1$, $R_2$, $R_3$ et $R_4$ sont tels que définis dans la formule (I), leurs énantiomères, diastéréoisomères, N-oxydes ainsi que leurs sels d'addition à un acide ou à une base pharmaceutiquement acceptable.

**11.** Composés de formule (I) selon l'une quelconque des revendications 1 à 10, **caractérisés en ce que** $R_3$ et $R_4$ représentent un groupement alkyle ($C_1$-$C_4$) linéaire ou ramifié, leurs énantiomères, diastéréoisomères, N-oxydes ainsi que leurs sels d'addition à un acide ou à une base pharmaceutiquement acceptable.

**12.** Composés de formule (I) selon l'une quelconque des revendications 1 à 11, **caractérisés en ce que** $R_3$ et $R_4$ représentent un groupement méthyle, leurs énantiomères, diastéréoisomères, N-oxydes ainsi que leurs sels d'addition à un acide ou à une base pharmaceutiquement acceptable

**13.** Composés de formule (I) selon l'une quelconque des revendications 1 à 12, **caractérisés en ce que** $R_2$ représente les groupements -OR"$_a$ ou -NR"$_a$-$T_a$-NR'$_a$R'$_b$ dans lesquels R'$_a$, R'$_b$, R"$_a$ et $T_a$ sont tels que définis dans la formule (I), leurs énantiomères, diastéréoisomères, N-oxydes ainsi que leurs sels d'addition à un acide ou à une base pharmaceutiquement acceptable

**14.** Composés de formule (I) selon l'une quelconque des revendications 1 à 13, **caractérisés en ce que** $R_2$ représente le groupement -OR"$_a$ dans lequel R"$_a$ est tel que défini dans la formule (I) et le groupement -NR"$_a$-$T_a$-NR'$_a$R'$_b$ dans lequel R"$_a$ représente un atome d'hydrogène, $T_a$ est tel que défini dans la formule (I) et R'$_a$ et R'$_b$ identiques ou différents, représentent un groupement alkyle ($C_1$-$C_6$) linéaire ou ramifié, leurs énantiomères, diastéréoisomères, N-oxydes ainsi que leurs sels d'addition à un acide ou à une base pharmaceutiquement acceptable.

**15.** Composés de formule (I) selon l'une quelconque des revendications 1 à 14, **caractérisés en ce que** X et Y représentent un atome d'hydrogène, leurs énantiomères, diastéréoisomères, N-oxydes ainsi que leurs sels d'addition à un acide ou à une base pharmaceutiquement acceptable.

**16.** Composés de formule (I) selon la revendication 1 qui sont le :

- (±)-*cis*-1,2-dihydroxy-6-méthoxy-3,3,14-triméthyl-1,2,3,14-tétrahydro-7*H*-benzo[α] pyrano[3,2-*h*]acridin-7-one,
- (±)-*cis*-1,2-diacétoxy-6-méthoxy-3,3,14-triméthyl-1,2,3,14-tétrahydro-7*H*-benzo[α] pyrano[3,2-*h*]acridin-7-one,
- (±)-*cis*-7-méthoxy-4,4,15-triméthyl-15,15c-dihydro-4*H*-benzo[α][1,3]dioxolo [4',5':4,5]pyrano[3,2-h]acridine-2,8(3a*H*)-dione,
- 6-méthoxy-3,3,14-triméthyl-3,14-dihydro-7*H*-benzo[α]pyrano[3,2-*h*]acridin-7-one,
- 6-hydroxy-3,3,14-triméthyl-3,14-dihydro-7*H*-benzo[α]pyrano[3,2-*h*]acridin-7-one,
- 6-hydroxy-3,3-diméthyl-3,14-dihydro-7*H*-benzo[α]pyrano[3,2-*h*]acridin-7-one, leurs énantiomères, diastéréoisomères, N-oxydes ainsi que leurs sels d'addition à un acide ou à une base pharmaceutiquement acceptable.

**17.** Procédé de préparation des composés de formule (I) selon la revendication 1 **caractérisés en ce qu'**on utilise comme produit de départ un composé de formule (II) :

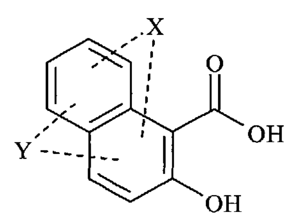

(II)

dans laquelle X et Y sont tels que définis dans la formule (I), composé de formule (II) qui est traité avec du diméthylsulfate en milieu basique, pour conduire aux composés de formule (III) :

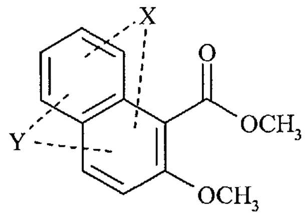

(III)

dans laquelle X et Y sont tels que définis précédemment, composés de formule (III) qui est traité par une solution alcaline de Claisen, une solution d'hydroxyde de potassium dans un mélange eau-méthanol, puis une solution d'acide chlorhydrique, pour conduire aux composés de formule (IV) :

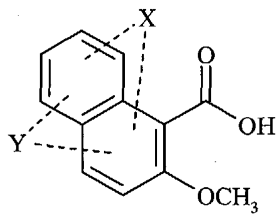

(IV)

dans laquelle X et Y sont tels que définis précédemment, composé de formule (IV) qui est traité par du chlorure de thionyle puis en milieu anhydre par un composé de formule (V) :

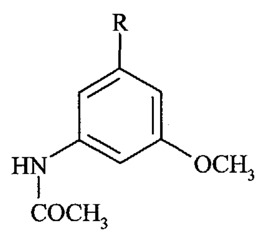

(V)

dans laquelle R représente un atome d'hydrogène, un groupement hydroxy, un groupement alkyle ($C_1$-$C_6$) linéaire ou ramifié ou un groupement alkoxy ($C_1$-$C_6$) linéaire ou ramifié pour conduire aux composés de formule (VI) :

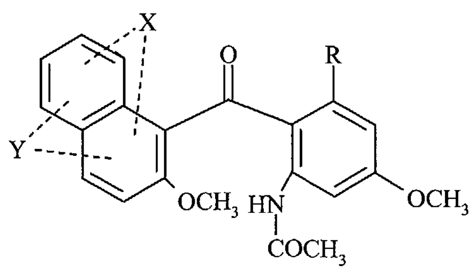

(VI)

dans laquelle X, Y et R sont tels que définis précédemment, composé de formule (VI) qui est traité par une suspension d'hydrure de sodium dans un solvant aprotique anhydre, pour conduire aux composés de formule (VII) :

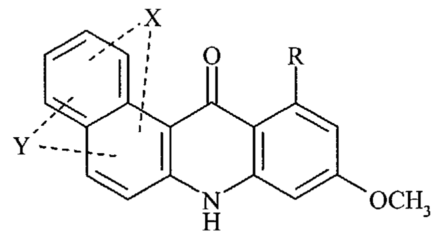

(VII)

dans laquelle X, Y et R sont tels que définis précédemment, composé de formule (VII) qui est traité par une solution aqueuse d'acide bromhydrique dans de l'acide acétique, pour conduire aux composés de formule (VIII) :

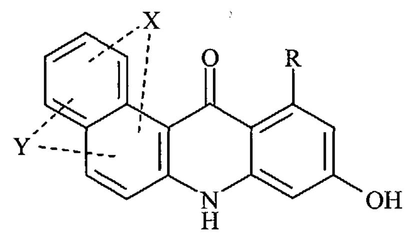

(VIII)

dans laquelle X, Y et R sont tels que définis précédemment, composé de formule (VIII) qui est ensuite traité en condition basique dans un solvant aprotique anhydre par un alcyne de formule (IX) :

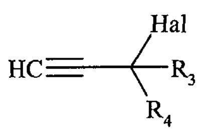

(IX)

dans laquelle Hal représente un atome d'halogène et $R_3$ et $R_4$ sont tels que définis dans la formule (I), pour conduire aux composés de formule (I/a), cas particulier des composés de formule (I) :

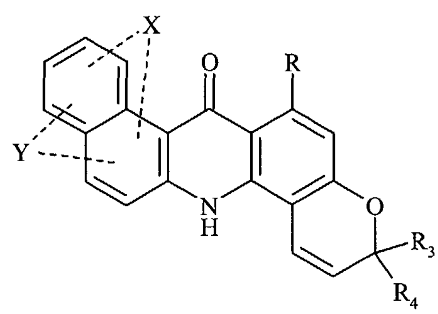

(I/a)

dans laquelle X, Y, R, $R_3$ et $R_4$ sont tels que définis précédemment, composés de formule (I/a) dont l'atome d'azote est éventuellement substitué, par action d'un halogénure d'alkyle ou d'un sulfate de dialkyle en présence d'un agent de déprotonation, en solvant polaire aprotique, pour conduire aux composés de formule (I/b), cas particulier des composés de formule (I) :

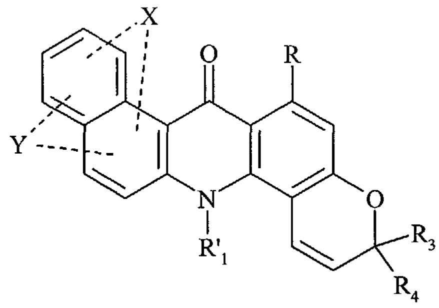

(I/b)

dans laquelle R'$_1$ représente un groupement alkyle ($C_1$-$C_6$) linéaire ou ramifié et X, Y, R, $R_3$ et $R_4$ sont tels que définis précédemment, composés de formule (I/b) qui sont éventuellement soumis, dans le cas où R représente un groupement hydroxy, à l'action d'un agent alkylant ou d'un agent acylant, pour conduire aux composés de formule (I/c), cas particulier des composés de formule (I) :

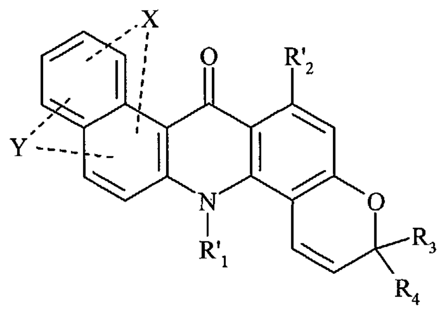

(I/c)

dans laquelle R'$_2$ représente un groupement choisi parmi -OR"$_a$, -O-$T_a$-OR"$_a$, - O-C(O)-$T_a$H, -O-$T_a$-NR'$_a$R'$_b$ dans lesquels R"$_a$,R'$_a$, R'$_b$ et $T_a$ sont tels que définis dans la formule (I) et X, Y, R'$_1$, $R_3$ et $R_4$ sont tels que définis précédemment, composés de formule (I/c) qui sont éventuellement traités, dans le cas où R'$_2$ représente un groupement alkoxy ($C_1$-$C_6$) linéaire ou ramifié par un composé de formule (X):

$$HNR_{10}R_{11} \quad (X)$$

dans laquelle $R_{10}$ représente un groupement choisi parmi R'$_a$ et R"$_a$ qui sont tels que définis dans la formule (I) et $R_{11}$ représente un groupement choisi parmi R'$_b$, -$T_a$-NR'$_a$R'$_b$, -C(O)-$T_a$H, -$T_a$-OR"$_a$ et -$T_a$-$CO_2$R"$_a$ dans lesquels $T_a$, R'$_a$, R'$_b$ et R"$_a$ sont tels que définis précédemment, pour conduire aux composés de formule (I/d), cas particulier des composés de formule (I) :

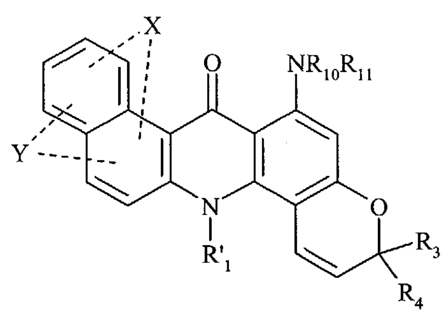

(I/d)

dans laquelle X, Y, R'$_1$, $R_3$, $R_4$, $R_{10}$ et $R_{11}$ sont tels que définis précédemment, l'ensemble des composés de formule (I/a) à (I/d) formant les composés de formule (I/e) :

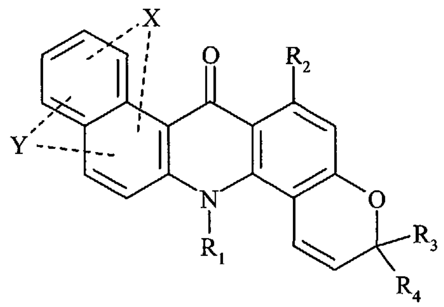

(I/e)

dans laquelle X, Y, $R_1$, $R_2$, $R_3$ et $R_4$ sont tels que définis dans la formule (I), ***composés de formule (I/e) pouvant être soumis,***

**a)** soit à l'action d'un agent réducteur, pour conduire aux composés de formule (I/f), cas particulier des composés de formule (I) :

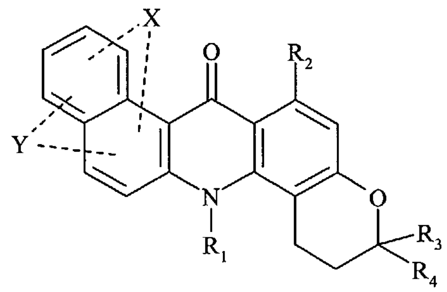

(I/f)

dans laquelle X, Y, $R_1$, $R_2$, $R_3$ et $R_4$ sont tels que définis précédemment,

**b)** soit à l'action du tétroxyde d'osmium en milieu polaire et en présence de 4-méthylmorpholine-N-oxyde, pour conduire aux composés de formules ($I/g_1$) et ($I/g_2$), cas particulier des composés de formule (I) :

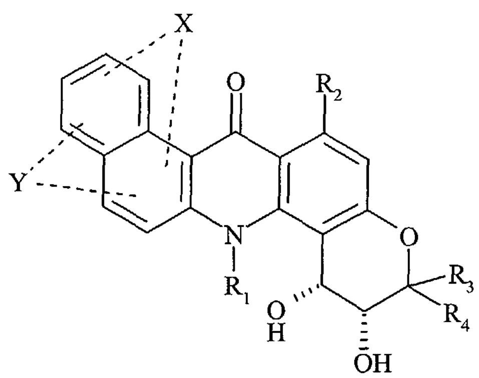

($I/g_1$)

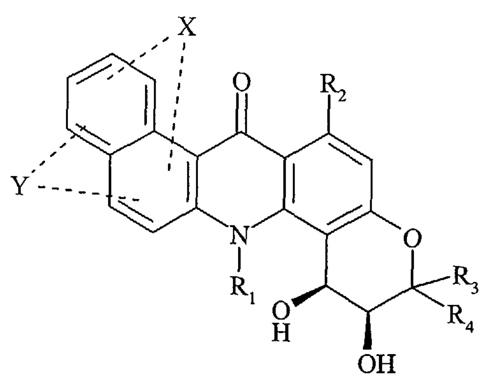

($I/g_2$)

dans laquelle X, Y, $R_1$, $R_2$, $R_3$ et $R_4$ sont tels que définis précédemment, l'ensemble des composés de formule ($I/g_1$) et ($I/g_2$) formant les composés *cis*-diol de formule (*cis*-I/g):

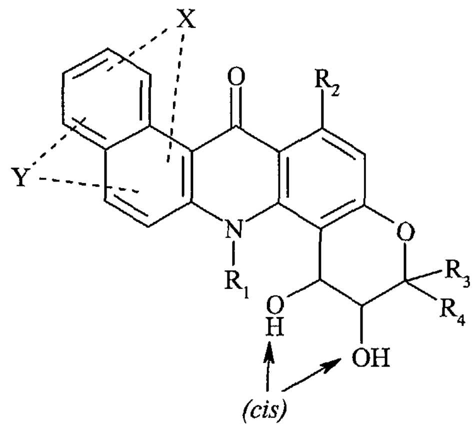

(*cis* - I/g)

dans laquelle X, Y, $R_1$, $R_2$, $R_3$ et $R_4$ sont tels que définis précédemment, composés *cis*-diol de formule (*cis*-I/g) qui sont soumis éventuellement à l'action d'un composé de formule (XI) :

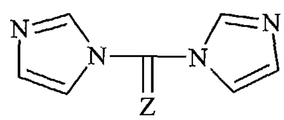

(XI)

dans laquelle Z est tel que défini dans la formule (I) pour conduire aux composés de formule (*cis*-I/h), cas particulier des composés de formule (I) :

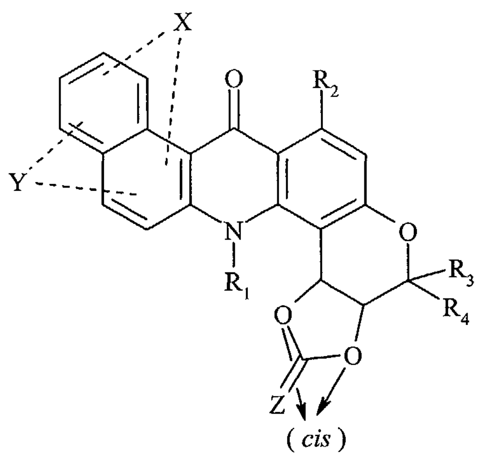

(*cis*-I/h)

dans laquelle X, Y, $R_1$, $R_2$, $R_3$ et $R_4$ sont tels que définis précédemment,

**c)** soit à l'action du permanganate de potassium en milieu polaire, pour conduire aux composés de formule (I/i), cas particulier des composés de formule (I) :

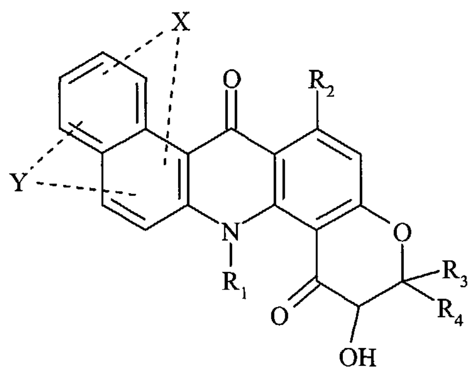

(I/i)

dans laquelle X, Y, $R_1$, $R_2$, $R_3$ et $R_4$ sont tels que définis précédemment,

**composés de formule (I/i) pouvant être soumis :**

α) soit à l'action d'un agent alkylant ou d'un agent acylant, pour conduire aux composés de formule (I/j), cas particulier des composés de formule (I) :

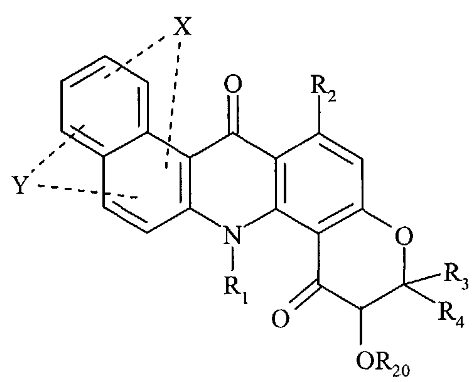

(I/j)

dans laquelle X, Y, $R_1$, $R_2$, $R_3$ et $R_4$ sont tels que définis précédemment, et $R_{20}$ représente un groupement choisi parmi alkyle ($C_1$-$C_6$) linéaire ou ramifié ou alkylcarbonyle ($C_1$-$C_6$) linéaire ou ramifié,

β) soit à des conditions réductrices en présence de $NaBH_4$, pour conduire aux composés de formule (I/k), cas particulier des composés de formule (I) :

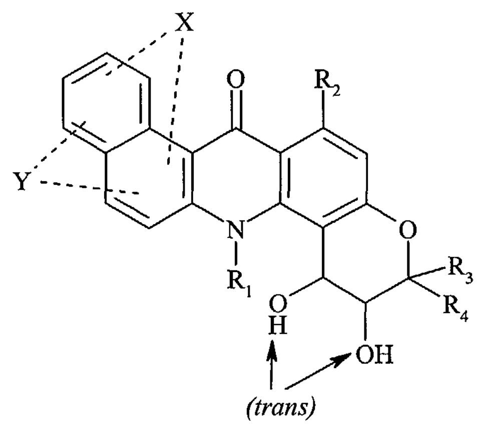

(I/k)

dans laquelle X, Y, $R_1$, $R_2$, $R_3$, et $R_4$ sont tels que définis précédemment, l'ensemble des composés de formules (*cis*-I/g) et (I/k) formant les composés de formule (I/l),

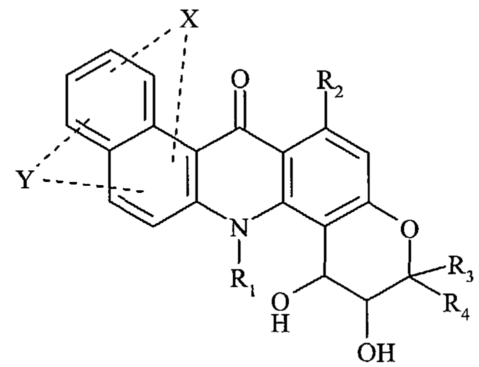

(I/l)

dans laquelle X, Y, $R_1$, $R_2$, $R_3$, et $R_4$ sont tels que définis précédemment,

γ) soit à l'action d'un chlorure de tosylate, suivi de l'action de $NaN_3$ en présence d'eau oxygénée, suivi d'une étape de réduction pour conduire aux composés de formule (I/m), cas particulier des composés de formule (I) :

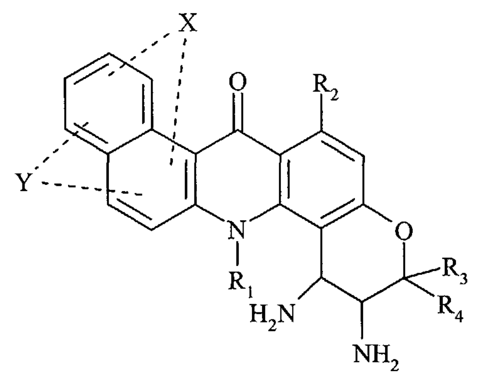

(I/m)

dans laquelle X, Y, $R_1$, $R_2$, $R_3$, et $R_4$ sont tels que définis précédemment, _composés de formule (I/m) qui sont soumis_

***1)*** soit à l'action du dioxyde de carbone, en présence de diphényl phosphite, pour conduire aux composés de formule (I/n), cas particulier des composés de formule (I):

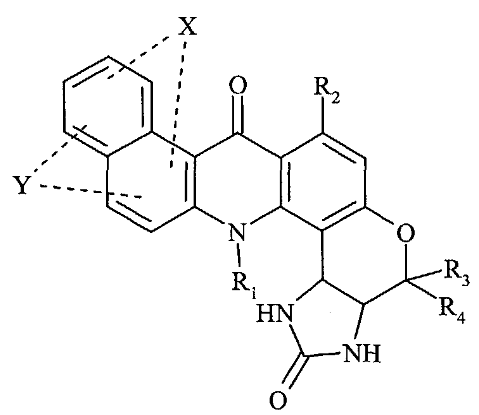

(I/n)

dans laquelle X, Y, $R_1$, $R_2$, $R_3$ et $R_4$ sont tels que définis précédemment,

***2)*** soit à l'action d'un composé de formule (XII) :

Hal-G'$_1$ (XII)

dans laquelle Hal est tel que défini précédemment et G'$_1$ représente un groupement choisi parmi -$R_c$, C($W_2$)-U-V, C($W_2$)-$W_3$-$T_1$, S(O)$_n$-$W_3$-$T_1$ et S(O)$_n$-U'-V' dans lesquels $R_c$, $W_2$, $W_3$, U, V, U', V', $T_1$ et n sont tels que définis dans la formule (I), pour conduire aux composés de formules (I/o$_1$), (I/o$_2$) et (I/o$_3$), cas particulier des composés de formule (I) :

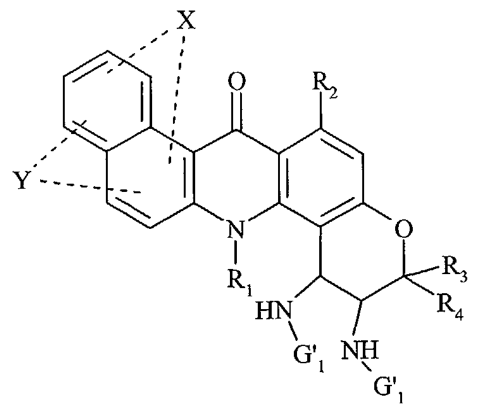

(I/o$_1$)

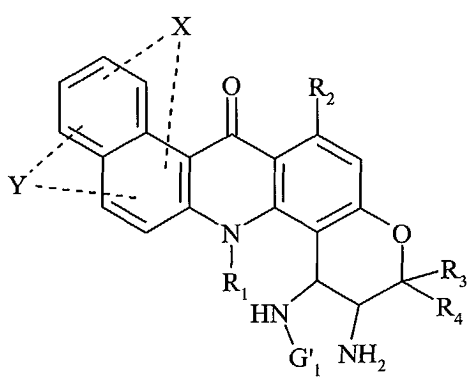

(I/o$_2$)

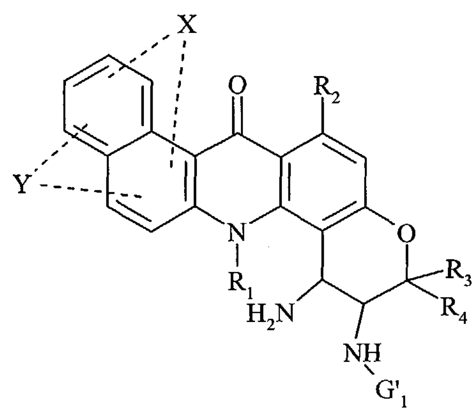

(I/o$_3$)

dans laquelle X, Y, $R_1$, $R_2$, $R_3$, $R_4$ et G'$_1$ sont tels que définis précédemment, composés de formules (I/o$_2$) et (I/o$_3$) dont la fonction amine primaire est protégée par un groupement protecteur des groupes amines primaires pour conduire aux composés de formules (XIII/a) et (XIII/b),

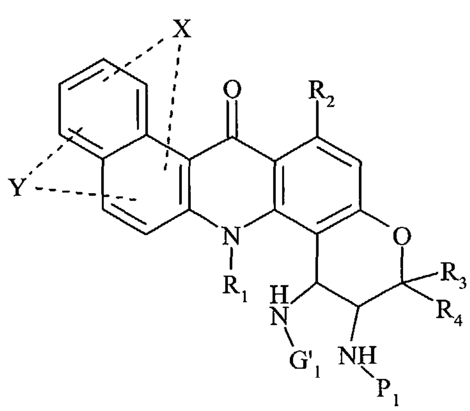

(XIII/a)

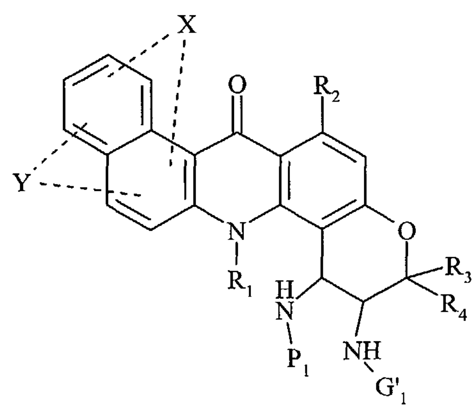

(XIII/b)

dans laquelle X, Y, $R_1$, $R_2$, $R_3$ $R_4$, $G'_1$ sont tels que définis précédemment et $P_1$ représente un groupement protecteur de fonctions amines primaires, composés de formules ($I/o_1$), (XIII/a) et (XIII/b) qui sont éventuellement soumis à l'action d'un composé de formule (XIV) :

$R_{c1}$-Hal (XIV)

dans laquelle Hal représente un halogène et $R_{c1}$ représente un groupement choisi parmi alkyle ($C_1$-$C_6$) linéaire ou ramifié, aryle ou arylalkyle ($C_1$-$C_6$) linéaire ou ramifié, puis qui sont soumis, dans le cas de composés de formule (XIII/a) et (XIII/b), à des conditions de déprotection de la fonction amine primaire, pour conduire aux composés de formules ($I/p_1$), ($I/p_2$) et ($I/p_3$), cas particuliers des composés de formule (I) :

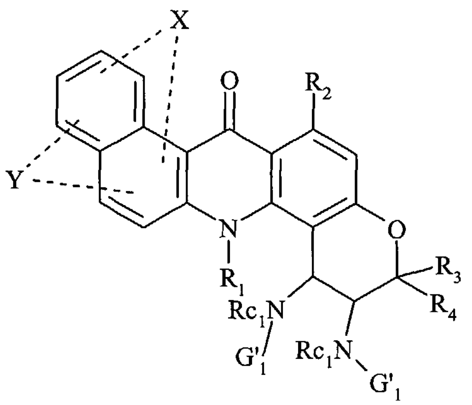

($I/p_1$)

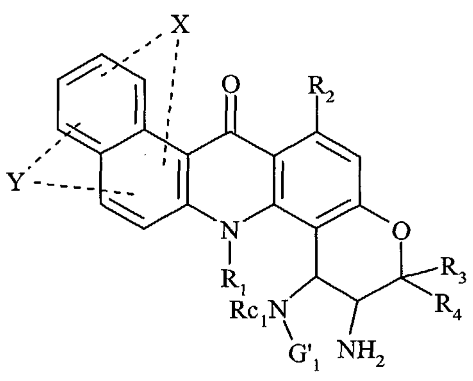

($I/p_2$)

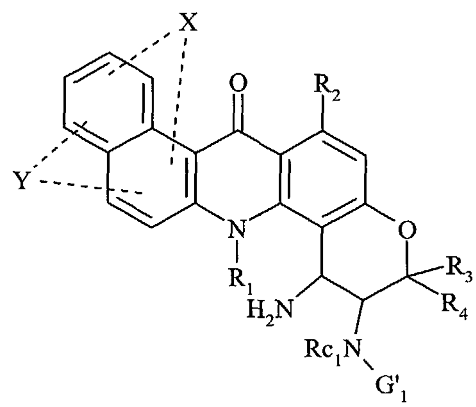

($I/p_3$)

dans laquelle X, Y, $R_1$, $R_2$, $R_3$ $R_{,4}$, $G'_1$ et $R_{c1}$ sont tels que définis précédemment, composés de formules ($I/p_2$) et ($I/p_3$) qui sont éventuellement soumis successivement à l'action d'un composé de formule (XIV) tel que défini précédemment puis d'un composé de formule (XV) :

$R_{d'1}$-Hal (XV)

dans laquelle Hal est tel que défini précédemment et $R_{d'1}$ peut prendre les même définitions que $R_{c1}$, pour conduire aux composés de formules ($I/q_2$) et ($I/q_3$), cas particuliers des composés de formule (I) :

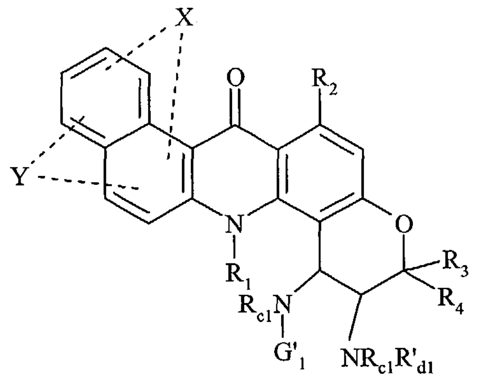

(I/q2)

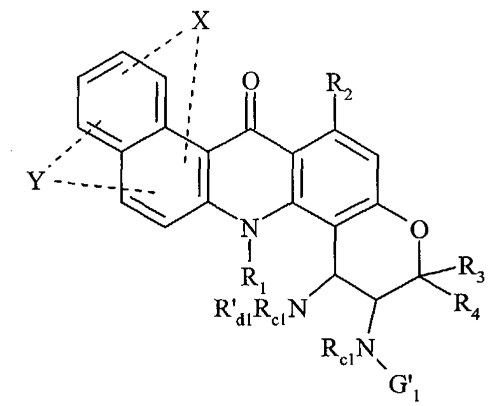

(I/q3)

dans laquelle X, Y, $R_1$, $R_2$, $R_3$ $R_4$, $G'_1$, $R_{c1}$ et $R_{d1}$ sont tels que définis précédemment, **composés de formule (I/l) pouvant être soumis:**

α) soit à l'action d'un composé de formule (XII) tel que défini précédemment, pour conduire aux composés de formule ($I/r_1$), ($I/r_2$) et ($I/r_3$), cas particuliers des composés de formule (I) :

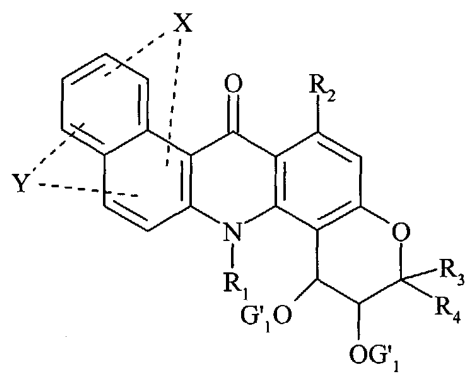

($I/r_1$)

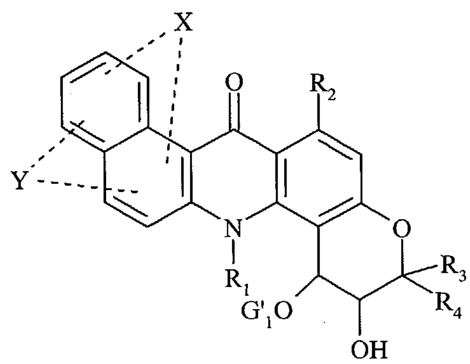

($I/r_2$)

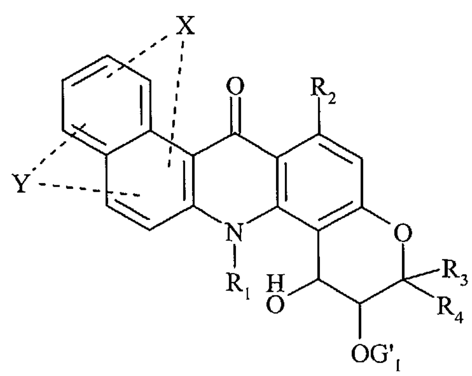

($I/r_3$)

dans lesquelles X, Y, $R_1$, $R_2$, $R_3$, $R_4$ et $G'_1$ sont tels que définis précédemment,

*composés de formule ($I/r_1$) qui sont soumis :*

***1)*** soit à l'action, lorsque $G'_1$ représente un groupement $C(W_2)$-U-V, d'un alcool de formule $R_{30}$-OH dans laquelle $R_{30}$ représente un groupement alkyle ($C_1$-$C_6$) linéaire ou ramifié, pour conduire aux composés de for-

mule ($I/s_1$), cas particulier des composés de formule (I) :

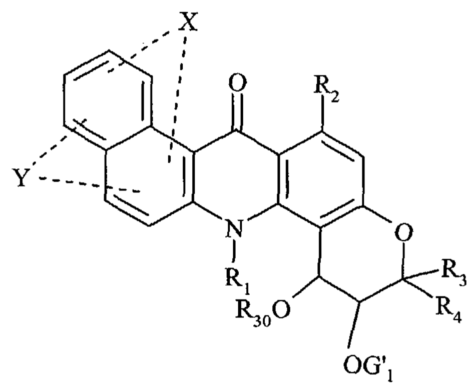

($I/s_1$)

dans laquelle X, Y, $R_1$, $R_2$, $R_3$ $R_4$, $G'_1$ et $R_{30}$ sont tels que définis précédemment,

***2)*** soit à l'action, lorsque $G'_1$ représente un groupement $C(W_2)$-U-V, d'un thiol de formule (XVI) :

$G_1$S-H (XVI)

dans laquelle $G_1$ représente un groupement choisi parmi $R_c$, $C(W_2)$-U-V, et $C(W_2)$-$W_3$-$T_1$ dans lesquels $R_c$, $W_2$, $W_3$, U, V et $T_1$ sont tels que définis dans la formule (I), pour conduire aux composés de formule ($I/t_1$), cas particulier des composés de formule (I) :

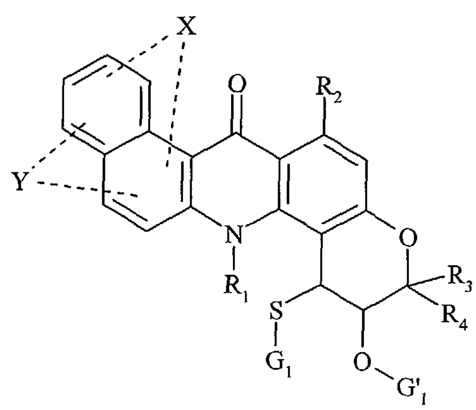

($I/t_1$)

dans laquelle X, Y, $R_1$, $R_2$, $R_3$, $R_4$, $G_1$ et $G'_1$ sont tels que définis précédemment, composés de formule ($I/t_1$) qui sont éventuellement traités par un agent de réduction, puis soumis à une réaction de déprotection de la fonction hydroxy, pour conduire aux composés de formule ($I/u_1$), cas particulier des composés de formule (I) :

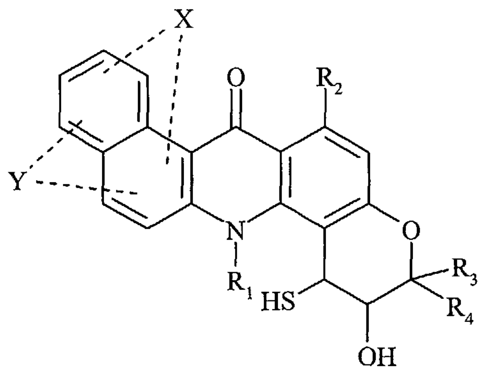

(I/$u_1$)

dans laquelle X, Y, $R_1$, $R_2$, $R_3$ et $R_4$ sont tels que définis précédemment, *<u>composés de formules (I/$r_2$) et (I/$r_3$) qui sont soumis :</u>*

***1)*** soit à l'action d'un anhydride de formule (XVII) ou d'un chlorure d'acide de formule (XVIII) :

$$[V\text{-}U\text{-}C(W_2)]_2O \text{ (XVII) } Cl\text{-}C(W_2)\text{-}U\text{-}V \text{ (XVIII)}$$

dans lesquelles $W_2$, U et V sont tels que définis dans la formule (I), pour conduire aux composés de formules (I/$v_2$) et (I/$v_3$), cas particuliers des composés de formule (I) :

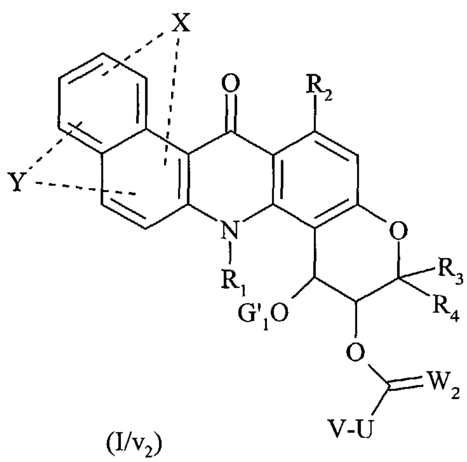

(I/$v_2$)

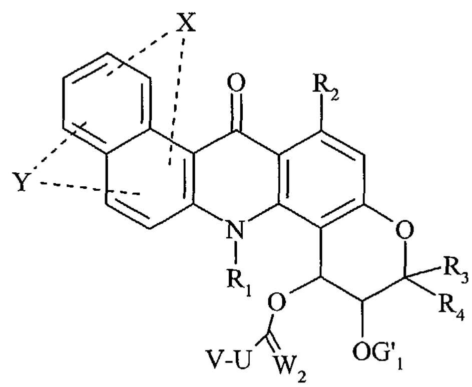

(I/$v_3$)

dans lesquelles X, Y, $R_1$, $R_2$, $R_3$, $R_4$, $G'_1$, $W_2$, U et V sont tels que définis précédemment,

***2)*** soit à des conditions de déshydratation en milieu acide, pour conduire aux composés de formule (I/$w_2$) et (I/$_{W3}$), cas particuliers des composés de formule (I) :

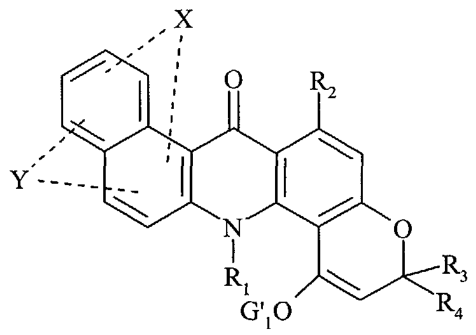

(I/$w_2$)

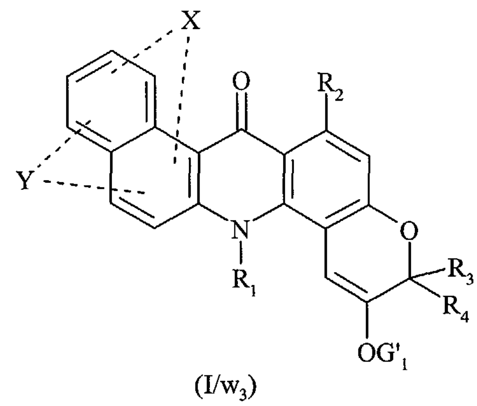

(I/$w_3$)

dans lesquelles X, Y, $R_1$, $R_2$, $R_3$, $R_4$ et $G'_1$ sont tels que définis précédemment,

β) soit à l'action d'un composé de formules (XIX) ou (XX)

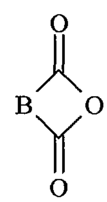

(XIX)

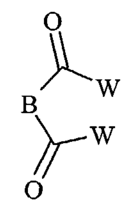

(XX)

dans laquelle B est tel que défini dans la formule (I) et W représente un atome d'halogène ou un groupement hydroxyle, pour conduire aux composés de formule (I/x), cas particulier des composés de formule (I) :

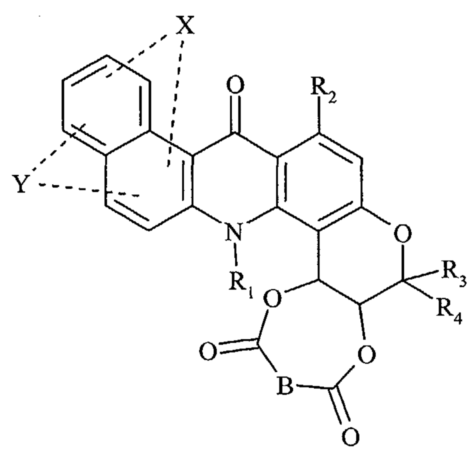

(I/x)

dans laquelle X, Y, $R_1$, $R_2$, $R_3$, $R_4$ et B sont tels que définis précédemment,

γ) soit à l'action d'un dihalogénure d'alkyle ($C_1$-$C_6$) linéaire, pour conduire aux composés de formule (I/y), cas particulier des composés de formule (I) :

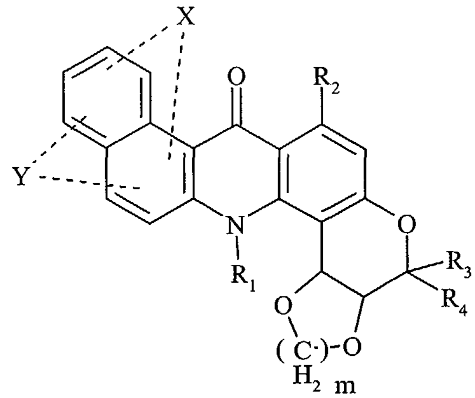

(I/y)

dans laquelle X, Y, $R_1$, $R_2$, $R_3$ et $R_4$ sont tels que définis précédemment, et m est tel que défini dans la formule (I),

δ) soit à l'action d'un équivalent d'un composé de formule (XVII) ou (XVIII), pour conduire aux composés de formule (I/z), cas particulier des composés de formule (I):

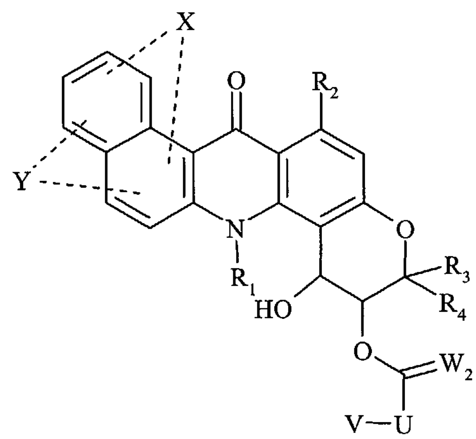

(I/z)

dans laquelle X, Y, $R_1$, $R_2$, $R_3$, $R_4$, $W_2$, U et V sont tels que définis précédemment, composés de formule (I/z) qui peuvent être soumis à un excès d'anhydride de formule (XVII') ou d'un chlorure d'acide de formule (XVIII')

$$[V_1\text{-}U_1\text{-}C(W_2)]_2O \text{ (XVII')} \quad Cl\text{-}C(W_2)\text{-}U_1\text{-}V_1 \text{ (XVIII')}$$

dans lesquelles $U_1$ et $V_1$ peuvent prendre les mêmes définitions que U et V et $W_2$ est tel que défini précédemment, pour conduire aux composés de formule (I/aa), cas particulier des composés de formule (I) :

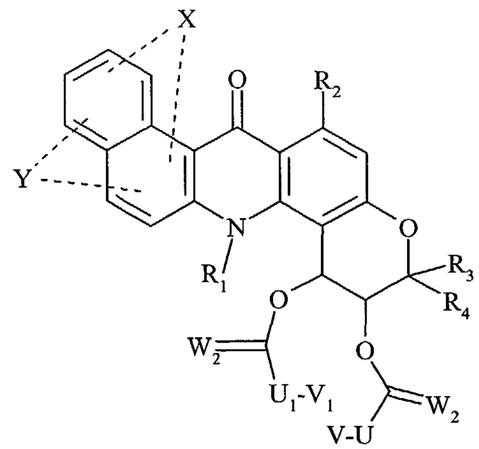

(I/aa)

dans laquelle X, Y, $R_1$, $R_2$, $R_3$, $R_4$, $W_2$, U, V, $U_1$ et $V_1$ sont tels que définis précédemment,

ε) soit à des conditions de déshydratation en milieu acide, pour conduire aux composés de formule (I/ab), cas particulier des composés de formule (I) :

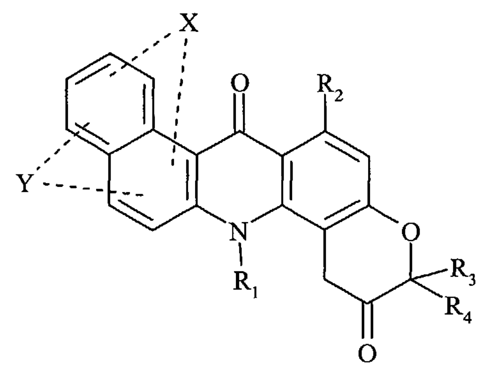

(I/ab)

dans laquelle X, Y, $R_1$, $R_2$, $R_3$ et $R_4$ sont tels que définis précédemment, composés de formule (I/ab) qui sont éventuellement réduits en présence de $NaBH_4$, pour conduire aux composés de formule (I/ac), cas particulier des composés de formule (I) :

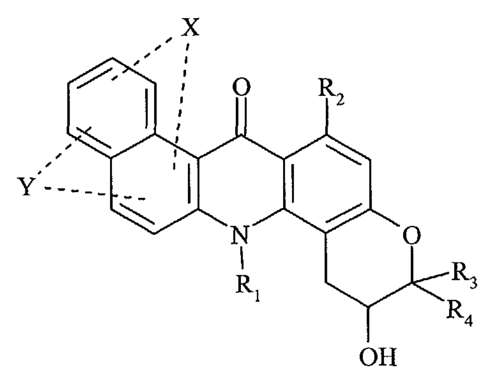

(I/ac)

dans laquelle X, Y, $R_1$, $R_2$, $R_3$ et $R_4$ sont tels que définis précédemment,

**d)** soit à l'action d'un péracide ou du diméthyle dioxirane, pour conduire aux composés de formule (I/ad), cas particulier des composés de formule (I) :

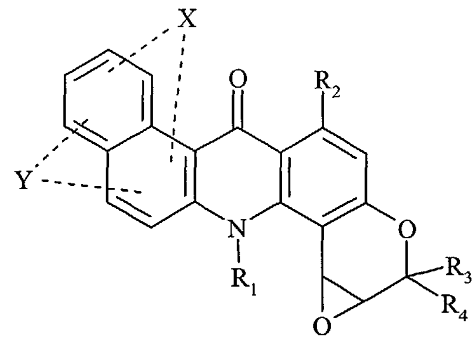

(I/ad)

dans laquelle X, Y, $R_1$, $R_2$, $R_3$ et $R_4$ sont tels que définis précédemment, composés de formule (I/ad) qui sont éventuellement traités par de l'ammoniac ou une amine primaire ou secondaire pour conduire aux composés de formules (I/$ae_1$) et (I/$ae_2$), cas particuliers des composés de formule (I) :

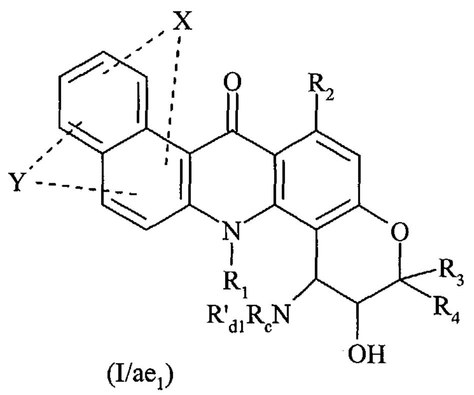

(I/$ae_1$)

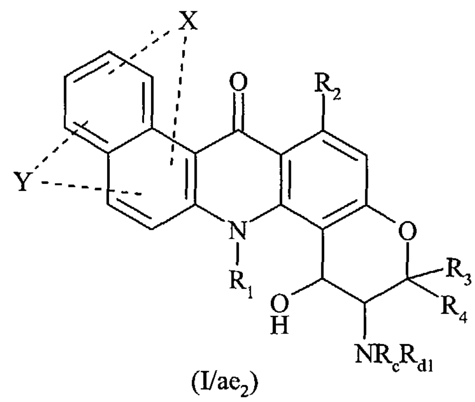

(I/$ae_2$)

dans laquelle X, Y, $R_1$, $R_2$, $R_3$ et $R_4$ sont tels que définis précédemment, $R_c$ est tel que défini dans la formule (I) et $R_{d1}$ représente un groupement choisi parmi $R_d$, - C($W_2$)-U-V, -C($W_2$)-$W_3$-$T_1$, -S(O)$_n$-$W_3$-$T_1$ et -S(O)$_n$-U'-V' dans lesquels $R_d$, $W_2$, $W_3$, U, V, $T_1$, U' et V' sont tels que définis précédemment, **composés de formules (I/$ae_1$) et (I/$ae_2$) pouvant être soumis**

α) soit, dans le cas où $R_c$ et $Rd_1$ représentent un atome d'hydrogène, à l'action d'un composé de formule (XI) tel que défini précédemment, pour conduire aux composés de formules (I/$af_1$) et (I/$af_2$), cas particuliers des composés de formule (I) :

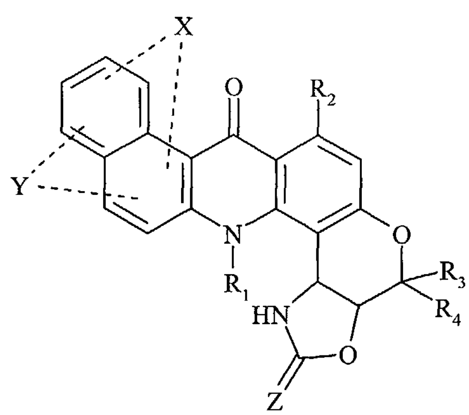

(I/$af_1$)

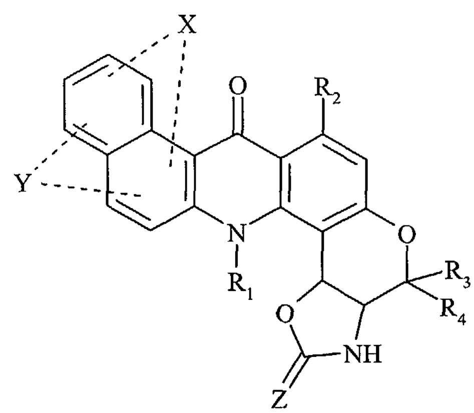

(I/$af_2$)

dans lesquelles X, Y, $R_1$, $R_2$, $R_3$, $R_4$ et Z sont tels que définis précédemment,

β) soit à l'action d'un composé de formules (XIX) ou (XX) tel que défini précédemment, pour conduire aux composés de formules (I/$ag_1$) et (I/$ag_2$), cas particuliers des composés de formule (I) :

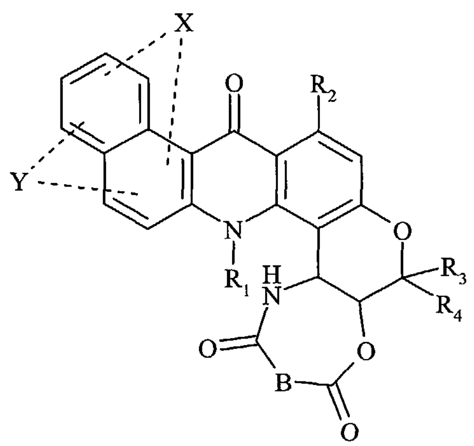

(I/$ag_1$)

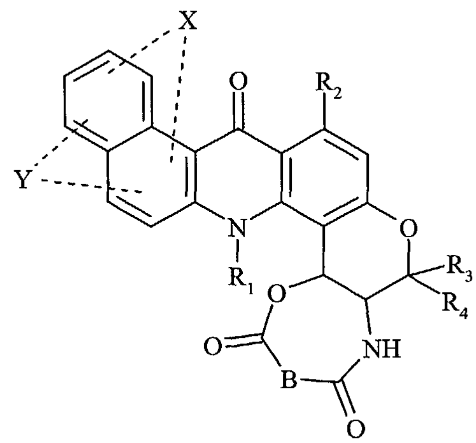

(I/$ag_2$)

dans lesquelles X, Y, $R_1$, $R_2$, $R_3$, $R_4$ et B sont tels que définis précédemment,

γ) soit à l'action d'un composé de formule (XII) tel que défini précédemment, pour conduire aux composés de formules (I/$ah_1$) et (I/$ah_2$), cas particuliers des composés de formule (I) :

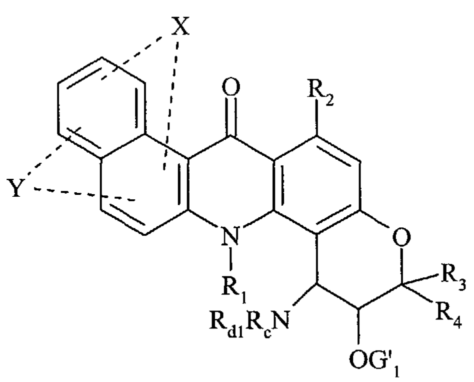

(I/$ah_1$)

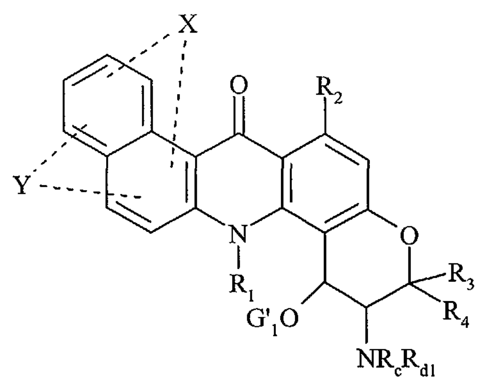

(I/$ah_2$)

dans lesquelles X, Y, $R_1$, $R_2$, $R_3$, $R_4$, $R_c$, $R_{d1}$ et $G'_1$ sont tels que définis précédemment,

δ) soit à l'action de dibromure de triphénylphosphine en présence de triéthylamine pour conduire aux composés de formules (I/ai), cas particulier des composés de formule (I) :

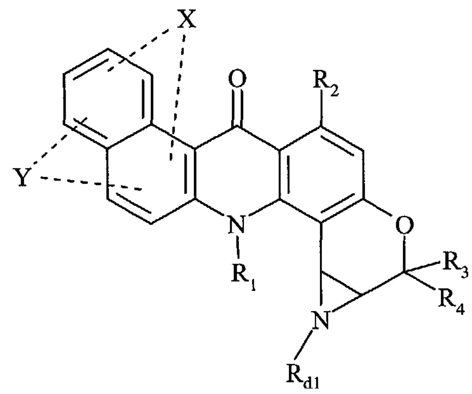

(I/ai)

dans laquelle X, Y, $R_1$, $R_2$, $R_3$, $R_4$ et $R_{d1}$ sont tels que définis précédemment,

ε) soit à des conditions de déshydratation en milieu acide, pour conduire aux composés de formules (I/$aj_2$) et (I/$aj_3$), cas particuliers des composés de formule (I) :

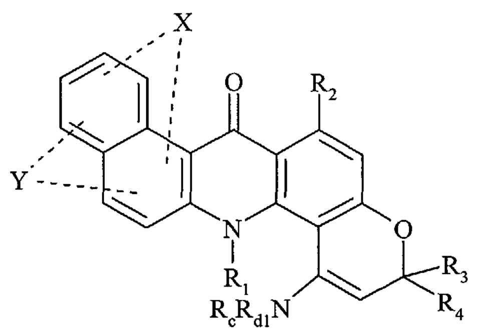

(I/$aj_2$)

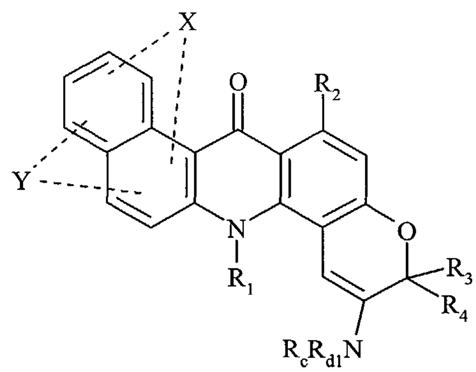

(I/$aj_3$)

dans laquelle X, Y, $R_1$, $R_2$, $R_3$ $R_4$, $R_c$ et $R_{d1}$ sont tels que définis précédemment,

e) soit à l'action d'un composé de formule (XXI) :

Hal-C($W_2$)-$T_1$ (XXI)

dans laquelle Hal représente un atome d'halogène et $W_2$ et $T_1$ sont tels que définis dans la formule (I), pour conduire aux composés de formule (I/aj), cas particulier des composés de formule (I) :

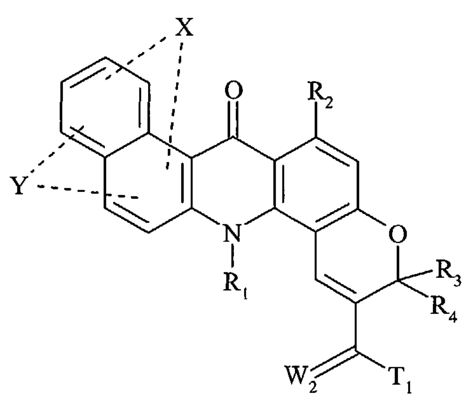

(I/aj)

dans laquelle X, Y, $R_1$, $R_2$, $R_3$ $R_4$, $W_2$ et $T_1$ sont tels que définis précédemment, les composés (I/a) à (I/aj) formant l'ensemble des composés de l'invention qui sont purifiés, le cas échéant, selon une technique classique de purification, qui peuvent, si on le désire, être séparés en leurs différents isomères selon une technique classique de séparation et qui sont transformés, si on le souhaite, en leurs N-oxydes et, le cas échéant, en leurs sels d'addition à un acide ou à une base pharmaceutiquement acceptable.

**18.** Procédé de préparation des composés de formule (I) pour lesquels A représente un groupement de formule-CH ($R_5$)-CH($R_6$) dans laquelle $R_5$ et $R_6$, identiques ou différents, indépendamment l'un de l'autre, représentent chacun un groupement choisi parmi l'atome d'hydrogène, un groupement $OR_c$, $W_1$-C($W_2$)-U-V dans lesquels $R_c$ est tel que défini dans la formule (I), $W_1$ et $W_2$ représentent un atome d'oxygène, U représente une chaîne alkylène ($C_1$-$C_8$) linéaire ou ramifiée et V représente un atome d'hydrogène ou $R_5$ et $R_6$ forment ensemble un groupement

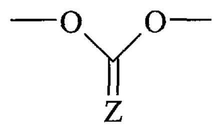

dans lequel Z est tel que défini dans la formule (I) peuvent avantageusement être obtenus à partir des composés de formule (I/e) :

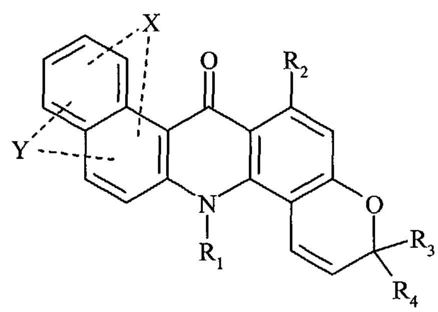

(I/e)

dans laquelle X, Y, $R_1$, $R_2$, $R_3$ et $R_4$ sont tels que définis précédemment, qui sont soumis à l'action de tétroxyde d'osmium en milieu polaire et en présence de *N*-oxyde de 4-méthylmorpholine, ou à l'action de permanganate de potassium suivi d'une réduction au borohydrure de sodium, pour conduire selon la méthode aux composés *cis*- ou *trans*-diol de formule (I/ak), cas particulier des composés de formule (I) :

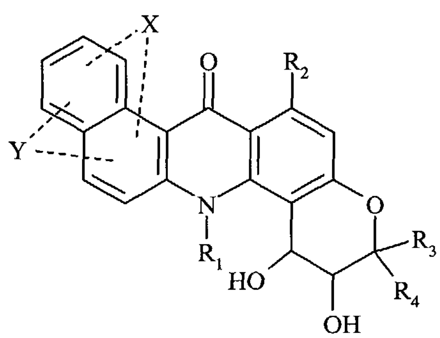

(I/ak)

dans laquelle X, Y, $R_1$, $R_2$, $R_3$ et $R_4$ sont tels que définis précédemment, composés *cis*-diol de formule (*cis*-I/ak) que l'on soumet éventuellement :

- ***soit à l'action*** de N,N'-carbonyldiimidazole ou de N,N'-thiocarbonyldiimidazole en présence de 2-butanone, pour conduire aux composés de formule (*cis*-I/al), cas particulier des composés de formule (I) :

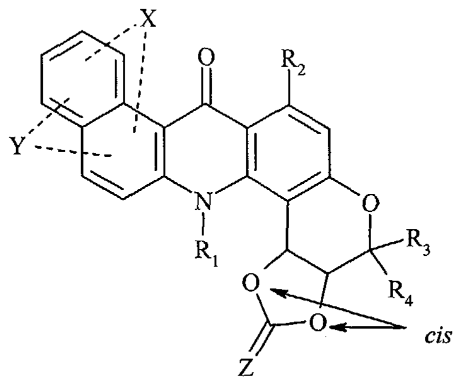

(*cis*-I/al)

dans laquelle X, Y, $R_1$, $R_2$, $R_3$ et $R_4$ sont tels que définis précédemment et Z est tel que défini dans la formule (I),

- ***soit à l'action*** d'un composé de formule (XXII) ou d'un composé de formule (XXIII) :

$$Hal — G_2 \text{ (XXII) ou } G_2\text{-O-}G_2 \text{ (XXIII)}$$

dans laquelle Hal représente un atome d'halogène et $G_2$ représente un groupement choisi parmi -C($W_2$)-U-V, -C($W_2$)-$W_3$-$T_1$, -S(O)$_n$-$W_3$-$T_1$ et -S(O)$_n$-U'-V' dans lesquels $W_2$, $W_3$, U, V, U', V' et n sont tels que définis dans la formule (I), pour conduire aux composés de formule (I/am), cas particulier des composés de formule (I) :

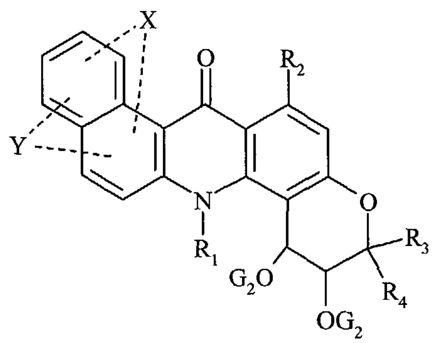

(I/am)

dans laquelle X, Y, $R_1$, $R_2$, $R_3$, $R_4$ et $G_2$ sont tels que définis précédemment, composé de formule (I/am), qui constitue l'ensemble des composés de formule (I), que l'on purifie, le cas échéant, selon une technique classique de purification , qui peuvent, si on le désire, être séparés en leurs différents isomères selon une technique classique de séparation et que l'on transforme , éventuellement, en leurs N-oxydes et, le cas échéant, en leurs sels d'addition à un acide ou à une base pharmaceutiquement acceptable.

**19.** Compositions pharmaceutiques contenant comme principe actif au moins un composé selon l'une quelconque des revendications 1 à 16, en combinaison avec un ou plusieurs excipients ou véhicules inertes, non toxiques, pharmaceutiquement acceptables.

**20.** Compositions pharmaceutiques selon la revendication 19 contenant au moins un principe actif selon l'une quelconque des revendication 1 à 16 utiles en tant que médicaments, dans le traitement des cancers.

Office européen des brevets

# RAPPORT DE RECHERCHE EUROPEENNE

Numéro de la demande
EP 04 29 1568

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | EP 1 297 835 A (SERVIER LAB)<br>2 avril 2003 (2003-04-02)<br>* page 24, ligne 47 - page 25, ligne 36; revendications 1-21 *<br>----- | 1-17 | C07D491/04<br>C07D491/14<br>A61K31/436<br>A61P35/00 |
| X | EP 1 061 081 A (ADIR)<br>20 décembre 2000 (2000-12-20)<br>* page 20, ligne 12 - page 21, ligne 5; revendications 1-12 *<br>----- | 1-17 | |
| A,D | WO 99/32491 A (ATASSI GHANEM ;KOCH MICHEL (FR); MICHEL SYLVIE (FR); RENARD PIERRE)<br>1 juillet 1999 (1999-07-01)<br>* le document en entier *<br>----- | 1-17 | |
| A,D | ELOMRI A ET AL: "Synthesis and cytotoxic and antitumor activity of esters in the 1,2-dihydroxy-1,2-dihydroacronycine series"<br>JOURNAL OF MEDICINAL CHEMISTRY, AMERICAN CHEMICAL SOCIETY. WASHINGTON, US,<br>vol. 24, no. 39, 1996, pages 4762-4764, XP002076704<br>ISSN: 0022-2623<br>* le document en entier *<br>----- | 1-17 | **DOMAINES TECHNIQUES RECHERCHES (Int.Cl.7)**<br>C07D<br>A61K |

*Le présent rapport a été établi pour toutes les revendications*

| Lieu de la recherche | Date d'achèvement de la recherche | Examinateur |
|---|---|---|
| Munich | 15 octobre 2004 | Goss, I |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 04 29 1568

La présente annexe indique les membres de la famille de brevets relatifs aux documents brevets cités dans le rapport de *recherche européenne visé ci-dessus.*
Lesdits members sont contenus au fichier informatique de l'Office européen des brevets à la date du
Les renseignements fournis sont donnés à titre indicatif et n'engagent pas la responsabilité de l'Office européen des brevets.

15-10-2004

| Document brevet cité au rapport de recherche | | Date de publication | Membre(s) de la famille de brevet(s) | | | Date de publication |
|---|---|---|---|---|---|---|
| EP 1297835 | A | 02-04-2003 | FR | 2827864 | A1 | 31-01-2003 |
| | | | BR | 0202864 | A | 03-06-2003 |
| | | | CA | 2395652 | A1 | 25-01-2003 |
| | | | CN | 1398863 | A | 26-02-2003 |
| | | | EP | 1297835 | A1 | 02-04-2003 |
| | | | HU | 0202485 | A2 | 28-04-2003 |
| | | | JP | 2003055381 | A | 26-02-2003 |
| | | | NO | 20023556 | A | 27-01-2003 |
| | | | NZ | 520377 | A | 26-03-2004 |
| | | | PL | 355188 | A1 | 27-01-2003 |
| | | | US | 2003073841 | A1 | 17-04-2003 |
| | | | US | 2004063702 | A1 | 01-04-2004 |
| | | | ZA | 200205961 | A | 01-04-2003 |
| EP 1061081 | A | 20-12-2000 | FR | 2795071 | A1 | 22-12-2000 |
| | | | AT | 216392 | T | 15-05-2002 |
| | | | AU | 774499 | B2 | 01-07-2004 |
| | | | AU | 4089800 | A | 21-12-2000 |
| | | | BR | 0002679 | A | 30-01-2001 |
| | | | CA | 2312409 | A1 | 16-12-2000 |
| | | | CN | 1279239 | A | 10-01-2001 |
| | | | DE | 60000125 | D1 | 23-05-2002 |
| | | | DE | 60000125 | T2 | 05-12-2002 |
| | | | DK | 1061081 | T3 | 12-08-2002 |
| | | | EA | 3361 | B1 | 24-04-2003 |
| | | | EP | 1061081 | A1 | 20-12-2000 |
| | | | ES | 2174803 | T3 | 16-11-2002 |
| | | | HU | 0002288 | A2 | 28-04-2001 |
| | | | JP | 3364473 | B2 | 08-01-2003 |
| | | | JP | 2001011078 | A | 16-01-2001 |
| | | | NO | 20002940 | A | 18-12-2000 |
| | | | NZ | 505184 | A | 28-09-2001 |
| | | | PL | 340788 | A1 | 18-12-2000 |
| | | | PT | 1061081 | T | 30-08-2002 |
| | | | SI | 1061081 | T1 | 30-06-2002 |
| | | | US | 6503919 | B1 | 07-01-2003 |
| | | | ZA | 200003035 | A | 11-01-2001 |
| WO 9932491 | A | 01-07-1999 | FR | 2772765 | A1 | 25-06-1999 |
| | | | AT | 219491 | T | 15-07-2002 |
| | | | AU | 738322 | B2 | 13-09-2001 |
| | | | AU | 1766399 | A | 12-07-1999 |
| | | | BR | 9813756 | A | 03-10-2000 |
| | | | CA | 2315909 | A1 | 01-07-1999 |
| | | | CN | 1109686 | B | 28-05-2003 |
| | | | DE | 69806189 | D1 | 25-07-2002 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE RELATIF A LA DEMANDE DE BREVET EUROPEEN NO. EP 04 29 1568

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-10-2004

| Document brevet cité au rapport de recherche | Date de publication | Membre(s) de la famille de brevet(s) | Date de publication |
|---|---|---|---|
| WO 9932491 A | | DE 69806189 T2 | 13-02-2003 |
| | | DK 1042326 T3 | 23-09-2002 |
| | | EP 1042326 A1 | 11-10-2000 |
| | | ES 2179548 T3 | 16-01-2003 |
| | | WO 9932491 A1 | 01-07-1999 |
| | | HK 1033131 A1 | 12-12-2003 |
| | | HU 0100342 A2 | 28-09-2001 |
| | | JP 2001526290 T | 18-12-2001 |
| | | NO 20003106 A | 16-08-2000 |
| | | NZ 504833 A | 30-11-2001 |
| | | PL 341281 A1 | 09-04-2001 |
| | | PT 1042326 T | 31-10-2002 |
| | | US 6288073 B1 | 11-09-2001 |
| | | ZA 9811645 A | 24-06-1999 |

EPO FORM P0460

Pour tout renseignement concernant cette annexe : voir Journal Officiel de l'Office européen des brevets, No.12/82